# EUROPEAN PATENT SPECIFICATION

(11) **EP 1 860 186 B1**
(45) Date of publication and mention of the grant of the patent: **17.11.2010**
(21) Application number: 07017847.0
(22) Date of filing: 27.02.2004
(51) Int. Cl.: C12N 15/11, C12N 15/00, C12N 5/10, C12Q 1/68, G01N 33/53

(54) **Method for distinguishing mesenchymal stem cells using different markers**
Verfahren zur Kennzeichnung mesenchymaler Stammzellen unter Verwendung verschiedener Marker
Procédé pour la distinction de cellules souches mésenchymateuses à l'aide de divers marqueurs

(30) Priority: 10.03.2003 JP 2003063077
(43) Date of publication of application: 28.11.2007
(62) Divisional of application: 04715524.7
(73) Proprietor: Japan Science And Technology Agency, Kawaguchi-shi Saitama 332-0012 (JP); Kato, Yukio, Hiroshima-shi 732-0062 (JP); Two Cells Co., Ltd, Hiroshima-shi, Hiroshioma 732-0811 (JP)
(72) Inventor: Koike, Chika University of Toyama Graduate School of Med. and Pharm. Sc., Toyama 930-0194 (JP); Tsuji, Koichiro, Hiroshima-shi Hiroshima 732-0811 (JP); Kato, Yukio, Hiroshima-shi Hiroshima 732-0062 (JP)
(74) Representative: Harding, Charles Thomas

(56) References cited:
- WO-A-01/83709
- WO-A-01/94629
- WO-A-02/28999
- WO-A-93/13119
- PITTENGER M F ET AL: "MULTILINEAGE POTENTIAL OF ADULT HUMAN MESENCHYMAL STEM CELLS" SCIENCE, AMERICAN ASSOCIATION FOR THE ADVANCEMENT OF SCIENCE,, US, vol. 284, no. 5411, 2 April 1999 (1999-04-02), pages 143-147, XP000867221 ISSN: 0036-8075
- TREMAIN N ET AL: "MICROSAGE ANALYSIS OF 2,353 EXPRESSED GENES IN A SINGLE CELL-DERIVED COLONY OF UNDIFFERENTIATED HUMAN MESENCHYMAL STEM CELLS REVEALS MRNAS OF MULTIPLE CELL LINEAGES" STEM CELLS, ALPHAMED PRESS, DAYTON, OH, US, vol. 19, 2001, pages 408-418, XP002907879 ISSN: 1066-5099
- LODIE T A ET AL: "Systematic analysis of reportedly distinct populations of multipotent bone marrow-derived stem cells reveals a lack of distinction" TISSUE ENGINEERING, LARCHMONT, NY, US, vol. 8, no. 5, October 2002 (2002-10), pages 739-751, XP002306489 ISSN: 1076-3279
- BARI DE C ET AL: "Multipotent mesenchymal stem cells from adult human synovial membrane" ARTHRITIS AND RHEUMATISM, LIPPINCOTT, PHILADELPHIA, US, vol. 44, no. 8, August 2001 (2001-08), pages 1928-1942, XP002266867 ISSN: 0004-3591
- ISHII M ET AL: "Molecular markers distinguish bone marrow mesenchymal stem cells from fibroblasts" BIOCHEMICAL AND BIOPHYSICAL RESEARCH COMMUNICATIONS, ACADEMIC PRESS INC. ORLANDO, FL, US, vol. 332, no. 1, 24 June 2005 (2005-06-24), pages 297-303, XP004892979 ISSN: 0006-291X
- CLARKE E ET AL: "Mesenchymal cell precursors from human bone marrow have a phenotype that is distinct from cultured mesenchymal cells and are exclusively present in a small subset of CD45lo SH2+ cells" BLOOD, W.B.SAUNDERS COMPANY, ORLANDO, FL, US, vol. 98, no. 11, PART 1, 2001, page 85A, XP002973272 ISSN: 0006-4971
- SANCHEZ DIEGO ET AL: "Expression pattern of the lipocalin Apolipoprotein D during mouse embryogenesis" MECHANISMS OF DEVELOPMENT, vol. 110, no. 1-2, January 2002 (2002-01), pages 225-229, XP002455241 ISSN: 0925-4773
- PRICE P A ET AL: "MOLECULAR CLONING OF MATRIX GLA PROTEIN IMPLICATIONS FOR SUBSTRATE RECOGNITION BY THE VITAMIN K-DEPENDENT GAMMA CARBOXYLASE" PROCEEDINGS OF THE NATIONAL ACADEMY OF SCIENCES OF THE UNITED STATES OF AMERICA, vol. 84, no. 23, 1987, pages 8335-8339, XP002466611 ISSN: 0027-8424
- YAGAMI KIMITOSHI ET AL: "Matrix GLA protein is a developmental regulator of chondrocyte mineralization and, when constitutively expressed, blocks endochondral and intramembranous ossification in the limb" JOURNAL OF CELL BIOLOGY, vol. 147, no. 5, 29 November 1999 (1999-11-29), pages 1097-1108, XP002466612 ISSN: 0021-9525
- STEVENS R L ET AL: "ISOLATION AND CHARACTERIZATION OF A COMPLEMENTARY DNA THAT ENCODES THE PEPTIDE CORE OF THE SECRETORY GRANULE PROTEOGLYCAN OF HUMAN PROMYELOCYTIC LEUKEMIA HL-60 CELLS" JOURNAL OF BIOLOGICAL CHEMISTRY, vol. 263, no. 15, 1988, pages 7287-7291, XP002466613 ISSN: 0021-9258
- FURUSHIMA KOZO ET AL: "Large-scale screening for candidate genes of ossification of the posterior longitudinal ligament of the spine" JOURNAL OF BONE AND MINERAL RESEARCH, vol. 17, no. 1, January 2002 (2002-01), pages 128-137, XP002466614 ISSN: 0884-0431
- KIVIRIKKO SIRPA ET AL: "Distribution of type XV collagen transcripts in human tissue and their production by muscle cells and fibroblasts" AMERICAN JOURNAL OF PATHOLOGY, vol. 147, no. 5, 1995, pages 1500-1509, XP002466615 ISSN: 0002-9440
- MYERS JEANNE C ET AL: "Identification of a previously unknown human collagen chain, alpha-1(XV), characterized by extensive interruptions in the triple-helical region" PROCEEDINGS OF THE NATIONAL ACADEMY OF SCIENCES OF THE UNITED STATES OF AMERICA, vol. 89, no. 21, 1992, pages 10144-10148, XP002466616 ISSN: 0027-8424
- GOOD P J ET AL: "A family of human RNA-binding proteins related to the Drosophila Bruno translational regulator" JOURNAL OF BIOLOGICAL CHEMISTRY, AMERICAN SOCIETY OF BIOLOCHEMICAL BIOLOGISTS, BIRMINGHAM,, US, vol. 275, no. 37, 15 September 2000 (2000-09-15), pages 28583-28592, XP002262155 ISSN: 0021-9258
- LADD ANDREA N ET AL: "The CELF family of RNA binding proteins is implicated in cell-specific and developmentally regulated alternative splicing" MOLECULAR AND CELLULAR BIOLOGY, vol. 21, no. 4, February 2001 (2001-02), pages 1285-1296, XP002466617 ISSN: 0270-7306

### SEQUENCE LISTING

<110> Japan Science and Technology Agency
   Yukio KATO
   Two Cells Co., Ltd.
<120> Marker for detecting mesenchymal stem cell and method of distinguishing mesenchymal stem cell using the marker.
<130> P024253EPA
<150> JP2003-63077
   <151> 2000-03-10
<160> 130
<170> Patent In version 3.4
<210> 1
   <211> 652
   <212> DNA
   <213> Homo sapiens
<400> 1
   gaattgccaa ttccttgttt gtgcaaaatg gatttcatgt caatgaggag tttttgcaaa     60
   tgatgaaaaa atattttaat gcagcagtaa atcatgtgga cttcagtcaa aatgtagccg    120
   tggccaacta catcaataag tgggtggaga ataacacaaa caatctggtg aaagatttgg    180
   tatccccaag ggattttgat gctgccactt atctggccct cattaatgct gtctatttca    240
   aggggaactg gaagtcgcag tttaggcctg aaaatactag aaccttttct ttcactaaag    300
   atgatgaaag tgaagtccaa attccaatga tgtatcagca aggagaattt tattatgggg    360
   aatttagtga tggctccaat gaagctggtg gtatctacca agtcctagaa ataccatatg    420
   aaggagatga aataagcatg atgctggtgc tgtccagaca ggaagttcct cttgctactc    480
   tggagccatt agtcaaagca cagctggttg aagaatgggc aaactctgtg aagaagcaaa    540
   aagtagaagt atacctgccc aggttcacag tggaacagga aattgattta aaagatgttt    600
   tgaaggctct tggaataact gaaaattttc atcaaagatg caaattttga ca            652
<210> 2
   <211> 1450
   <212> DNA
   <213> Homo sapiens
<400> 2
   ctggatagaa cagctcaagc cttgccactt cgggcttctc actgcagctg ggcttggact     60
   tcggagtttt gccattgcca gtgggacgtc tgagactttc tccttcaagt acttggcaga    120
   tcactctctt agcagggtct gcgcttcgca gccgggatga agctggtttc cgtcgccctg    180
   atgtacctgg gttcgctcgc cttcctaggc gctgacaccg ctcggttgga tgtcgcgtcg    240
   gagtttcgaa agaagtggaa taagtgggct ctgagtcgtg ggaagaggga actgcggatg    300
   tccagcagct accccaccgg gctcgctgac gtgaaggccg ggcctgccca gacccttatt    360
   cggccccagg acatgaaggg tgcctctcga agccccgaag acagcagtcc ggatgccgcc    420
   cgcatccgag tcaagcgcta ccgccagagc atgaacaact tccagggcct ccggagcttt    480
   ggctgccgct tcgggacgtg cacggtgcag aagctggcac accagatcta ccagttcaca    540
   gataaggaca aggacaacgt cgcccccagg agcaagatca gcccccaggg ctacggccgc    600
   cggcgccggc gctccctgcc cgaggccggc ccgggtcgga ctctggtgtc ttctaagcca    660
   caagcacacg gggctccagc ccccccgagt ggaagtgctc cccactttct ttaggattta    720
   ggcgcccatg gtacaaggaa tagtcgcgca agcatcccgc tggtgcctcc cgggacgaag    780
   gacttcccga gcggtgtggg gaccgggctc tgacagccct gcggagaccc tgagtccggg    840
   aggcaccgtc cggcggcgag ctctggcttt gcaagggccc ctccttctgg gggcttcgct    900
   tccttagcct tgctcaggtg caagtgcccc agggggcggg gtgcagaaga atccgagtgt    960
   ttgccaggct taaggagagg agaaactgag aaatgaatgc tgagaccccc ggagcagggg   1020
   tctgagccac agccgtgctc gcccacaaac tgatttctca cggcgtgtca ccccaccagg   1080
   gcgcaagcct cactattact tgaactttcc aaaacctaaa gaggaaaagt gcaatgcgtg   1140
   ttgtacatac agaggtaact atcaatattt aagtttgttg ctgtcaagat tttttttgta   1200
   acttcaaata tagagatatt tttgtacgtt atatattgta ttaagggcat tttaaaagca   1260
   attatattgt cctcccccta ttttaagacg tgaatgtctc agcgaggtgt aaagttgttc   1320
   gccgcgtgga atgtgagtgt gtttgtgtgc atgaaagaga aagactgatt acctcctgtg   1380
   tggaagaagg aaacaccgag tctctgtata atctatttac ataaaatggg tgatatgcga   1440
   acagcaaacc                                                          1450
<210> 3
   <211> 185
   <212> PRT
   <213> Homo sapiens
<400> 3
   Met Lys Leu Val Ser Val Ala Leu Met Tyr Leu Gly Ser Leu Ala Phe
    1               5                  10                  15
   Leu Gly Ala Asp Thr Ala Arg Leu Asp Val Ala Ser Glu Phe Arg Lys
               20                  25                  30
   Lys Trp Asn Lys Trp Ala Leu Ser Arg Gly Lys Arg Glu Leu Arg Met
           35                  40                  45
   Ser Ser Ser Tyr Pro Thr Gly Leu Ala Asp Val Lys Ala Gly Pro Ala
       50                  55                  60
   Gln Thr Leu Ile Arg Pro Gln Asp Met Lys Gly Ala Ser Arg Ser Pro
   65                  70                  75                  80
   Glu Asp Ser Ser Pro Asp Ala Ala Arg Ile Arg Val Lys Arg Tyr Arg
                   85                  90                  95
   Gln Ser Met Asn Asn Phe Gln Gly Leu Arg Ser Phe Gly Cys Arg Phe
               100                 105                 110
   Gly Thr Cys Thr Val Gln Lys Leu Ala His Gln Ile Tyr Gln Phe Thr
           115                 120                 125
   Asp Lys Asp Lys Asp Asn Val Ala Pro Arg Ser Lys Ile Ser Pro Gln
       130                 135                 140
   Gly Tyr Gly Arg Arg Arg Arg Arg Ser Leu Pro Glu Ala Gly Pro Gly
   145                 150                 155                 160
   Arg Thr Leu Val Ser Ser Lys Pro Gln Ala His Gly Ala Pro Ala Pro
                   165                 170                 175
   Pro Ser Gly Ser Ala Pro His Phe Leu
               180                 185
<210> 4
   <211> 809
   <212> DNA
   <213> Homo sapiens
<400> 4
   atgcctgtct tcatcttgaa agaaaagctc caggtccctt ctccagccac ccagccccaa      60
   gatggtgatg ctgctgctgc tgctttccgc actggctggc ctcttcggtg cggcagaggg     120
   acaagcattt catcttggga agtgccccaa tcctccggtg caggagaatt ttgacgtgaa     180
   taagtatctc ggaagatggt acgaaattga gaagatccca acaacctttg agaatggacg     240
   ctgcatccag gccaactact cactaatgga aaacggaaag atcaaagtgt taaaccagga     300
   gttgagagct gatggaactg tgaatcaaat cgaaggtgaa gccaccccag ttaacctcac     360
   agagcctgcc aagctggaag ttaagttttc ctggtttatg ccatcggcac cgtactggat     420
   cctggccacc gactatgaga actatgccct cgtgtattcc tgtacctgca tcatccaact     480
   ttttcacgtg gattttgctt ggatcttggc aagaaaccct aatctccctc cagaaacagt      540
   ggactctcta aaaaatatcc tgacttctaa taacattgat gtcaagaaaa tgacggtcac     600
   agaccaggtg aactgcccca agctctcgta accaggttct acagggaggc tgcacccact     660
   ccatgttact tctgcttcgc tttcccctac ccccaccccc cccataaaga caaaccaatc     720
   aaccacgaca aaggaagttg acctaaacat gtaaccatgc cctaccctgt taccttgcta     780
   gctgcaaaat aaacttgttg ctgacctgc                                       809
<210> 5
   <211> 189
   <212> PRT
   <213> Homo sapiens
<400> 5
   Met Val Met Leu Leu Leu Leu Leu Ser Ala Leu Ala Gly Leu Phe Gly
   1               5                   10                  15
   Ala Ala Glu Gly Gln Ala Phe His Leu Gly Lys Cys Pro Asn Pro Pro
               20                  25                  30
   Val Gln Glu Asn Phe Asp Val Asn Lys Tyr Leu Gly Arg Trp Tyr Glu
           35                  40                  45
   Ile Glu Lys Ile Pro Thr Thr Phe Glu Asn Gly Arg Cys Ile Gln Ala
       50                  55                  60
   Asn Tyr Ser Leu Met Glu Asn Gly Lys Ile Lys Val Leu Asn Gln Glu
   65                  70                  75                  80
   Leu Arg Ala Asp Gly Thr Val Asn Gln Ile Glu Gly Glu Ala Thr Pro
                   85                  90                  95
   Val Asn Leu Thr Glu Pro Ala Lys Leu Glu Val Lys Phe Ser Trp Phe
               100                 105                 110
   Met Pro Ser Ala Pro Tyr Trp Ile Leu Ala Thr Asp Tyr Glu Asn Tyr
           115                 120                 125
   Ala Leu Val Tyr Ser Cys Thr Cys Ile Ile Gln Leu Phe His Val Asp
       130                 135                 140
   Phe Ala Trp Ile Leu Ala Arg Asn Pro Asn Leu Pro Pro Glu Thr Val
   145                 150                 155                 160
   Asp Ser Leu Lys Asn Ile Leu Thr Ser Asn Asn Ile Asp Val Lys Lys
                   165                 170                 175
   Met Thr Val Thr Asp Gln Val Asn Cys Pro Lys Leu Ser
               180                 185
<210> 6
   <211> 2127
   <212> DNA
   <213> Homo sapiens
<400> 6
   gaaagatgga tcactccagc tcaaagagaa catgtgggaa tgaaaggaca ggctgggccc 60
   aaaggagaaa agggtgatgc tggggaggag cttcctggcc ctcctgaacc ttctgggcct 120
   gttggaccca cggcaggagc agaagcagag ggctctggcc taggctgggg ctcggacgtc 180
   ggctctggct ctggtgacct ggtgggcagt gagcagctgc tgagaggtcc tccaggaccc 240
   ccagggccac ctggcttacc tgggattcca ggaaaaccag gaactgatgt tttcatggga 300
   ccccctggat ctcctggaga ggatggacct gctggtgaac ctgggccccc gggccctgag 360
   ggacagcctg gagttgatgg agccaccggc cttcccggga tgaaagggga gaagggagca 420
   agagggccta atggctcagt tggtgaaaag ggtgaccctg gcaacagagg cttacctgga 480
   cccccgggga aaaagggaca agctggccct cctggggtca tgggaccccc agggcctcct 540
   ggaccccctg ggcccccagg ccctggatgc acaatgggac ttggattcga ggataccgaa 600
   ggctctggaa gcacccagct attgaatgaa cccaaactct ccagaccaac ggctgcaatt 660
   ggtctcaaag gagagaaagg agaccgggga cccaagggag aaagggggat ggatggagcc 720
   agtattgtgg gaccccctgg gccgagaggg ccacctgggc acatcaaggt cttgtctaat 780
   tccttgatca atatcaccca tggattcatg aatttctcgg acattcctga gctggtgggg 840
   cctccggggc cggacgggtt gcctgggctg ccaggatttc caggtcctag aggaccaaaa 900
   ggtgacactg gtttacctgg ctttccagga ctaaaaggag aacagggcga gaagggagag 960
   ccgggtgcca tcctgacaga ggacattcct ctggaaaggc tgatggggaa aaagggtgaa 1020
   cctggaatgc atggagcccc aggaccaatg gggcccaaag gaccaccagg acataaagga 1080
   gaatttggcc ttcccgggcg acctggtcgc ccaggactga atggcctcaa gggtaccaaa 1140
   ggagatccag gggtcattat gcagggccca cctggcttac ctggccctcc aggcccccct 1200
   gggccacctg gagctgtgat taacatcaaa ggagccattt tcccaatacc cgtccgacca 1260
   cactgcaaaa tgccagttga tactgctcat cctgggagtc cagagctcat cacttttcac 1320
   ggtgttaaag gagagaaagg atcctggggt cttcctggct caaagggaga aaaaggcgac 1380
   cagggagccc agggaccacc aggtcctcca cttgatctag cttacctgag acactttctg   1440
   aacaacttga agggggagaa tggagacaag gggttcaaag gtgaaaaagg agaaaaagga   1500
   gacattaatg gcagcttcct tatgtctggg cctccaggcc tgcccggaaa tccaggcccg   1560
   gctggccaaa aaggggagac agtcgttggg ccccaaggac ccccaggtgc tcctggtctg   1620
   cctgggccac ctggctttgg aagacctggt gatcctgggc caccggggcc cccggggcca   1680
   ccaggacctc cagctatcct gggagcagct gtggcccttc caggtccccc tggccctcca   1740
   ggacagccag ggcttcccgg atccagaaac ctggtcacag cattcagcaa catggatgac   1800
   atgctgcaga aagcgcattt ggttatagaa ggaacattca tctacctgag ggacagcact   1860
   gagtttttca ttcgtgttag agatggctgg aaaaaattac agctgggaga actgatcccc   1920
   attcctgccg acagccctcc accccctgcg ctttccagca acccacatca gcttctgcct   1980
   ccaccaaacc ctatttcaag tgccaattat gagaagcctg ctctgcattt ggctgctctg   2040
   aacatgccat tttctgggga cattcgagct gattttcagt gcttcaagca ggccagagct   2100
   gcaggactgt tgtccaccta ccgagca                                       2127
<210> 7
   <211> 2384
   <212> DNA
   <213> Homo sapiens
<400> 7
   cctgctcgtt cctgacagaa ttaacggcac agccaacaag atgaacggag ctttggatca     60
   ctcagaccaa ccagacccag atgccattaa gatgtttgtc ggacagatcc cccggtcatg    120
   gtcggaaaag gagctgaaag aactttttga gccttacgga gccgtctacc agatcaacgt    180
   cctccgggac cggagtcaga accctccgca gagtaaaggt tgttgtttcg taacatttta    240
   tacaagaaaa gctgcacttg aggcccagaa tgcactgcac aatattaaaa ctttacctgg    300
   gatgcatcat cccattcaga tgaaacctgc agatagtgaa aagtccaacg ctgtggaaga    360
   cagaaaattg ttcataggaa tggtttcgaa gaaatgtaat gagaacgaca tcagggtgat    420
   gttctctcca tttggccaga tagaagaatg ccggatcctc cggggacctg atgggctgag    480
   tcgaggctgt gcgtttgtca cattttctac aagggcaatg gcacagaatg caatcaaagc    540
   catgcatcag tctcagacca tggagggctg ctcttcacct atcgtggtga agtttgctga    600
   cactcagaag gacaaagagc aaaggcgcct ccagcagcag ctcgctcagc agatgcagca    660
   gctcaacact gccacctggg ggaacctgac agggctgggc ggactgaccc cacagtatct    720
   ggcgctcctg cagcaggcca cctcctccag caacctgggt gcgttcagcg gcattcaaca    780
   aatggcaggc atgaatgctt tacagttgca gaacctggcg acgctggctg ctgctgcagc    840
   tgcggcccag acctcagcca ccagcaccaa tgcaaaccct ctctctacca cgagcagcgc    900
   cctgggagcc ctcacgagtc ccgtggctgc ttcaaccccc aactccactg ctggtgcagc    960
   catgaactcc ttgacctctc tcgggactct gcaaggactg gctggagcca ctgttggact    1020
   gaataatatt aatgcactag cagttgctca aatgctctca ggtatggcgg ctctgaatgg    1080
   aggacttggc gccacaggct tgacgaatgg cacggctggc accatggacg ccctcaccca    1140
   ggcctactca ggaattcaac agtacgcagc cgccgcgctg cccactctgt acagccagag    1200
   cctgctgcag cagcagagcg ctgcaggcag ccagaaggaa ggtccagagg gggcaaacct    1260
   ctttatttac caccttccac aggaatttgg agaccagcac attctgcaga tgttcatgcc    1320
   ttttggaaat gttatctctg ctaaagtctt cattgacaaa cagaccaatc tgagcaagtg    1380
   ctttggtttt gttagctacg acaatccagt ctctgcacaa gctgctatcc aagctatgaa    1440
   tggctttcag atcggcatga aacgcttgaa ggtgcagctg aagcgttcca aaaacgacag    1500
   caaaccttac tgatcctaac cccagaggct ccctgctctc atttagcttt tcttaggaca    1560
   tcttcatgcc cgttagttca tcgtttgcct agcatgtccc tgtggcgtct caaaaaaaag    1620
   tttcatcgtc ccgtcattgt ttctgatgtc tttctgacct cacatcatat ttggttctcc    1680
   tactgacctt tgatctagtt tgacctttga aatttgcatg tgacctcatc tagctatgaa    1740
   ttctgggaag tcaatgtgaa aaacattgct gcattcatgc aagactgaaa tttattatta    1800
   gacaaattca ttatagaaaa aacctgtggc aaaaacgttt ctttcttatt ttttttcttt     1860
   tcctaaaaca gacttgaaag tattatacag ggattggcat tcttcccggt cactggtaac    1920
   aatagcaata tgtgtccagg gacacagaat gttggtttct aacagactac ttccaaaaac    1980
   agtttgagaa aaaaactgtc tgattttaag tctctagagg tctgtaatag tttttacatt    2040
   tttcaggcag tgtaaagttt tttgataagg ccattttagg tggctcactt tctcattaag    2100
   atatatatat agaaccactt tttgtagatt agtataagaa aaatatttac cctgttttgg    2160
   ggcaaatgct acctatttgt gtcacctttt gctgaactca cagttagaca atccatggtt    2220
   taatgcacat gaaattacct atattttata ctgtttcaat gtacaggaga aaggttactg    2280
   taaactgtgt tatgttggtg cttctgtgaa ttaagttgtg gtttcatcat gagtcttaat    2340
   gttctttgtt gataagacaa gtttagaatt ggtttactta atac                    2384
<210> 8
   <211> 490
   <212> PRT
   <213> Homo sapiens
<400> 8
   Met Asn Gly Ala Leu Asp His Ser Asp Gln Pro Asp Pro Asp Ala Ile
    1               5                  10                  15
   Lys Met Phe Val Gly Gln Ile Pro Arg Ser Trp Ser Glu Lys Glu Leu
               20                  25                  30
   Lys Glu Leu Phe Glu Pro Tyr Gly Ala Val Tyr Gln Ile Asn Val Leu
           35                  40                  45
   Arg Asp Arg Ser Gln Asn Pro Pro Gln Ser Lys Gly Cys Cys Phe Val
       50                  55                  60
   Thr Phe Tyr Thr Arg Lys Ala Ala Leu Glu Ala Gln Asn Ala Leu His
    65                 70                  75                  80
   Asn Ile Lys Thr Leu Pro Gly Met His His Pro Ile Gln Met Lys Pro
                    85                  90                  95
   Ala Asp Ser Glu Lys Ser Asn Ala Val Glu Asp Arg Lys Leu Phe Ile
               100                 105                 110
   Gly Met Val Ser Lys Lys Cys Asn Glu Asn Asp Ile Arg Val Met Phe
           115                 120                 125
   Ser Pro Phe Gly Gln Ile Glu Glu Cys Arg Ile Leu Arg Gly Pro Asp
       130                 135                 140
   Gly Leu Ser Arg Gly Cys Ala Phe Val Thr Phe Ser Thr Arg Ala Met
   145                 150                 155                 160
   Ala Gln Asn Ala Ile Lys Ala Met His Gln Ser Gln Thr Met Glu Gly
                   165                 170                 175
   Cys Ser Ser Pro Ile Val Val Lys Phe Ala Asp Thr Gln Lys Asp Lys
               180                 185                 190
   Glu Gln Arg Arg Leu Gln Gln Gln Leu Ala Gln Gln Met Gln Gln Leu
           195                 200                 205
   Asn Thr Ala Thr Trp Gly Asn Leu Thr Gly Leu Gly Gly Leu Thr Pro
       210                 215                 220
   Gln Tyr Leu Ala Leu Leu Gln Gln Ala Thr Ser Ser Ser Asn Leu Gly
   225                 230                 235                 240
   Ala Phe Ser Gly Ile Gln Gln Met Ala Gly Met Asn Ala Leu Gln Leu
                   245                 250                 255
   Gln Asn Leu Ala Thr Leu Ala Ala Ala Ala Ala Ala Ala Gln Thr Ser
               260                 265                 270
   Ala Thr Ser Thr Asn Ala Asn Pro Leu Ser Thr Thr Ser Ser Ala Leu
           275                 280                 285
   Gly Ala Leu Thr Ser Pro Val Ala Ala Ser Thr Pro Asn Ser Thr Ala
       290                 295                 300
   Gly Ala Ala Met Asn Ser Leu Thr Ser Leu Gly Thr Leu Gln Gly Leu
   305                 310                 315                 320
   Ala Gly Ala Thr Val Gly Leu Asn Asn Ile Asn Ala Leu Ala Val Ala
                   325                 330                 335
   Gln Met Leu Ser Gly Met Ala Ala Leu Asn Gly Gly Leu Gly Ala Thr
               340                 345                 350
   Gly Leu Thr Asn Gly Thr Ala Gly Thr Met Asp Ala Leu Thr Gln Ala
           355                 360                 365
   Tyr Ser Gly Ile Gln Gln Tyr Ala Ala Ala Ala Leu Pro Thr Leu Tyr
       370                 375                 380
   Ser Gln Ser Leu Leu Gln Gln Gln Ser Ala Ala Gly Ser Gln Lys Glu
   385                 390                 395                 400
   Gly Pro Glu Gly Ala Asn Leu Phe Ile Tyr His Leu Pro Gln Glu Phe
                   405                 410                 415
   Gly Asp Gln His Ile Leu Gln Met Phe Met Pro Phe Gly Asn Val Ile
               420                 425                 430
   Ser Ala Lys Val Phe Ile Asp Lys Gln Thr Asn Leu Ser Lys Cys Phe
           435                 440                 445
   Gly Phe Val Ser Tyr Asp Asn Pro Val Ser Ala Gln Ala Ala Ile Gln
       450                 455                 460
   Ala Met Asn Gly Phe Gln Ile Gly Met Lys Arg Leu Lys Val Gln Leu
   465                 470                 475                 480
   Lys Arg Ser Lys Asn Asp Ser Lys Pro Tyr
                   485                 490
<210> 9
   <211> 419
   <212> DNA
   <213> Homo sapiens
<400> 9
   tttttttttt tttttttcag catagtgaac gttaatattt ttatttcttt tgtaatgaag 60
   agtacaaatg gttgggagca ggacatcaca ggaggaggaa agatagcgcc atctctgcag 120
   aagaactcct gagccacaca cagaaggaaa gttgatcccc agggcagcct ttcccaccaa 180
   aaaaatcagg cccaatccag gagagtttgc cagtagctcc ccagggttcc agggtgtctg 240
   ccagccttcc taggaatcgt gggcaggctt ctaggtgcca gtgactcaaa ctcctttttc 300
   cacttcccag ttcaacctgg tcactctcat ccccacaagt tcccaatctg aatcccattc 360
   tctgaccatt ctctgcttcc ttgtttttaa tctcatttga gagtgatcct cacgggttc 419
<210> 10
   <211> 762
   <212> DNA
   <213> Homo sapiens
<400> 10
   gccgggagga gcggcgcgcg ctcggacctc tcccgccctg ctcgttcgct ctccagcttg 60
   ggatggccgg ctacctgcgg gtcgtgcgct cgctctgcag agcctcaggc tcgcggccgg 120
   cctgggcgcc ggcggccctg acagccccca cctcgcaaga gcagccgcgg cgccactatg 180
   ccgacaaaag gatcaaggtg gcgaagccgt ggtggagatg gatggtgatg agatgacccg 240
   tattatctgg cagttcatca aggagaagct catcctgccc cacgtggaca tccagctaaa 300
   gtattttgac ctcgggctcc caaaccgtga ccagactgat gaccaggtca ccattgactc 360
   tgcactggcc acccagaagt acagtgtggc tgtcaagtgt gccaccatca cccctgatga 420
   ggcccgtgtg gaagagttca agctgaagaa gatgtggaaa agtcccaatg gaactatccg 480
   gaacatcctg ggggggactg tcttccggga gcccatcatc tgcaaaaaca tcccacgcct 540
   agtccctggc tggaccaagc ccatcaccat tggcaggcac gcccatggcg accagtacaa 600
   ggccacagac tttgtggcag accgggccgg cactttcaaa atggtcttca ccccaaaaga 660
   tggcagtggt gtcaaggagt gggaagtgta caacttcccc gcaggcgcgc gtgggcatgg 720
   gcatgtacma caccgacgag tccatctcag gttttgcgca ca 762
<210>. 11
   <211> 689
   <212> DNA
   <213> Homo sapiens
<400> 11
   aactccatct ttgaaaaaca tttaataatg taatgtgttt gtggtacagg gtgagtacag 60
   atgcacagga ggccataggg tttaggcaaa ggggagcaca aaagttgaag atgaggcgct 120
   gccatcaatg ctgggacttc aggccaaggg caggaactga ggaagccaca agggaggaca 180
   ttttctgcag ttgctgaacc agtagcaact aggtcctgag aaagccctct ctcgtggaag 240
   aataacagcc aggcgggaaa gcttttcatc ctgcaaagct ggggaagaag attcttcctt 300
   aaattgtcat ctgcacttca gctcaggaat cctgttggct gaagtccaga gtgtcctttc 360
   tgattcctga agtagatgaa cagcccggcc ccaaggaaga gcaggcccag cacaaagccc 420
   ccgactccac tcagcatctt gctctgtgca gattcagacc gtgcactcca ttccactgtg 480
   agagggctcg ttacgcttgg gtgctccact tggcaggtgt aaacctctcc actccgagga 540
   actgtttcta gcatcaccag ggtctggaat gtccagtctc cattctggat caggcgcgtg 600
   gacaccaccc cagccttctc ttcctgcccg ttccggaacc agctgacttc aatgctgcct 660
   ggatagaaac cactcacaga gcagaccag 689
<210> 12
   <211> 1959
   <212> DNA
   <213> Homo sapiens
<400> 12
   gggagcacgc gggagtccgg ctgcccaccc atggcagggt ctaccagaag ggccacgagc     60
   tggtgttggc caatattgct gaaagtgatg ctggtgtcta cacctgccac gcggccaacc    120
   tggctggtca gcggagacag gatgtcaaca tcactgtggc cactgtgccc tcctggctga    180
   agaagcccca agacagccag ctggaggagg gcaaacccgg ctacttggat tgcctgaccc    240
   aggccacacc aaaacctaca gttgtctggt acagaaacca gatgctcatc tcagaggact    300
   cacggttcga ggtgttcctg gcaaaggctc agggcttgga ggagggagtg gcagagaccc    360
   tggtacttgt gaagagcctg cagagcaagg atgagcagca gcagctggac ttccggaggg    420
   agttggagat gtttgggaag ctgaaccacg ccaacgtggt gcggctcctg ggcctgtgcc    480
   gggaggctga gccccactac atggtgctgg aatatgtgga tctgggagac ctcaagcagt    540
   tcctgaggat ttccaagagc aaggatgaaa aattgaagtc acagcccctc agcaccaagc    600
   agaaggtggc cctatgcacc caggtagccc tgggcatgga gcacctgtcc aacaaccgct    660
   ttgtgcataa ggacttggct gcgcgtaact gcctggtcag tgcccagaga caagtgaagg    720
   tgtctgccct gggcctcagc aaggatgtgt acaacagtga gtactaccac ttccgccagg    780
   cctgggtgcc gctgcgctgg atgtcccccg aggccatcct ggagggtgac ttctctacca    840
   agtctgatgt ctgggccttc ggtgtgctga tgtgggaagt gtttacacat ggagagatgc    900
   cccatggtgg gcaggcagat gatgaagtac tggcagattt gcaggctggg aaggctagac    960
   ttcctcagcc cgagggctgc ccttccaaac tctatcggct gatgcagcgc tgctgggccc   1020
   tcagccccaa ggaccggccc tccttcagtg agattgccag cgccctggga gacagcaccg   1080
   tggacagcaa gccgtgagga gggagcccgc tcaggatggc ctgggcaggg gaggacatct   1140
   ctagagggaa gctcacagca tgatgggcaa gatccctgtc ctcctgggcc ctgaggcccc   1200
   tgccctagtg caacaggcat tgctgaggtc tgagcagggc ctggcctttc ctcctcttcc   1260
   tcaccctcat cctttgggag gctgacttgg acccaaactg ggcgactagg gctttgagct   1320
   gggcagtttt ccctgccacc tcttcctcta tcagggacag tgtgggtgcc acaggtaacc   1380
   ccaatttctg gccttcaact tctccccttg accgggtcca actctgccac tcatctgcca   1440
   actttgcctg gggagggcta ggcttgggat gagctgggtt tgtggggagt tccttaatat   1500
   tctcaagttc tgggcacaca gggttaatga gtctcttggc ccactggtcc cacttggggg   1560
   tctagaccag gattatagag gacacagcaa gtgagtcctc cccactctgg gcttgtgcac   1620
   actgacccag acccacgtct tccccaccct tctctccttt cctcatccta agtgcctggc   1680
   agatgaagga gttttcagga gcttttgaca ctatataaac cgcccttttt gtatgcacca   1740
   cgggcggctt ttatatgtaa ttgcagcgtg gggtgggtgg gcatgggagg taggggtggg   1800
   ccctggagat gaggagggtg ggccatcctt accccacact tttattgttg tcgttttttg   1860
   tttgttttgt ttttttgttt ttgtttttgt ttttacactc gctgctctca ataaataagc   1920
   cttttttaca aaaaaaaaaa aaaaaaaaaa aaaaaaaaa                          1959
<210> 13
   <211> 364
   <212> PRT
   <213> Homo sapiens
<400> 13
   Glu His Ala Gly Val Arg Leu Pro Thr His Gly Arg Val Tyr Gln Lys
   1               5                   10                  15
   Gly His Glu Leu Val Leu Ala Asn Ile Ala Glu Ser Asp Ala Gly Val
               20                  25                  30
   Tyr Thr Cys His Ala Ala Asn Leu Ala Gly Gln Arg Arg Gln Asp Val
           35                  40                  45
   Asn Ile Thr Val Ala Thr Val Pro Ser Trp Leu Lys Lys Pro Gln Asp
       50                  55                  60
   Ser Gln Leu Glu Glu Gly Lys Pro Gly Tyr Leu Asp Cys Leu Thr Gln
   65                  70                  75                  80
   Ala Thr Pro Lys Pro Thr Val Val Trp Tyr Arg Asn Gln Met Leu Ile
                   85                  90                  95
   Ser Glu Asp Ser Arg Phe Glu Val Phe Leu Ala Lys Ala Gln Gly Leu
               100                 105                 110
   Glu Glu Gly Val Ala Glu Thr Leu Val Leu Val Lys Ser Leu Gln Ser
           115                 120                 125
   Lys Asp Glu Gln Gln Gln Leu Asp Phe Arg Arg Glu Leu Glu Met Phe
       130                 135                 140
   Gly Lys Leu Asn His Ala Asn Val Val Arg Leu Leu Gly Leu Cys Arg
   145                 150                 155                 160
   Glu Ala Glu Pro His Tyr Met Val Leu Glu Tyr Val Asp Leu Gly Asp
                   165                 170                 175
   Leu Lys Gln Phe Leu Arg Ile Ser Lys Ser Lys Asp Glu Lys Leu Lys
               180                 185                 190
   Ser Gln Pro Leu Ser Thr Lys Gln Lys Val Ala Leu Cys Thr Gln Val
   195 200 205
   Ala Leu Gly Met Glu His Leu Ser Asn Asn Arg Phe Val His Lys Asp
   210 215 220
   Leu Ala Ala Arg Asn Cys Leu Val Ser Ala Gln Arg Gln Val Lys Val
   225 230 235 240
   Ser Ala Leu Gly Leu Ser Lys Asp Val Tyr Asn Ser Glu Tyr Tyr His
   245 250 255
   Phe Arg Gln Ala Trp Val Pro Leu Arg Trp Met Ser Pro Glu Ala Ile
   260 265 270
   Leu Glu Gly Asp Phe Ser Thr Lys Ser Asp Val Trp Ala Phe Gly Val
   275 280 285
   Leu Met Trp Glu Val Phe Thr His Gly Glu Met Pro His Gly Gly Gln
   290 295 300
   Ala Asp Asp Glu Val Leu Ala Asp Leu Gln Ala Gly Lys Ala Arg Leu
   305 310 315 320
   Pro Gln Pro Glu Gly Cys Pro Ser Lys Leu Tyr Arg Leu Met Gln Arg
   325 330 335
   Cys Trp Ala Leu Ser Pro Lys Asp Arg Pro Ser Phe Ser Glu Ile Ala
   340 345 350
   Ser Ala Leu Gly Asp Ser Thr Val Asp Ser Lys Pro
   355 360
<210> 14
   <211> 430
   <212> DNA
   <213> Homo sapiens
<400> 14
   ggttgatgtt tcaacaaagt agttttaagt tccatccatt cagattttaa gcattttgat 60
   ttatttaaaa agggattgtt catagaaaaa aattactttg aacagtatac aggactggtg 120
   gagattggct atttcacaac atcagacagt acaatcagta tctgccatat ggctggtctc 180
   tgtagacgcc ctttgctgtc ctcgctgaag gtgccttgtg tcttgagtta gtccactctt 240
   cttgcccgta ggaatcataa gtctcctcac tgagtccatg tccgtaatca tagtaatcag 300
   caccactttg ggctggggtg ctatagctgt tatcatagga atcataactc tgttcatcat 360
   aagcagtgcc atatccatca tcatagtcat attctccata agtctcttgg tgtcgggggt 420
   ggcggtggaa 430
<210> 15
   <211> 616
   <212> DNA
   <213> Homo sapiens
<400> 15
   ggtatgatga ccaaacataa aaagtgtttt ataattgttg gtgttttaat aacaactaat      60
   attattactc tgatagttaa actaactcga gattctcaga gtttatgccc ctatgattgg     120
   attggtttcc aaaacaaatg ctattatttc tctaaagaag aaggagattg gaattcaagt     180
   aaatacaact gttccactca acatgccgac ctaactataa ttgacaacat agaagaaatg     240
   aattttctta ggcggtataa atgcagttct gatcactgga ttggactgaa gatggcaaaa    300
   aatcgaacag gacaatgggt agatggagct acatttacca aatcgtttgg catgagaggg     360
   agtgaaggat gtgcctacct cagcgatgat ggtgcagcaa cagctagatg ttacaccgaa     420
   agaaaatgga tttgcaggaa aagaatacac taagttaatg tctaagataa tggggaaaat     480
   agaaaataac attattaagt gtaaaaccag caaagtactt ttttaattaa acaaagttcg     540
   agttttgtac ctgtctggtt aattctgctt acgtgtcagg ctacacataa aagccacttc     600
   aaagattggc aaaaaa                                                     616
<210> 16
   <211> 149
   <212> PRT
   <213> Homo sapiens
<400> 16
   Met Met Thr Lys His Lys Lys Cys Phe Ile Ile Val Gly Val Leu Ile
   1               5                   10                  15
   Thr Thr Asn Ile Ile Thr Leu Ile Val Lys Leu Thr Arg Asp Ser Gln
               20                  25                  30
   Ser Leu Cys Pro Tyr Asp Trp Ile Gly Phe Gln Asn Lys Cys Tyr Tyr
           35                  40                  45
   Phe Ser Lys Glu Glu Gly Asp Trp Asn Ser Ser Lys Tyr Asn Cys Ser
       50                  55                  60
   Thr Gln His Ala Asp Leu Thr Ile Ile Asp Asn Ile Glu Glu Met Asn
   65                  70                  75                  80
   Phe Leu Arg Arg Tyr Lys Cys Ser Ser Asp His Trp Ile Gly Leu Lys
                   85                  90                  95
   Met Ala Lys Asn Arg Thr Gly Gln Trp Val Asp Gly Ala Thr Phe Thr
               100                 105                 110
   Lys Ser Phe Gly Met Arg Gly Ser Glu Gly Cys Ala Tyr Leu Ser Asp
           115                 120                 125
   Asp Gly Ala Ala Thr Ala Arg Cys Tyr Thr Glu Arg Lys Trp Ile Cys
       130                 135                 140
   Arg Lys Arg Ile His
   145
<210> 17
   <211> 1973
   <212> DNA
   <213> Homo sapiens
<400> 17
   gggatattgg agtagcaaga ggctgggaag ccatcactta ccttgcactg agaaagaaga      60
   caaaggccag tatgcacagc tttcctccac tgctgctgct gctgttctgg ggtgtggtgt     120
   ctcacagctt cccagcgact ctagaaacac aagagcaaga tgtggactta gtccagaaat     180
   acctggaaaa atactacaac ctgaagaatg atgggaggca agttgaaaag cggagaaata     240
   gtggcccagt ggttgaaaaa ttgaagcaaa tgcaggaatt ctttgggctg aaagtgactg     300
   ggaaaccaga tgctgaaacc ctgaaggtga tgaagcagcc cagatgtgga gtgcctgatg     360
   tggctcagtt tgtcctcact gaggggaacc ctcgctggga gcaaacacat ctgacctaca     420
   ggattgaaaa ttacacgcca gatttgccaa gagcagatgt ggaccatgcc attgagaaag     480
   ccttccaact ctggagtaat gtcacacctc tgacattcac caaggtctct gagggtcaag     540
   cagacatcat gatatctttt gtcaggggag atcatcggga caactctcct tttgatggac     600
   ctggaggaaa tcttgctcat gcttttcaac caggcccagg tattggaggg gatgctcatt     660
   ttgatgaaga tgaaaggtgg accaacaatt tcagagagta caacttacat cgtgttgcgg     720
   ctcatgaact cggccattct cttggactct cccattctac tgatatcggg gctttgatgt     780
   accctagcta caccttcagt ggtgatgttc agctagctca ggatgacatt gatggcatcc     840
   aagccatata tggacgttcc caaaatcctg tccagcccat cggcccacaa accccaaaag     900
   cgtgtgacag taagctaacc tttgatgcta taactacgat tcggggagaa gtgatgttct     960
   ttaaagacag attctacatg cgcacaaatc ccttctaccc ggaagttgag ctcaatttca    1020
   tttctgtttt ctggccacaa ctgccaaatg ggcttgaagc tgcttacgaa tttgccgaca    1080
   gagatgaagt ccggtttttc aaagggaata agtactgggc tgttcaggga cagaatgtgc    1140
   tacacggata ccccaaggac atctacagct cctttggctt ccctagaact gtgaagcata    1200
   tcgatgctgc tctttctgag gaaaacactg gaaaaaccta cttctttgtt gctaacaaat    1260
   actggaggta tgatgaatat aaacgatcta tggatccagg ttatcccaaa atgatagcac    1320
   atgactttcc tggaattggc cacaaagttg atgcagtttt catgaaagat ggatttttct    1380
   atttctttca tggaacaaga caatacaaat ttgatcctaa aacgaagaga attttgactc    1440
   tccagaaagc taatagctgg ttcaactgca ggaaaaattg aacattacta atttgaatgg    1500
   aaaacacatg gtgtgagtcc aaagaaggtg ttttcctgaa gaactgtcta ttttctcagt    1560
   catttttaac ctctagagtc actgatacac agaatataat cttatttata cctcagtttg    1620
   catatttttt tactatttag aatgtagccc tttttgtact gatataattt agttccacaa     1680
   atggtgggta caaaaagtca agtttgtggc ttatggattc atataggcca gagttgcaaa    1740
   gatcttttcc agagtatgca actctgacgt tgatcccaga gagcagcttc agtgacaaac    1800
   atatcctttc aagacagaaa gagacaggag acatgagtct ttgccggagg aaaagcagct    1860
   caagaacaca tgtgcagtca ctggtgtcac cctggatagg caagggataa ctcttctaac    1920
   acaaaataag tgttttatgt ttggaataaa gtcaaccttg tttctactgt ttt           1973
<210> 18
   <211> 469
   <212> PRT
   <213> Homo sapiens
<400> 18
   Met His Ser Phe Pro Pro Leu Leu Leu Leu Leu Phe Trp Gly Val Val
   1               5                   10                  15
   Ser His Ser Phe Pro Ala Thr Leu Glu Thr Gln Glu Gln Asp Val Asp
               20                  25                  30
   Leu Val Gln Lys Tyr Leu Glu Lys Tyr Tyr Asn Leu Lys Asn Asp Gly
           35                  40                  45
   Arg Gln Val Glu Lys Arg Arg Asn Ser Gly Pro Val Val Glu Lys Leu
       50                  55                  60
   Lys Gln Met Gln Glu Phe Phe Gly Leu Lys Val Thr Gly Lys Pro Asp
   65                  70                  75                  80
   Ala Glu Thr Leu Lys Val Met Lys Gln Pro Arg Cys Gly Val Pro Asp
                   85                  90                  95
   Val Ala Gln Phe Val Leu Thr Glu Gly Asn Pro Arg Trp Glu Gln Thr
               100                 105                 110
   His Leu Thr Tyr Arg Ile Glu Asn Tyr Thr Pro Asp Leu Pro Arg Ala
           115                 120                 125
   Asp Val Asp His Ala Ile Glu Lys Ala Phe Gln Leu Trp Ser Asn Val
       130                 135                 140
   Thr Pro Leu Thr Phe Thr Lys Val Ser Glu Gly Gln Ala Asp Ile Met
   145                 150                 155                 160
   Ile Ser Phe Val Arg Gly Asp His Arg Asp Asn Ser Pro Phe Asp Gly
                   165                 170                 175
   Pro Gly Gly Asn Leu Ala His Ala Phe Gln Pro Gly Pro Gly Ile Gly
               180                 185                 190
   Gly Asp Ala His Phe Asp Glu Asp Glu Arg Trp Thr Asn Asn Phe Arg
           195                 200                 205
   Glu Tyr Asn Leu His Arg Val Ala Ala His Glu Leu Gly His Ser Leu
       210                 215                 220
   Gly Leu Ser His Ser Thr Asp Ile Gly Ala Leu Met Tyr Pro Ser Tyr
   225                 230                 235                 240
   Thr Phe Ser Gly Asp Val Gln Leu Ala Gln Asp Asp Ile Asp Gly Ile
                   245                 250                 255
   Gln Ala Ile Tyr Gly Arg Ser Gln Asn Pro Val Gln Pro Ile Gly Pro
               260                 265                 270
   Gln Thr Pro Lys Ala Cys Asp Ser Lys Leu Thr Phe Asp Ala Ile Thr
           275                 280                 285
   Thr Ile Arg Gly Glu Val Met Phe Phe Lys Asp Arg Phe Tyr Met Arg
       290                 295                 300
   Thr Asn Pro Phe Tyr Pro Glu Val Glu Leu Asn Phe Ile Ser Val Phe
   305                 310                 315                 320
   Trp Pro Gln Leu Pro Asn Gly Leu Glu Ala Ala Tyr Glu Phe Ala Asp
                   325                 330                 335
   Arg Asp Glu Val Arg Phe Phe Lys Gly Asn Lys Tyr Trp Ala Val Gln
               340                 345                 350
   Gly Gln Asn Val Leu His Gly Tyr Pro Lys Asp Ile Tyr Ser Ser Phe
           355                 360                 365
   Gly Phe Pro Arg Thr Val Lys His Ile Asp Ala Ala Leu Ser Glu Glu
       370                 375                 380
   Asn Thr Gly Lys Thr Tyr Phe Phe Val Ala Asn Lys Tyr Trp Arg Tyr
   385                 390                 395                 400
   Asp Glu Tyr Lys Arg Ser Met Asp Pro Gly Tyr Pro Lys Met Ile Ala
                   405                 410                 415
   His Asp Phe Pro Gly Ile Gly His Lys Val Asp Ala Val Phe Met Lys
               420                 425                 430
   Asp Gly Phe Phe Tyr Phe Phe His Gly Thr Arg Gln Tyr Lys Phe Asp
           435                 440                 445
   Pro Lys Thr Lys Arg Ile Leu Thr Leu Gln Lys Ala Asn Ser Trp Phe
       450                 455                 460
   Asn Cys Arg Lys Asn
   465
<210> 19
   <211> 1141
   <212> DNA
   <213> Homo sapiens
<400> 19
   ccgccaggcg ctttctcgga cgccttgccc agcgggccgc ccgaccccct gcaccatgga     60
   ccccgctcgc cccctggggc tgtcgattct gctgcttttc ctgacggagg ctgcactggg     120
   cgatgctgct caggagccaa caggaaataa cgcggagatc tgtctcctgc ccctagacta     180
   cggaccctgc cgggccctac ttctccgtta ctactacgac aggtacacgc agagctgccg     240
   ccagttcctg tacgggggct gcgagggcaa cgccaacaat ttctacacct gggaggcttg     300
   cgacgatgct tgctggagga tagaaaaagt tcccaaagtt tgccggctgc aagtgagtgt     360
   ggacgaccag tgtgaggggt ccacagaaaa gtatttcttt aatctaagtt ccatgacatg     420
   tgaaaaattc ttttccggtg ggtgtcaccg gaaccggatt gagaacaggt ttccagatga     480
   agctacttgt atgggcttct gcgcaccaaa gaaaattcca tcattttgct acagtccaaa     540
   agatgaggga ctgtgctctg ccaatgtgac tcgctattat tttaatccaa gatacagaac     600
   ctgtgatgct ttcacctata ctggctgtgg agggaatgac aataactttg ttagcaggga     660
   ggattgcaaa cgtgcatgtg caaaagcttt gaaaaagaaa aagaagatgc caaagcttcg     720
   ctttgccagt agaatccgga aaattcggaa gaagcaattt taaacattct taatatgtca     780
   tcttgtttgt ctttatggct tatttgcctt tatggttgta tctgaagaat aatatgacag     840
   catgaggaaa caaatcattg gtgatttatt caccagtttt tattaataca agtcactttt     900
   taaaaaattt ggattttttt atatataact agctgctatt caaatgtgag tctaccattt     960
   ttaatttatg gttcaactgt ttgtgagact gaattcttgc aatgcataag atataaaagc    1020
   aaatatgact cactcatttc ttggggtcgt attcctgatt tcagaagagg atcataactg    1080
   aaacaacata agacaatata atcatgtgct tttaacatat ttgagaataa aaaggactag    1140
   c                                                                    1141
<210> 20
   <211> 22
   <212> DNA
   <213> Artificial
<220>
   <223> serine (or cysteine) proteinase inhibitor sense primer
<400> 20
   ccaaactttg aaagccaagg tg 22
<210> 21
   <211> 21
   <212> DNA
   <213> Artificial
<220>
   <223> serine (or cysteine) proteinase inhibitor antisense primer
<400> 21
   tgcacttcaa agaccagcag g 21
<210> 22
   <211> 21
   <212> DNA
   <213> Artificial
<220>
   <223> adrenomedullin sense primer
<900> 22
   aggaatagtc gcgcaagcat c 21
<210> 23
   <211> 22
   <212> DNA
   <213> Artificial
<220>
   <223> adrenomedullin antisense primer
<400> 23
   cacgcattgc acttttcctc tt 22
<210> 24
   <211> 21
   <212> DNA
   <213> Artificial
<220>
   <223> apolipoprotein D sense primer
<400> 24
   tgaagccacc ccagttaacc t 21
<210> 25
   <211> 23
   <212> DNA
   <213> Artificial
<220>
   <223> apolipoprotein D antisense primer
<400> 25
   ggtcaacttc ctttgtgcgt ggt 23
<210> 26
   <211> 22
   <212> DNA
   <213> Artificial
<220>
   <223> collagen, type XV, alpha 1 sense primer
<400> 26
   accaaaccct atttcaagtg cc 22
<210> 27
   <211> 21
   <212> DNA
   <213> Artificial
<220>
   <223> collagen, type XV, alpha 1 antisense primer
<400> 27
   tagttatcca caaggcggac g 21
<210> 28
   <211> 21
   <212> DNA
   <213> Artificial
<220>
   <223> CUG triplet repeat RNA-binding protein 2 sense primer
<400> 28
   atgtttgtcg gacagatccc c 21
<210> 29
   <211> 21
   <212> DNA
   <213> Artificial
<220>
   <223> CUG triplet repeat RNA-binding protein 2 antisense primer
<400> 29
   atgtgacaaa cgcacagcct c 21
<210> 30
   <211> 20
   <212> DNA
   <213> Artificial
<220>
   <223> dermatopontin sense primer
<400> 30
   atttgaaccg gcaaggcttc 20
<210> 31
   <211> 21
   <212> DNA
   <213> Artificial
<220>
   <223> dermatopontin antisense primer
<400> 31
   aagtggttgt tgctcctcgg a 21
<210> 32
   <211> 20
   <212> DNA
   <213> Artificial
<220>
   <223> isocitrate dehydrogenase sense primer
<400> 32
   tgccatccag aagaaatggc 20
<210> 33
   <211> 19
   <212> DNA
   <213> Artificial
<220>
   <223> isocitrate dehydrogenase antisense primer
<400> 33
   aggcaaagat gctggcgat 19
<210> 34
   <211> 22
   <212> DNA
   <213> Artificial
<220>
   <223> MHC Class 2 DR alpha sense primer
<400> 34
   tagctgtgga caaagccaac ct 22
<210> 35
   <211> 22
   <212> DNA
   <213> Artificial
<220>
   <223> MHC Class 2 DR alpha antisense primer
<400> 35
   ggcacacacc acgttctctg ta 22
<210> 36
   <211> 22
   <212> DNA
   <213> Artificial
<220>
   <223> protein tyrosine kinase 7 sense primer
<400> 36
   tggaaccaga gcgtacgact gt 22
<210> 37
   <211> 19
   <212> DNA
   <213> Artificial
<220>
   <223> protein tyrosine kinase 7 antisense primer
<400> 37
   tggctttgca gcgcttctt 19
<210> 38
   <211> 22
   <212> DNA
   <213> Artificial
<220>
   <223> Sam-68 like phosphotyrosine protein, T-STAR sense primer
<400> 38
   aggagaaggc aaggatgaag aa 22
<210> 39
   <211> 22
   <212> DNA
   <213> Artificial
<220>
   <223> Sam-68 like phosphotyrosine protein, T-STAR antisense primer
<400> 39
   tgttaactcc tggagctgtg ct 22
<210> 40
   <211> 22
   <212> DNA
   <213> Artificial
<220>
   <223> C-type lectin superfamily member 2 sense primer
<400> 40
   gcccctatga ttggattggt tt 22
<210> 41
   <211> 22
   <212> DNA
   <213> Artificial
<220>
   <223> C-type lectin superfamily member 2 antisense primer
<400> 41
   catccttcac tccctctcat gc 22
<210> 42
   <211> 25
   <212> DNA
   <213> Artificial
<220>
   <223> MMP (matrix metaroproteinase) 1 sense primer
<400> 42
   tgactctaga aacacaagag caaga 25
<210> 43
   <211> 25
   <212> DNA
   <213> Artificial
<220>
   <223> MMP (martix metaroproteinase) 1 antisense primer
<400> 43
   aaggttagct tactgtcaca cgctt 25
<210> 44
   <211> 17
   <212> DNA
   <213> Artificial
<220>
   <223> TFPI (tissue factor pathway inhibitor) 2 sense primer
<400> 44
   gtcgattctg cttttcc 17
<210> 45
   <211> 20
   <212> DNA
   <213> Artificial
<220>
   <223> TFPI (tissue factor pathway inhibitor) 2 antisense primer
<400> 45
   atggaatttt ctttggtgcg 20
<210> 46
   <211> 1045
   <212> DNA
   <213> Homo sapiens
<400> 46
   ctcccgagct ctactgactc ccaacagagc gcccaagcaa gaaaatggcc ataagtggag      60
   tccctgtgct aggatttttc atcatagctg tgctgatgag cgctcaggaa tcatgggcta     120
   tcaaagaaga acatgtgatc atccaggccg agttctatct gaatcctgac caatcaggcg     180
   agtttatgtt tgactttgat ggtgatgaga ttttccatgt ggatatggca aagaaggaga     240
   cggtctggcg gcttgaagaa tttggacgat ttgccagctt tgaggctcaa ggtgcattgg     300
   ccaacatagc tgtggacaaa gccaacctgg aaatcatgac aaagcgctcc aactatactc     360
   cgatcaccaa tgtacctcca gaggtaactg tgctcacgaa cagccctgtg gaactgagag     420
   agcccaacgt cctcatctgt ttcatcgaca agttcacccc accagtggtc aatgtcacgt     480
   ggcttcgaaa tggaaaacct gtcaccacag gagtgtcaga gacagtcttc ctgcccaggg     540
   aagaccacct tttccgcaag ttccactatc tccccttcct gccctcaact gaggacgttt     600
   acgactgcag ggtggagcac tggggcttgg actgagcctc ttctcaagca ctgggaggtt     660
   gatgctccaa gcccgtctcc agagacttac gagacgtggt gtgtgccctg gggctgactg     720
   tgggtctggt cgggcttcct ttgggaccct ctttcatcat ccacggcgag tgcgccaaaa     780
   gcattgccca gaaacccagg gggccctctg taaggcctgt gcgggggcag aacggtccct     840
   taaaagaaag aacctcgtgg gcaaaacctg gcgcaagacg tcatacaaag ggggggcata      900
   taacacgcgt gccccagaac gaccgcactc agggctcgcc gcctataaca ccccggtgtg     960
   gtacccccaa ggcgcgctaa aactaaacag cgcgcacata gtagctcagg cgatacacca     1020
   ctcacatata taatagcacc gctaa                                          1045
<210> 47
   <211> 18
   <212> DNA
   <213> Artificial
<220>
   <223> adrenomedullin primer for real time PCR
<400> 47
   ggtttccgtc gccctgat 18
<210> 48
   <211> 24
   <212> DNA
   <213> Artificial
<220>
   <223> adrenomedullin primer for real time PCR
<400> 48
   gagcccactt attccacttc tttc 24
<210> 49
   <211> 22
   <212> DNA
   <213> Artificial
<220>
   <223> matrix metaroproteinase 1 primer for real time PCR
<400> 49
   gatggacctg gaggaaatct tg 22
<210> 50
   <211> 24
   <212> DNA
   <213> Artificial
<220>
   <223> matrix metaroproteinase 1 primer for real time PCR
<400> 50
   ccgcaacacg atgtaagttg tact 24
<210> 51
   <211> 21
   <212> DNA
   <213> Artificial
<220>
   <223> tissue factor pathway inhibitor 2 primer for real time PCR
<400> 51
   ggcaacgcca acaatttcta c 21
<210> 52
   <211> 26
   <212> DNA
   <213> Artificial
<220>
   <223> tissue factor pathway inhibitor 2 primer for real time PCR
<400> 52
   caaactttgg gaactttttc tatcct 26
<210> 53
   <211> 23
   <212> DNA
   <213> Artificial
<220>
   <223> serine (or cysteine) proteinase inhibitor primer for real time PCR
<400> 53
   tgggtggaga ataacacaaa caa 23
<210> 54
   <211> 22
   <212> DNA
   <213> Artificial
<220>
   <223> serine (or cysteine) proteinase inhibitor primer for real time PCR
<400> 54
   ccagataagt ggcagcatca aa 22
<210> 55
   <211> 28
   <212> DNA
   <213> Artificial
<220>
   <223> protein tyrosine kinase 7 primer for real time PCR
<400> 55
   gggagttcct taatattctc aagttctg 28
<210> 56
   <211> 27
   <212> DNA
   <213> Artificial
<220>
   <223> protein tyrosine kinase 7 primer for real time PCR
<400> 56
   gctgtgtcct ctataatcct ggtctag 27
<210> 57
   <211> 20
   <212> DNA
   <213> Artificial
<220>
   <223> collagen, type XV, alpha 1 primer for real time PCR
<400> 57
   ccagcaaccc acatcagctt 20
<210> 58
   <211> 23
   <212> DNA
   <213> Artificial
<220>
   <223> collagen, type XV, alpha 1 primer for real time PCR
<400> 58
   atgcagagca ggcttctcat aat 23
<210> 59
   <211> 29
   <212> DNA
   <213> Artificial
<220>
   <223> Sam68-like phosphotyrosine protein primer for real time PCR
<400> 59
   tcacaacatc agacagtaca atcagtatc 29
<210> 60
   <211> 22
   <212> DNA
   <213> Artificial
<220>
   <223> Sam68-like phosphotyrosine protein primer for real time PCR
<400> 60
   acgggcaaga agagtggact aa 22
<210> 61
   <211> 23
   <212> DNA
   <213> Artificial
<220>
   <223> apolipoprotein D primer for real time PCR
<400> 61
   tgagaagatc ccaacaacct ttg 23
<210> 62
   <211> 24
   <212> DNA
   <213> Artificial
<220>
   <223> apolipoprotein D primer for real time PCR
<400> 62
   tgatctttcc gttttccatt agtg 24
<210> 63
   <211> 22
   <212> DNA
   <213> Artificial
<220>
   <223> major histocompatibility complex DR beta primer for real time PCR
<400> 63
   ggctgaaqtc cagagtgtcc tt 22
<210> 64
   <211> 18
   <212> DNA
   <213> Artificial
<220>
   <223> major histocompatibility complex DR beta primer for real time PCR
<400> 64
   gctgggcctg ctcttcct 18
<210> 65
   <211> 19
   <212> DNA
   <213> Artificial
<220>
   <223> major histocompatibility complex DR alpha primer for real time PCR
<400> 65
   gcccagggaa gaccacctt 19
<210> 66
   <211> 23
   <212> DNA
   <213> Artificial
<220>
   <223> major histocompatibility complex DR alpha primer for real time PCR
<400> 66
   cagtcgtaaa cgtcctcagt tga 23
<210> 67
   <211> 27
   <212> DNA
   <213> Artificial
<220>
   <223> C-type lectin primer for real time PCR
<400> 67
   atccattttc tttcggtgta acatcta 27
<210> 68
   <211> 23
   <212> DNA
   <213> Artificial
<220>
   <223> C-type lectin primer for real time PCR
<400> 68
   catgagaggg agtgaaggat gtg 23
<210> 69
   <211> 24
   <212> DNA
   <213> Artificial
<220>
   <223> CUG triplet repeat RNA-binding protein 2 primer for real time PCR
<400> 69
   catgaatgct ttacagttgc agaa 24
<210> 70
   <211> 19
   <212> DNA
   <213> Artificial
<220>
   <223> CUG triplet repeat RNA-binding protein 2 primer for real time PCR
<400> 70
   gcgctgctcg tggtagaga 19
<210> 71
   <211> 22
   <212> DNA
   <213> Artificial
<220>
   <223> INTEGRIN, ALPHA 6 (ITGA6), MRNA primer for real time PCR
<400> 71
   gatgacagtg ttccccgata cc 22
<210> 72
   <211> 20
   <212> DNA
   <213> Artificial
<220>
   <223> INTEGRIN, ALPHA 6 (ITGA6), MRNA primer for real time PCR
<400> 72
   tgtaagtcag ccacgccaaa 20
<210> 73
   <211> 21
   <212> DNA
   <213> Artificial
<220>
   <223> SOLUTE CARRIER FAMILY 20 (PHOSPHATE TRANSPORTER), MEMBER 1 primers for real time PCR
   <400> 73
   aatggagaag ctgacatggc c 21
<210> 74
   <211> 21
   <212> DNA
   <213> Artificial
<220>
   <223> SOLUTE CARRIER FAMILY 20 (PHOSPHATE TRANSPORTER), MEMBER 1 primer for real time PCR
<400> 74
   ccaaacccac agaccaacac a 21
<210> 75
   <211> 21
   DNA
   <213> Artificial
<220>
   <223> RIBONUCLEOTIDE REDUCTASE M2 POLYPEPTIDE (RRM2) primer for real time PCR
<400> 75
   tccaaggaca ttcagcactg g 21
<210> 76
   <211> 19
   <212> DNA
   <213> Artificial
<220>
   <223> RIBONUCLEOTIDE REDUCTASE M2 POLYPEPTIDE (RRM2), MRNA primer for real time PCR
<400> 76
   atcgacgcaa aagaaccgg 19
<210> 77
   <211> 22
   <212> DNA
   <213> Artificial
<220>
   <223> FOLLISTATIN (FST), TRANSCRIPT VARIANT FST317, MRNA primer for real time PCR
   <400> 77
   ggaagtccag taccaaggca ga 22
<210> 78
   <211> 19
   <212> DNA
   <213> Artificial
<220>
   <223> FOLLISTATIN (FST), TRANSCRIPT VARIANT FST317, MRNA primer for real time PCR
<400> 78 tggcattgtc actggcaca 19
<210> 79
   <211> 20
   <212> DNA
   <213> Artificial
<220>
   <223> SPROUTY (DROSOPHILA) HOMOLOG 2 (SPRY2), MRNA primer for real time PCR
<400> 79
   gccatccgaa acaccaatga 20
<210> 80
   <211> 22
   <212> DNA
   <213> Artificial
<220>
   <223> SPROUTY (DROSOPHILA) HOMOLOG 2 (SPRY2), MRNA primer for real time PCR
<400> 80
   tgccacagtc ctcacacctg ta 22
<210> 81
   <211> 21
   <212> DNA
   <213> Artificial
<220>
   <223> RAB3B, MEMBER RAS ONCOGENE FAMILY (RAB3B), MRNA primer for real time PCR
<400> 81
   atgctgatga cacgttcacc c 21
<210> 82
   <211> 22
   <212> DNA
   <213> Artificial
<220>
   <223> RAB3B, MEMBER RAS ONCOGENE FAMILY (RAB3B), MRNA primer for real time PCR
<400> 82
   ggcctgcctt acactgatgt tc 22
<210> 83
   <211> 21
   <212> DNA
   <213> Artificial
<220>
   <223> SOLUTE CARRIER FAMILY 2 (FACILITATED GLUCOSE TRANSPORTER) primer for real time PCR
<400> 83
   gccttttcgt taaccgcttt g 21
<210> 84
   <211> 19
   <212> DNA
   <213> Artificial
<220>
   <223> SOLUTE CARRIER FAMILY 2 (FACILITATED GLUCOSE TRANSPORTER) primer for real time PCR
<400> 84
   tcctcgttgc ggttgatga 19
<210> 85
   <211> 21
   <212> DNA
   <213> Artificial
<220>
   <223> INTERLEUKIN 13 RECEPTOR, ALPHA 2 (IL13RA2), MRNA primer for real time PCR,
   <400> 85
   tgaaggctat ttgaagtcgc c 21
<210> 86
   <211> 19
   <212> DNA
   <213> Artificial
<220>
   <223> INTERLEUKIN 13 RECEPTOR, ALPHA 2 (IL13RA2), MRNA primer for real time PCR
<400> 86
   cttcgcttca atgcccttg 19
<210> 87
   <211> 21
   <212> DNA
   <213> Artificial
<220>
   <223> SERINE/THREONINE KINASE 12 (STK12), MRNA primer for real time PCR
<400> 87
   tggaaacgtg tacttggctc g 21
<210> 88
   <211> 21
   <212> DNA
   <213> Artificial
<220>
   <223> SERINE/THREONINE KINASE 12 (STK12), MRNA primer for real time PCR
<400> 88
   accagccgaa gtcagcaatc t 21
<210> 89
   <211> 22
   <212> DNA
   <213> Artificial
<220>
   <223> MINICHROMOSOME MAINTENANCE DEFICIENT (S. CEREVISIAE) primer for real time PCR
<400> 89
   aagtttggcc tgactaccag ca 22
<210> 90
   <211> 22
   <212> DNA
   <213> Artificial
<220>
   <223> MINICHROMOSOME MAINTENANCE DEFICIENT (S. CEREVISIAE) primer for real time PCR
<400> 90
   ccagacgtgt ataccccaat gg 22
<210> 91
   <211> 22
   <212> DNA
   <213> Artificial
<220>
   <223> THYROID HORMONE RECEPTOR INTERACTOR 13 (TRIP13), MRNA primer for real time PCR
<400> 91
   cctgtgtaaa gcgttagccc ag 22
<210> 92
   <211> 22
   <212> DNA
   <213> Artificial
<220>
   <223> THYROID HORMONE RECEPTOR INTERACTOR 13 (TRIP13), MRNA primer for real time PCR
<400> 92 _
   gtggcccaat gtactgcttg at 22
<210> 93
   <211> 22
   <212> DNA
   <213> Artificial
<220>
   <223> KINESIN-LIKE 6 (MITOTIC CENTROMERE-ASSOCIATED KINESIN) primer for real time PCR
<400> 93
   ccagctttaa cgaagccatg at 22
<210> 94
   <211> 22
   <212> DNA
   <213> Artificial
<220>
   <223> KINESIN-LIKE 6 (MITOTIC CENTROMERE-ASSOCIATED KINESIN) primer for real time PCR
<400> 94
   gcatttacca gaacctgtcc ca 22
<210> 95
   <211> 22
   <212> DNA
   <213> Artificial
<220>
   <223> CYCLIN-DEPENDENT KINASE INHIBITOR 3 primer for real time PCR
<400> 95
   tcggtttatg tgctcttcca gg 22
<210> 96
   <211> 22
   <212> DNA
   <213> Artificial
<220>
   <223> CYCLIN-DEPENDENT KINASE INHIBITOR 3 primer for real time PCR
<400> 96
   atcctcttag gtctcgcagg ct 22
<210> 97
   <211> 22
   <212> DNA
   <213> Artificial
<220>
   <223> CHROMOSOME CONDENSATION PROTEIN G (HCAP-G), MRNA primer for real time PCR
<400> 97
   gctcgccaga aattcgagtc ta 22
<210> 98
   <211> 22
   <212> DNA
   <213> Artificial
<220>
   <223> CHROMOSOME CONDENSATION PROTEIN G (HCAP-G), MRNA primer for real time PCR
<400> 98
   catgatcact ttcagagtcg gc 22
<210> 99
   <211> 21
   <212> DNA
   <213> Artificial
<220>
   <223> CDC28 PROTEIN KINASE 1 (CKS1), MRNA primer for real time PCR
<400> 99
   gcccactacc caagaaacca a 21
<210> 100
   <211> 22
   <212> DNA
   <213> Artificial
<220>
   <223> CDC28 PROTEIN KINASE 1 (CKS1), MRNA primer for real time PCR
<400> 100
   cataaatccg caagtcacca ca 22
<210> 101
   <211> 20
   <212> DNA
   <213> Artificial
<220>
   <223> PROTEIN REGULATOR OF CYTOKINESIS 1 (PRC1), MRNA primer for real time PCR
<400> 101
   cccaagtgga attgatgcga 20
<210> 102
   <211> 22
   <212> DNA
   <213> Artificial
<220>
   <223> PROTEIN REGULATOR OF CYTOKINESIS 1 (PRC1), MRNA primer for real time PCR
<400> 102
   cccctcacac actgcttcat tt 22.
<210> 103
   <211> 21
   <212> DNA
   <213> Artificial
<220>
   <223> CELL DIVISION CYCLE 2, G1 TO S AND G2 TO M (CDC2), MRNA primer for real time PCR
<400> 103
   ctgattttgg ccttgccaga g 21
<210> 104
   <211> 22
   <212> DNA
   <213> Artificial
<220>
   <223> CELL DIVISION CYCLE 2, G1 TO S AND G2 TO M (CDC2), MRNA primer for real time PCR
<400> 104
   ggatgattca gtgccatttt gc 22
<210> 105
   <211> 21
   <212> DNA
   <213> Artificial
<220>
   <223> CDC20 (CELL DIVISION CYCLE 20, S. CEREVISIAE, HOMOLOG) primer for real time PCR
<900> 105
   acagttcgcg ttcgagagtg a 21
<210> 106
   <211> 21
   <212> DNA
   <213> Artificial
<220>
   <223> CDC20 (CELL DIVISION CYCLE 20, S. CEREVISIAE, HOMOLOG) primer for real time PCR
<400> 106
   ttccactgag ccgaaggatc t 21
<210> 107
   <211> 22
   <212> DNA
   <213> Artificial
<220>
   <223> LIKELY ORTHOLOG OF MATERNAL EMBRYONIC LEUCINE ZIPPER KINASE primer for real time PCR
<400> 107
   tggctctctc ccagtagcat tc 22
<210> 108
   <211> 21
   <212> DNA
   <213> Artificial
<220>
   <223> LIKELY ORTHOLOG OF MATERNAL EMBRYONIC LEUCINE ZIPPER KINASE primer for real time PCR
<400> 108
   tcacttgcgg tcacatcttc c 21
<210> 109
   <211> 22
   <212> DNA
   <213> Artificial
<220>
   <223> MINICHROMOSOME MAINTENANCE DEFICIENT (S. CEREVISIAE) 7 primer for real time PCR
<400> 109
   agagaacaca aggattgccc ag 22
<210> 110
   <211> 22
   <212> DNA
   <213> Artificial
<220>
   <223> MINICHROMOSOME MAINTENANCE DEFICIENT (S. CEREVISIAE) 7 primer for real time PCR
<400> 110
   atgacaggag ctgagacttg gc 22
<210> 111
   <211> 22
   <212> DNA
   <213> Artificial
<220>
   <223> CYCLIN A2 (CCNA2), MRNA primer for real time PCR
<400> 111
   ggcactgctg ctatgctgtt ag 22
<210> 112
   <211> 21
   <212> DNA
   <213> Artificial
<220>
   <223> CYCLIN A2 (CCNA2), MRNA primer for real time PCR
<400> 112
   tgaaggtcca tgagacaagg c 21
<210> 113
   <211> 19
   <212> DNA
   <213> Artificial
<220>
   <223> THYMIDINE KINASE 1, SOLUBLE (TK1), MRNA primer for real time PCR
<400> 113
   ggcgcaatga gctgcatta 19
<210> 114
   <211> 22
   <212> DNA
   <213> Artificial
<220>
   <223> THYMIDINE KINASE 1, SOLUBLE (TK1), MRNA primer for real time PCR
<400> 114
   aaatggcttc ctctggaagg tc 22
<210> 115
   <211> 21
   <212> DNA
   <213> Artificial
<220>
   <223> cyclin-dependent kinase inhibitor 2A (melanoma, p16, inhibits CDK 4) (CDKN2A), mRNA primer for real time PCR
<400> 115
   tccccgattg aaagaaccag a 21
<210> 116
   <211> 22
   <212> DNA
   <213> Artificial
<220>
   <223> cyclin-dependent kinase inhibitor 2A (melanoma, p16, inhibits CDK 4) (CDKN2A), mRNA primer for real time PCR
<400> 116
   aagagaagcc agtaaccccc ct 22
<210> 117
   <211> 22
   <212> DNA
   <213> Artificial
<220>
   <223> MHC Class 2 DR beta sense primer
<400> 117
   tgtgtatcct gcaaagaccc ag 22
<210> 118
   <211> 21
   <212> DNA
   <213> Artificial
<220>
   <223> MHC Class 2 DR beta antisense primer
<400> 118
   tcctgttggc tgaagtccag a 21
<210> 119
   <211> 24
   <212> DNA
   <213> Artificial
<220>
   <223> adrenomedullin probe for real time PCR
<400> 119
   acctgggttc gctcgccttc ctag 24
<210> 120
   <211> 28
   <212> DNA
   <213> Artificial
<220>
   <223> matrix metaroproteinase 1 probe for real time PCR
<400> 120
   tcatgctttt caaccaggcc caggtatt 28
<210> 121
   <211> 21
   <212> DNA
   <213> Artificial
<220>
   <223> tissue factor pathway inhibitor 2 probe for real time PCR
<400> 121
   ctgggaggct tgcgacgatg c 21
<210> 122
   <211> 28
   <212> DNA
   <213> Artificial
<220>
   <223> serine (or cysteine) proteinase inhibitor probe for real time PCR
<400> 122
   ctggtgaaag atttggtatc cccaaggg 28
<210> 123
   <211> 25
   <212> DNA
   <213> Artificial
<220>
   <223> protein tyrosine kinase 7 probe for real time PCR
<400> 123
   tctcttggcc cactggtccc acttg 25
<210> 124
   <211> 28
   <212> DNA
   <213> Artificial
<220>
   <223> collagen, type XV, alpha 1 probe for real time PCR
<400> 124
   tgcctccacc aaaccctatt tcaagtgc 28
<210> 125
   <211> 27
   <212> DNA
   <213> Artificial
<220>
   <223> Sam68-like phosphotyrosine protein probe for real time PCR
<400> 125
   ctctgtagac gccctttgct gtcctcg 27
<210> 126
   <211> 25
   <212> DNA.
   <213> Artificial
<220>
   <223> apolipoprotein D probe for real time PCR
<400> 126
   atggacgctg catccaggcc aacta 25
<210> 127
   <211> 25
   <212> DNA
   <213> Artificial
<220>
   <223> major histocompatibility complex DR beta probe for real time PCR
<400> 127
   cctgaagtag atgaacagcc cggcc 25
<210> 128
   <211> 28
   <212> DNA
   <213> Artificial
<220>
   <223> major histocompatibility complex DR alpha probe for real time PCR
<400> 128
   tccgcaagtt ccactatctc cccttcct 28
<210> 129
   <211>. 25
   <212> DNA
   <213> Artificial
<220>
   <223> C-type lectin probe for real time PCR
<400> 129
   ctgttgctgc accatcatcg ctgag 25
<210> 130
   <211> 25
   <212> DNA
   <213> Artificial
<220>
   <223> CUG triplet repeat RNA-binding protein 2 probe for real time PCR
<400> 130
   ctcagccacc agcaccaatg caaac 25

## Description

### Technical Field

The present description relates to detection and separation/distinguishment of mesenchymal stem cells; particularly, a gene marker for detecting mesenchymal stem cells and a polypeptide marker for detecting mesenchymal stem cells used for detecting, separating/distinguishing mesenchymal stem cells; and a method for detecting and distinguishing mesenchymal stem cells by using the markers.

### Background Art

Mesenchymal stem cells exist in the bone marrow, etc., of mammals, and are known as multipotent stem cells which differentiate into adipocytes, chondrocytes, and osteocytes. Due to their multipotency in differentiation, mesenchymal stem cells draw attention as xenograft, homograft and autograft materials for regenerative medicine of many tissues, such as bone, cartilage, tendon, muscle, fat, and periodontal tissue (Gene & Medicine, Vol. 4, No. 2 (2000) p58-61). A general statement as to the present condition and prospect of study of mesenchymal stem cells has been issued recently, and there is a report concerning collection and culture of mesenchymal stem cells (Experimental Medicine, Vol. 19, No. 3 (February issue) 2001, p350-356). Further, it has been reported that mesenchymal stem cells also exist in an adipose tissue (Tissue Engineering P. A. Zuk et al., Multilineage cells from human adipose tissue: implications for cell-based therapies. 7: 211-228, 2001).

In recent years, some patent applications relating to culture, differentiation, etc., of mesenchymal stem cells have been published. For instance, Published Japanese Translation of PCT International Publication No. 11-506610 discloses a composition and method for maintaining the viability of human mesenchymal precursor cells in a serum-free environment; Published Japanese Translation of PCT International Publication No. 10-512756 discloses a method comprising contacting the mesenchymal stem cells with a bioactive factor, such as osteoinductive factor, adjunct factor for differentiation, chondroinductive factor which comprise prostaglandin, ascorbic acid, collagenous extracellular matrix, etc. , in order to induce differentiation of mesenchymal stem cells; Japanese Laid-Open Patent Application No. 2000-217576 discloses a method for differentiating a multipotential mesenchymal stem cell into adipocytes, which includes incubation of the mulitpotential mesenchymal stem cell in the presence of prolactin or a substance with an equivalent effect, respectively.

The present inventors previously found that it is possible to proliferate mesenchymal stem cells remarkably rabidly while maintaining their differentiation capacity, by culturing mesenchymal stem cells in the presence of extracellular matrix of basement membrane, or in a medium containing a fibroblast growth factor (FGF), etc., and disclosed a culturing method capable of obtaining significantly larger amount of mesenchymal stem cells than a conventional culturing method (Japanese Laid-Open Patent Application No. 2003-52360). In addition, the present inventors disclosed a method for separating and collecting mesenchymal stem cells from oral tissues, in order to conduct separation and collection being safe for a collected parent body and easy for collecting, upon collecting mesenchymal stem cells (Japanese Laid-Open Patent Application No. 2003-52365).

Recently, with the development of regenerative medicine, mesenchymal stem cells draw attention as xenograft, homograft and autograf t materials for regenerative medicine of many tissues , suchasbone, cartilage, tendon, muscle, fat, periodontaltissue, because of their multipotency in differentiation. In order to use mesenchymal stem cells for regenerative medicine of tissues, it is necessary to collect the stem cells from body tissues first, to proliferate them, and to further differentiate and proliferate them to prepare a tissue. Mesenchymal stem cells exist in the bone marrow and periosteum, and it is necessary to develop a method for collecting mesenchymal stem cells from these tissues safely and easily for the practical use of these cells in tissue regenerative medicine. In addition, for the practical use of mesenchymal stem cells in tissue regenerative medicine, it is necessary to develop a technique for securing a sufficient amount of mesenchymal stem cells. In order to do this, it is important to develop a technique for culturing and proliferating the collected mesenchymal stem cells while maintaining their differentiation capacity.

As stated above, the present inventors have developed the method for conducting separation and collection being safe for a collected parent body and easy for collecting, upon collecting mesenchymal stem cells. Further, the present inventors have developed a method for explosively proliferating mesenchymal stem cells while maintaining their differentiation capacity by culturingmesenchymal stem cells in the presence of extracellular matrix of basement membrane, or in a medium containing a fibroblast growth factor (FGF), etc. However, for the purpose of practical use of the cultured and proliferated mesenchymal stem cells in regenerative medicine, it is necessary to confirm that the cultured cells are mesenchymal stem cells, and to develop a method for detecting and distinguishing the mesenchymal stem cells. As for mesenchymal stem cells, marker genes that characterize the cells have not been identified conventionally. Therefore, for the purpose of practical use of mesenchymal stem cells in regenerative medicine, the identification of marker genes that characterize mesenchymal stem cells, and the development of a method for detecting, and separating/distinguishing mesenchymal stem cells are important problems to be solved.

The present description provides a gene marker for detecting mesenchymal stem cells and a polypeptide marker for detecting mesenchymal stem cells used for detecting and distinguishing mesenchymal stem cells; a primer for PCR amplification for detecting the gene marker; and a method for detecting and distinguishing mesenchymal stem cells by using the markers and the primer.

In order to find a gene which can serve as a marker for detecting mesenchymal stem cells, the present inventors compared the expression of various genes in mesenchymal stem cells, and searched for a gene which can serve as a marker. As a result, the present inventors found that there was a difference between fibroblasts and mesenchymal stem cells in the expression levels of 13 genes shown in the sequence listing, and the genes were expressed specifically in mesenchymal stem cells, and that the genes could serve as a marker for detecting mesenchymal stem cells. The present invention has been thus completed. The present description comprises a probe for detecting mesenchymal stem cell marker genes, and a PCR primer for amplifying the genes in test cell upon detecting the mesenchymal stem cell genemarkers. Further, the present description comprises a polypeptide marker for detecting mesenchymal stem cells comprising a polypeptide wherein the mesenchymal stem cell marker gene of the present description is expressed, and an antibody for detecting the polypeptide marker, which specifically binds to the polypeptide marker. Still further, the present description comprises a method for distinguishing and separating mesenchymal stem cells using the probe for detecting mesenchymal stem cell marker genes, and the antibody which specifically binds to the polypeptide marker.

The process of the completion of the present description is as follows: among various stem cells, mesenchymal stem cells are capable of differentiating into many tissues such as bone, cartilage, tendon, fat, skeletal muscle, cardiac muscle, blood vessel, nerve, etc., and are significantly expected as cells for regenerative medicine. However, marker genes that characterize mesenchymal stem cells have not been identified. Therefore, the present inventors have conducted a keen search for identifying marker genes that characterize mesenchymal stem cells. In brief, the search comprises the following steps: proliferating mesenchymal stem cells with the method, previously developed by the present inventors, wherein mesenchymal stem cells derived from bone marrow are cultured in the presence of FGF, etc., thereby explosively proliferating the cells while maintaining their differentiation capacity; culturing the mesenchymal stem cells obtained by this method and human fibroblasts in the presence of FGF; extracting mRNA from the cultured cells; by using the mRNA as a template and a DNA chip (Incyte) as a probe, comparing the genes expressed in human-derived mesenchymal stem cells and fibroblasts by DNA microarray technology.

Among 9400 genes, 63 genes exhibited significantly high expression, and 141 genes exhibited significantly low expression in mesenchymal stem cells. Among them, 87 genes (29 genes exhibiting high expression and 58 genes exhibiting low expression inmesenchymal stem cells) exhibitedadifference, of three-fold or more, in expression levels. Total RNAs were extracted from mesenchymal stem cells derived from 3 individuals and fibroblasts derived from 3 individuals, and by using them as a template, the expression levels of the above-mentioned 87 genes were examined by semiquantitative RT-PCR. As a result, most genes exhibited no difference in the expression levels. In addition, there were many cases wherein differences in the expression were considered to be caused by individual differences, because differences in the expression levels were observed between mesenchymal stem cells, or fibroblasts, from different individuals.

There were 3 genes (tissue factor pathway inhibitor 2, major histocompatibility complex class2 DR beta 3, serine (or cysteine) proteinase inhibitor) whose high expression in mesenchymal stem cells was confirmed by RT-PCR, and 10 genes (matrix metalloprotease 1, collagen type XV alpha, CUG triplet repeat RNA binding protein, dermatopontin, protein tyrosine kinase 7, isocitrate dehydrogenase 2, Sam68-like phosphotyrosine protein, C-type lectin superfamily member 2, adrenomedullin, apolopoprotein D) whose low expression in mesenchymal stem cells was confirmed by RT-PCR. By measuring the expression levels of these 13 genes, it has become possible to identify mesenchymal stem cells, which are difficult to be determined, in a short time and at a low cost. In addition, as a result of gene search in the present invention, many genes exhibiting differences in the expression levels between mesenchymal stem cells, or fibroblasts, have been confirmed. These genes serve as a gene marker for detecting mesenchymal stem cells, and can be used for detecting mesenchymal stem cells as microarray, DNA chip or the like by constructing a probe for detecting genes.

### Disclosure of the Invention

The present invention provides a method for distinguishing a mesenchymal stem cell from fibroblasts *in vitro,* wherein expression of all of the following mesenchymal stem cell marker genes in a test cell are detected; apolipoprotein D (SEQ ID NO 4 in the sequence listing); collagen type XV alpha 1 (SEQ ID NO 6 in the sequence listing); CUG triplet repeat RNA-binding protein 2 (SEQ ID NO 7 in the sequence listing); matrix metalloprotease 1 (SEQ ID NO 17 in the sequence listing); Homo sapiens bone morphogenetic protein 4 (BMP4: Gen Bank Acc. No. NM_001202); Homo sapiens clone 24775 mRNA sequence (Gen Bank Acc. No.AA402981); matrix Gla protein (MGP: Gen Bank Acc. No.BF668572); proteoglycan 1, secretory granule (Gen Bank Acc. No.AV734015); Homo sapiens GS3955 protein (Gen Bank Acc. No. NM_021643); Homo sapiens dynein, cytoplasmic, intermediate polypeptide 1 (DNCI1: Gen Bank Acc. No. NM_004411); and, brain-derived neurotrophic factor (Gen Bank Acc. No. X60201).

The present description specifically comprises: a gene marker for detecting a mesenchymal stem cell which is a gene having a base sequence shown in SEQ ID NO: 1, 2, 4, 6, 7, 9, 10, 11, 12, 14, 15, 17, 19 or 46 in the sequence listing ("1"), a gene marker for detecting a mesenchymal stem cell which is a gene of INTEGRIN, ALPHA 6 (ITGA6), MRNA (NM_000210); a gene of SOLUTE CARRIER FAMILY 20 (PHOSPHATE TRANSPORTER), MEMBER 1 (NM_005415) ; a gene of RIBONUCLEOTIDE REDUCTASE M2 POLYPEPTIDE (RRM2), MRNA (NM_001034) ; a gene of FOLLISTATIN (FST) , TRANSCRIPT VARIANT FST317, MRNA (NM_006350) ; a geneof SPROUTY (DROSOPHILA) HOMOLOG 2 (SPRY2), MRNA (NM_005842); a gene of RAB3B, MEMBER RAS ONCOGENE FAMILY (RAB3B), MRNA (NM_002867) ; a gene of SOLUTE CARRIER FAMILY 2 (FACILITATEDGLUCOSE TRANSPORTER) (NM 006516); a gene of INTERLEUKIN 13 RECEPTOR, ALPHA 2 (IL13RA2), MRNA (NO_000640); a gene of SERINE/THREONINE KINASE 12 (STK12), MRNA (NM_004217); a gene of MINICHROMOSOME MAINTENANCE DEFICIENT (S. CEREVISIAE) 5 (CE) (NM_006739); a gene of THYROID HORMONE RECEPTOR INTERACTOR 13 (TRIP13), MRNA (NM_004237); a gene of KINESIN-LIKE 6 (MITOTIC CENTROMERE-ASSOCIATED KINESIN) (K) (NM_006845); a gene of CYCLIN-DEPENDENT KINASE INHIBITOR 3 (CDK2-ASSOCIATED DUAL (NM_005192): a gene of CHROMOSOME CONDENSATION PROTEIN G (HCAP-G), MRNA (NM_022346); a gene of CDC28 PROTEIN KINASE 1 (CKS1), MRNA (NM_001826) ; a gene of PROTEIN REGULATOR OF CYTOKINESIS 1 (PRC1), MRNA (NM_003981); a gene of CELL DIVISION CYCLE 2, G1 TOS AND G2 TOM (CDC2), MRNA (NM_001786); a gene of CDC20 (CELL DIVISION CYCLE 20, S. CEREVISIAE, HOMOLOG) (CD) (NM_001255); a gene of LIKELY ORTHOLOG OF MATERNAL EMBRYONIC LEUCINE ZIPPER KINAS (NM_014791); a gene of MINICHROMOSOME MAINTENANCE DEFICIENT (S. CEREVISIAE) 7 (M) (NM_005916); a gene of CYCLINA2 (CCNA2), MRNA (NM_001237) ; a gene of THYMIDINE KINASE 1, SOLUBLE (TK1), MRNA (M_003258); or a gene of cyclin-dependent kinase inhibitor 2A (melanoma, p16, inhibits CDK4) (CDKN2A), mRNA (NM_000077) ("2"), a gene marker for detecting a mesenchymal stem cell which is a gene of EGF-containing fibulin-like extracellular matrix protein 1 (NM_018894); a gene of Human insulin-like growth factor binding protein 5 (IGFBP5) mRNA (AU132011); a gene of Homo sapiens clone 24775 mRNA sequence (AA402981); proteoglycan 1, secretory granule (AV734015); a gene of insulin-like growth factor binding protein 5 (AA374325); a gene of solute carrier family 21 (organic anion transporter), member 3 (AF085224); agene of transglutaminase 2 (C polypeptide, protein-glutamine-gamma-glutamyl transferase) (AL552373); a gene of coagulation factor II (thrombin) receptor (M62424); a gene of plasminogen activator, urokinase (NM_002658); agent of tissue inhibitor of metalloproteinase 3 (Sorsby fundus dystrophy, pseudoinflammatory) (W96324); a gene of matrix Gla protein (BF668572); a gene of 8-cell CLL/lymphoma 1 (Z23022); a gene of CD74 antigen (invariant polypeptide of major histocompatibility complex, class II antigen-associated) (BG333618); a gene of adducin 3 (gamma) (AL135243); a gene of solute carrier family 16 (monocarboxylic acid transporters), member 4 (NM_004696) ; a gene of protein S (alpha) (NM_000313) ; a gene of phosphatidylinositol (4,5) bisphosphate 5-phosphatase homolog; phosphatidylinositol polyphosphate 5-phosphatase type IV (AF187891); a gene of progesterone membrane binding protein (BE858855); a gene of brain-derived neurotrophic factor (X60201); or a gene of apolipoprotein E (BF967316) ("3"), a gene marker for detecting a mesenchymal stem cell which is a gene of HOMO SAPIENS WNT INHIBITORY FACTOR-1 (WIF-1), MRNA(NM_007191); a gene of HOMO SAPIENS HYPOTHETICAL PROTEIN FLJ11175 (FLJ11175), MRNA (NM_018349); a gene of HOMO SAPIENS RHO GDP DISSOCIATION INHIBITOR (GDI) BETA (ARHGDIB), MRNA (NM_001175); a gene of HOMO SAPIENS POTASSIUM INTERMEDIATE/SMALL CONDUCTANCE CALCIUM-ACTIVATE (NM_021614); a gene of HOMO SAPIENS NUCLEAR FACTOR (ERYTHROID-DERIVED 2)-LIKE 3 (NFE2L3), MRNA (NM_004289); a gene of HOMO SAPIENS GS3955 PROTEIN (GS3955), MRNA (NM_021643); a gene of HOMO SAPIENS CDNA 3' END SIMILAR TO SIMILAR TO GLYCOPROTEIN MUC18 (AA302605); a gene of HOMO SAPIENS HYPOTHETICAL PROTEIN FLJ21841 (FLJ21841), MRNA (NM_024609); a gene of HOMO SAPIENS ZINC FINGER PROTEIN 185 (LIM DOMAIN) (ZNF185), MRNA (NM_007150); a gene of SC1 = PUTATIVE TRANS-ACTING FACTOR INVOLVED IN CELL CYCLE-CONTROL [HUMAN, MRNA, 24] (S53374); a gene of HOMO SAPIENS NADH DEHYDROGENASE (UBIQUINONE) 1 ALPHA SUBCOMPLEX, 8 (19K) (NM_014222); a gene of HOMO SAPIENS HOMOLOG OF YEAST MCM10; HYPOTHETICAL PROTEIN PRO2249 (PR02) (NO_018518); a gene of HOMO SAPIENS COLLAGEN, TYPE VII, ALPHA 1 (EPIDERMOLYSIS BULLOSA, DYSTRO (NM_000094): a gene of HOMO SAPIENS AUTOCRINE MOTILITY FACTOR RECEPTOR (AMFR), MRNA (NM_001144); a gene of HOMO SAPIENS CLONE 24421 MRNA SEQUENCE /CDS = UNKNOWN /GB = AF070641 /GI = 3283914 /UG = (AF070641); a gene of HOMO SAPIENS MRNA; a gene of CDNA DKFZP586E1621 (FROM CLONE DKFZP586E1621) /CDS = UNKNOWN /GB (AL080235) ("4"), a gene marker for detecting a mesenchymal stem cell which is a gene of HOMO SAPIENS RESERVED (KCNK12), MRNA (NM_022055); a gene of HOMO SAPIENS UBIQUITIN-CONJUGATING ENZYME E2C (UBE2C), MRNA (NM_007019); a gene of HOMO SAPIENS CDNA FLJ10517 FIS, CLONE NT2RP2000812 /CDS = (61,918) /GB = AK001379 /G (AK001379) ; a gene of HOMO SAPIENS CARTILAGE LINKING PROTEIN 1 (CRTL1), MRNA (NM_001884); a gene of HOMO SAPIENS MRNA; CDNA DKFZP434F2322 (FROM CLONE DKFZP434F2322) /CDS = UNKNOWN /GB (AL133105) ; a gene of HOMO SAPIENS, CLONE MGC:9549 IMAGE: 3857382, MRNA, COMPLETE CDS /CDS = (92,1285) /G (BC012453); a gene of HOMO SAPIENS HYPOTHETICAL PROTEIN DKFZP586J1119 (DKFZP586J1119), MRNA (NM_032270); a gene of HOMO SAPIENS GLIA MATURATION FACTOR, GAMMA (GMFG), MRNA (NM_004877); a gene of HOMO SAPIENS CDNA: FLJ23095 FIS, CLONE LNG07413 /CDS = UNKNOWN /GB = AK026748 /GI = 10 (AK026748); a gene of HOMO SAPIENS HYPOTHETICAL PROTEIN DKFZP762E1312 (DKFZP762E1312), MRNA (NM_018410); a gene of HOMO SAPIENS IROQUOIS-CLASS HOMEODOMAIN PROTEIN (IRX-2A), MRNA (NM_005853); or a gene of HOMO SAPIENS HYPOTHETICAL PROTEIN FLJ10540 (FLJ10540), MRNA (NM_018131) ("5") , a gene marker for detecting a mesenchymal stem cell which is a gene of HOMO SAPIENS MRNA; CDNA DKFZP434C1915 (FROM CLONE DKFZP434C1915); PARTIAL CDS /C (AL137698); a gene of HOMO SAPIENS MINICHROMOSOME MAINTENANCE DEFICIENT (S. CEREVISIAE) 7 (M) (NM_005916); a gene, of HOMO SAPIENS DYNEIN, CYTOPLASMIC, INTERMEDIATE POLYPEPTIDE 1 (DNCI1), M (NM_004411); a gene of HUMAN MRNA FOR PROTEIN GENE PRODUCT (PGP) 9.5. (X04741); a gene of HOMO SAPIENS RAD51-INTERACTING PROTEIN (PIR51), MRNA (NM_006479); a gene of HOMO SAPIENS BACULOVIRAL IAP REPEAT-CONTAINING 5 ISURVIVIN) (BIRC5), MR (NM_001168); a gene of HOMO SAPIENS UDP-N-ACETYL-ALPHA-D-GALACTOSAMINE:POLYPEPTIDE N-ACETYLGAL (NM_004482); a gene of HOMO SAPIENS CYCLIN B1 (CCNB1), MRNA (NM_031966); a gene of HOMO SAPIENS FLAP STRUCTURE-SPECIFIC ENDONUCLEASE 1 (FEN1) , MRNA (NM_004111); a gene of HOMO SAPIENS SERINE/THREONINE KINASE 15 (STK15), MRNA (NM_003600); a gene of HOMO SAPIENS MAD2 (MITOTIC ARREST DEFICIENT, YEAST, HOMOLOG)-LIKE 1 (MA) (NM_002358); a gene of HOMO SAPIENS NUCLEOLAR PROTEIN ANKT (ANKT), MRNA (NM_018454); a gene of HOMO SAPIENS HSPC150 PROTEIN SIMILAR TO UBIQUITIN-CONJUGATING ENZYME (H) (NM_014176) ; a gene of HOMO SAPIENS CYTOCHROME P450 RETINOID METABOLIZING PROTEIN (P450RAI-2), (NM_019885); a gene of HOMO SAPIENS NIMA (NEVER IN MITOSIS GENE A) -RELATED KINASE 2 (NEK2), MR (NM_002497); a gene of HOMO SAPIENS BONE MORPHOGENETIC PROTEIN 4 (BMP4), MRNA (NM_001202); a gene of HOMO SAPIENS CDNA FLJ10674 FIS, CLONE NT2RP2006436 /CDS = UNKNOWN /GB = AK001536 /GI (AK001536); or a gene of HOMO SAPIENS CENTROMERE PROTEIN A (17 KD) (CENPA), MRNA (NM_001809) ("6"), a gene marker for detecting a mesenchymal stem cell which is a gene of HOMO SAPIENS MRNA; CDNA DKFZP564P116 (FROM CLONE DKFZP564P116) /CDS = UNKNOWN /GB = A (AL049338); a gene of HOMO SAPIENS TTK PROTEIN KINASE (TTK), MRNA (NM_003318); a gene of HOMO SAPIENS KARYOPHERIN ALPHA 2 (RAG COHORT 1, IMPORTIN ALPHA 1) (KPNA (NM_002266); a gene of HOMO SAPIENS POLYMERASE (DNA DIRECTED), THETA (POLQ), MRNA (NM_006596); a gene of HOMO SAPIENS ETS VARIANT GENE 5 (ETS-RELATED MOLECULE) (ETV5), MRNA (NM_004454); a gene of HOMO SAPIENS TRANSFORMING, ACIDIC COILED-COIL CONTAINING PROTEIN 3 (TAC) (NM_006342); a gene of HOMO SAPIENS KINESIN-LIKE 1 (KNSL1), MRNA (NM_004523); a gene of HOMO SAPIENS CENTROMERE PROTEIN E (312 KD) (CENPE), MRNA (NM_001813); a gene of HOMO SAPIENS CDNA, 3' END /CLONE = IMAGE:1651289 /CLONE_END = 3'/GB = AI12 (AI123815) ; a gene of HOMO SAPIENS DISTAL-LESS HOMEO BOX 5 (DLX5), MRNA (NM_005221); a gene of HOMO SAPIENS V-MYB AVIAN MYELOBLASTOSIS VIRAL ONCOGENE HOMOLOG-LIKE 2 (NM_002466); or a gene of HOMO SAPIENS SERUM DEPRIVATION RESPONSE (PHOSPHATIDYLSERINE-BINDING PRO (NM_004657) ("7"), a gene marker for detecting a mesenchymal stem cell which is a gene of HOMO SAPIENS HYPOTHETICAL PROTEIN FLJ10604 <FLJ10604), MRNA (NM_018154); a gene of HOMO SAPIENS RIBONUCLEASE HI, LARGE SUBUNIT (RNASEHI), MRNA (NM_006397); a gene of HOMO SAPIENS ANILLIN (DROSOPHILA SCRAPS HOMOLOG), ACTIN BINDING PROTEIN (NM_018685); a gene of HOMO SAPIENS H4 HISTONE FAMILY, MEMBER G (H4FG), RNA (NM_003542); a gene of HOMO SAPIENS G PROTEIN-COUPLED RECEPTOR 37 (ENDOTHELIN RECEPTOR TYPE B- (NM_005302) HOMO SAPIENS UBIQUITIN CARRIER PROTEIN (E2-EPF), MRNA (NM_014501); a gene of HOMO SAPIENS, SIMILAR TO TUMOR DIFFERENTIALLY EXPRESSED 1, CLONE IMAGE:3639252, (BC007375); a gene of HOMO SAPIENS HYPOTHETICAL PROTEIN MGC2577 (MGC2577), MRNA (NM_031299); a gene of HOMO SAPIENS HYPOTHETICAL PROTEIN FLJ13912 (FLJ13912), MRNA (NM_022770); a gene of HOMO SAPIENS ANNEXIN A3 (ANXA3), MRNA (NM_005139); a gene of HOMO SAPIENS STATHMIN1/ONCOPROTEIN 18 (STMN1), MRNA (NM_005563) HOMO SAPIENS, SIMILAR TO RIBOSOMAL PROTEIN L39, CLONE MGC : 20168 IMAGE: 4555759, M (BC012328); or a gene of HOMO SAPIENS POLO (DROSOPHIA) -LIRE KINASE (PLK), MRNA (M_005030) ("8"), a gene marker for detecting a mesenchymal stem cell which is a gene of HOMO SAPIENS PROTEASOME (PROSOME, MACROPAIN) 26S SUBUNIT, ATPASE, 5 (PS) (NM_002805); a gene of HOMO SAPIENS EUKARYOTIC TRANSLATION INITIATION FACTOR 4 GAMMA, 2 (EIF4G) (NM_001418); a gene of HOMO SAPIENS DIHYDROPYRIMIDINASE-LIKE 3 (DPYSL3), MRNA) (NM_001387); a gene of HOMO SAPIENS ADAPTOR-RELATED PROTEIN COMPLEX 1, SIGMA 2 SUBUNIT (AP1S2) (NM_003916); a gene of HOMO SAPIENS PLECTIN 1, INTERMEDIATE FILAMENT BINDING PROTEIN, 500 KD (P) (M_000445); a gene of HOMO SAPIENS HETEROGENEOUS NUCLEAR RIBONUCLEOPROTEIN U (SCAFFOLD ATTAC (NM_031844): a gene of HOMO SAPIENS HYPOTHETICAL PROTEIN MGC5306 (MGC5306), MRNA (NM_024116); a gene of HOMO SAPIENS MHC CLASS I POLYPEPTIDE-RELATED SEQUENCE A (MICA), MRNA (NM_000247); a gene of HOMO SAPIENS PROTEIN ASSOCIATED WITH PRK1 (AWP1), MRNA (NM_019006); a gene of HOMO SAPIENS PROGESTERONE RECEPTOR MEMBRANE COMPONENT 2 (PGRMC2), MRNA (NM_006320) ; a gene of HOMO SAPIENS POLYGLUTAMINE BINDING PROTEIN 1 (PQBP1), MRNA (NM_005710); a gene of HOMO SAPIENS S-ADENOSYLMETHIONINE DECARBOXYLASE 1 (AMD1), MRNA (NM_001634); HOMO SAPIENS INTERFERON-INDUCED, HEPATITIS C-ASSOCIATED MICROTUBULAR AG (M_006417); a gene of HOMO SAPIENS PROTEIN KINASE, AMP-ACTIVATED, GAMMA 1 NON-CATALYTIC SUBUN (NM_002733); HOMO SAPIENS PROCOLLAGEN-PROLINE, 2-OXOGLUTARATE 4-DIOXYGENASE (PROLINE (NM_004199); a genes of HOMO SAPIENS ENDOTHELIAL DIFFERENTIATION, LYSOPHOSPHATIDIC ACID G-PROTE (NM_012152); or a gene of HOMO SAPIENS KIAA0127 GENE PRODUCT (KIAA0127), MRNA (M_014755) ("9"), a probe for detecting a mesenchymal stem cell marker gene having a DNA sequence which hybridizes with the marker gene for detecting a mesenchymal stem cell according to any one of "1" to "9" under a stringent condition ("10"), the probe for detecting a mesenchymal stem cell marker gene according to "10", which comprises whole or part of an antisense strand of the base sequence according to any one of "1" to "9" ("11"), a microarray or a DNA chip for detecting a mesenchymal stem cell marker gene, wherein at least one of the DNA according to "10" or "11" is immobilized ("12") , the microarray or the DNA chip for detecting a mesenchymal stem cell marker gene according to "12", wherein a probe for detecting two or more genes in a group of genes having a base sequence shown in SEQ ID NO: 1, 2, 4, 6, 7, 9, 10, 11, 12, 14, 15, 17, 19 or 46 in the sequence listing and a group of the genes according to any one of "2" to "9" is immobilized ("13"), a polypeptide marker for detecting a mesenchymal stem cell which is a polypeptide having an amino acid sequence shown in SEQ ID NO: 3, 5, 8, 13, 16 or 18 in the sequence listing ("14"), an antibody which is induced by using the polypeptide according to "14" and which specifically binds to the polypeptide ("15"), the antibody according to "15", which is a monoclonal antibody ("16"), and the antibody according to "15", which is a polyclonal antibody ("17").

The present description also comprises: a method for distinguishing a mesenchymal stem cell wherein expression of the mesenchymal stem cell marker gene according to any one of "1" to "9" in a test cell is detected ("18"), the method for distinguishing a mesenchymal stemcell according to "18", wherein the expression of the mesenchymal stem cell marker gene is detected by using Northern blotting ("19"), the method for distinguishing a mesenchymal stem cell according to "18", wherein the expression of the mesenchymal stem cell marker gene is detected by using the probe for detecting a mesenchymal stem cell marker gene according to "10" or "11" ("20"), the method for distinguishing a mesenchymal stem cell according to "18", wherein the expression of the mesenchymal stem cell marker gene is detected by using the microarray or the DNA chip for detecting a mesenchymal stem cell marker gene according to "12" or "13" ("21"), a method for distinguishing a mesenchymal stem cell, wherein the detection of the expression of the mesenchymal stem cell marker gene according to any one of "18" to "21" comprises use of quantitative or semiquantitative PCR ("22"), and the method for distinguishing a mesenchymal stem cell according to "22", wherein the use of quantitative or semiquantitative PCR is RT-PCR method ("23").

The present description further comprises: an RT-PCR primer for amplifying a mesenchymal stem cell marker gene which comprises a sense primer having a base sequence shown in SEQ ID NO: 20 in the sequence listing, and an antisense primer having a base sequence shown in SEQ ID NO: 21 in the sequence listing; a sense primer having a base sequence shown in SEQ ID NO: 22 in the sequence listing, and an antisense primer having a base sequence shown in SEQ ID NO: 23 in the sequence listing; a sense primer having a base sequence shown in SEQ ID NO: 24 in the sequence listing, and an antisense primer having a base sequence shown in SEQ ID NO: 25 in the sequence listing; a sense primer having a base sequence shown in SEQ ID NO: 26 in the sequence listing, and an antisense primer having a base sequence shown in SEQ ID NO: 27 in the sequence listing; a sense primer having a base sequence shown in SEQ ID NO: 28 in the sequence listing, and an antisense primer having a base sequence shown in SEQ ID NO: 29 in the sequence listing; a sense primer having a base sequence shown in SEQ ID NO: 30 in the sequence listing, and an antisense primer having a base sequence shown in SEQ IDNO: 31 in the sequence listing; a sense primer having a base sequence shown in SEQ ID NO: 32 in the sequence listing, and an antisense primer having a base sequence shown in SEQ ID NO: 33 in the sequence listing; a sense primer having a base sequence shown in SEQ ID NO: 34 in the sequence listing, and an antisense primer having a base sequence shown in SEQ ID NO: 35 in the sequence listing; a sense primer having a base sequence shown in SEQ ID NO: 36 in the sequence listing, and an antisense primer having a base sequence shown in SEQ ID NO: 37 in the sequence listing; a sense primer having a base sequence shown in SEQ ID NO: 38 in the sequence listing and an antisense primer having a base sequence shown in SEQ ID NO: 39 in the sequence listing; a sense primer having a base sequence shown in SEQ ID NO: 40 in the sequence listing, and an antisense primer having a base sequence shown in SEQ ID NO: 41 in the sequence listing; a sense primer having a base sequence shown in SEQ ID NO: 42 in the sequence listing, and an antisense primer having a base sequence shown in SEQ ID NO: 43 in the sequence listing; or a sense primer having a base sequence shown in SEQ ID NO: 44 in the sequence listing, and an antisense primer having a base sequence shown in SEQ ID NO: 45 in the sequence listing ("24"), a real time PCR primer for amplifying a mesenchymal stem cell marker gene which comprises a sense primer having a base sequence shown in SEQ ID NO: 47 in the sequence listing, and an antisense primer having a base sequence shown in SEQ ID NO: 48 in the sequence listing; a sense primer having a base sequence shown in SEQ ID NO: 49 in the sequence listing, and an antisense primer having a base sequence shown in SEQ ID NO: 50 in the sequence listing; a sense primer having a base sequence shown in SEQ ID NO: 51 in the sequence listing, and an antisense primer having a base sequence shown in SEQ ID NO: 52 in the sequence listing; a sense primer having a base sequence shown in SEQ ID NO: 53 in the sequence listing, and an antisense primer having a base sequence shown in SEQ ID NO: 54 in the sequence listing; a sense primer having a base sequence shown in SEQ ID NO: 55 in the sequence listing, and an antisense primer having a base sequence shown in SEQ ID NO: 56 in the sequence listing; a sense primer having a base sequence shown in SEQ ID NO: 57 in the sequence listing, and an antisense primer having a base sequence shown in SEQ ID NO: 58 in the sequence listing; a sense primer having a base sequence shown in SEQ ID NO: 59 in the sequence listing, and an antisense primer having a base sequence shown in SEQ ID NO: 60 in the sequence listing; a sense primer having a base sequence shown in SEQ ID NO: 61 in the sequence listing, and an antisense primer having a base sequence shown in SEQ ID NO: 62 in the sequence listing; a sense primer having a base sequence shown in SEQ ID NO: 63 in the sequence listing, and an antisense primer having a base sequence shown in SEQ ID NO: 64 in the sequence listing; a sense primer having a base sequence shown in SEQ ID NO: 65 in the sequence listing, and an antisense primer having a base sequence shown in SEQ ID NO: 66 in the sequence listing; a sense primer having a base sequence shown in SEQ ID NO: 67 in the sequence listing, and an antisense primer having a base sequence shown in SEQ ID NO: 68 in the sequence listing; or a sense primer having a base sequence shown in SEQ ID NO: 69 in the sequence listing, and an antisense primer having a base sequence shown in SEQ ID NO: 70 in the sequence listing ("25"), and an RT-PCR primer for amplifying a mesenchymal stem cell marker gene which comprises a sense primer having a base sequence shown in SEQ ID NO: 71 in the sequence listing, and an antisense primer having a base sequence shown in SEQ ID NO: 72 in the sequence listing; a sense primer having a base sequence shown in SEQ ID NO: 73 in the sequence listing, and an antisense primer having a base sequence shown in SEQ ID NO: 74 in the sequence listing; a sense primer having a base sequence shown in SEQ ID NO: 75 in the sequence listing, and an antisense primer having a base sequence shown in SEQ ID NO: 76 in the sequence listing; a sense primer having a base sequence shown in SEQ ID NO: 77 in the sequence listing, and an antisense primer having a base sequence shown in SEQ ID NO: 78 in the sequence listing; a sense primer having a base sequence shown in SEQ ID NO: 79 in the sequence listing, and an antisense primer having a base sequence shown in SEQ ID NO: 80 in the sequence listing; a sense primer having a base sequence shown in SEQ ID NO: 81 in the sequence listing, and an antisense primer having a base sequence shown in SEQ ID NO: 82 in the sequence listing; a sense primer having a base sequence shown in SEQ ID NO: 83 in the sequence listing, and an antisense primer having a base sequence shown in SEQ ID NO: 84 in the sequence listing; a sense primer having a base sequence shown in SEQ ID NO: 85 in the sequence listing, and an antisense primer having a base sequence shown in SEQ ID NO: 86 in the sequence listing; a sense primer having a base sequence shown in SEQ ID NO: 87 in the sequence listing, and an antisense primer having a base sequence shown in SEQ ID NO: 88 in the sequence listing; a sense primer having a base sequence shown in SEQ ID NO: 89 in the sequence listing, and an antisense primer having a base sequence shown in SEQ ID NO: 90 in the sequence listing; a sense primer having a base sequence shown in SEQ ID NO: 91 in the sequence listing, and an antisense primer having a base sequence shown in SEQ ID NO: 92 in the sequence listing; a sense primer having a base sequence shown in SEQ ID NO: 93 in the sequence listing, and an antisense primer having a base sequence shown in SEQ ID NO: 94 in the sequence listing; a sense primer having a base sequence shown in SEQ ID NO: 95 in the sequence listing, and an antisense primer having a base sequence shown in SEQ ID NO: 96 in the sequence listing; a sense primer having a base sequence shown in SEQ ID NO: 97 in the sequence listing, and an antisense primer having a base sequence shown in SEQ ID NO: 98 in the sequence listing; a sense primer having a base sequence shown in SEQ ID NO: 99 in the sequence listing, and an antisense primer having a base sequence shown in SEQ ID NO: 100 in the sequence listing; a sense primer having a base sequence shown in SEQ ID NO: 101 in the sequence listing, and an antisense primer having a base sequence shown in SEQ ID NO: 102 in the sequence listing; a sense primer having a base sequence shown in SEQ ID NO: 103 in the sequence listing, and an antisense primer having a base sequence shown in SEQ ID NO: 104 in the sequence listing; a sense primer having a base sequence shown in SEQ ID NO: 105 in the sequence listing, and an antisense primer having a base sequence shown in SEQ ID NO: 106 in the sequence listing; a sense primer having a base sequence shown in SEQ ID NO: 107 in the sequence listing, and an antisense primer having a base sequence shown in SEQ ID NO: 108 in the sequence listing; a sense primer having a base sequence shown in SEQ ID NO: 109 in the sequence listing, and an antisense primer having a base sequence shown in SEQ ID NO: 110 in the sequence listing; a sense primer having a base sequence shown in SEQ ID NO: 111 in the sequence listing, and an antisense primer having a base sequence shown in SEQ ID NO: 112 in the sequence listing; a sense primer having a base sequence shown in SEQ ID NO: 113 in the sequence listing, and an antisense primer having a base sequence shown in SEQ ID NO: 114 in the sequence listing; or a sense primer having a base sequence shown in SEQ ID NO: 115 in the sequence listing, and an antisense primer having a base sequence shown in SEQ ID NO: 116 in the sequence listing ("26"). The present description still further comprises: the method for distinguishing a mesenchymal stem cell according to "18", wherein a gene in a test cell is amplified by using at least one pair of primers comprising the sense primer and the antisense primer according to any one of "24" to "26", and the amplified gene is detected by using the probe for detecting a mesenchymal stem cell marker gene according to "10" or "11" ("27"), a method for distinguishing a mesenchymal stem cell, wherein expression of a mesenchymal stem cell marker polypeptide in a test cell is detected by using the antibody according to any one of "15" to "17" ("28"), a method for distinguishing a mesenchymal stem cell, wherein expression of one or more genes in a group of mesenchymal stem cell marker genes comprising SEQ ID NOs : 1, 11 and 19 in the sequence listing, and expression of one or more genes in a group of mesenchymal stem cell marker genes comprising SEQ ID NOs: 2, 4, 6, 7, 9, 10, 12, 14, 15 and 17 in the sequence listing, in a test cell, are evaluated in combination ("29"), a method for distinguishing and separating a mesenchymal stem cell, wherein expression of a marker gene for detecting a mesenchymal stem cell and/or a marker polypeptide for detecting a mesenchymal stem cell in a test cell is detected by using the probe for detecting a mesenchymal stem cell marker gene according to "10" or "11" and/or the antibody according to any one of "15" to "17", and the distinguished mesenchymal stemcell is separated ("30"), the method for distinguishing and separating a mesenchymal stem cell according to "30", wherein a mesenchymal stem cell in a test cell is labeled by a fluorescent antibody method using the antibody according to any one of "15" to "17", and the labeled mesenchymal stem cell is separated ("31"), and a kit for distinguishing a mesenchymal stem cell which comprises at least one of the probe for detecting a mesenchymal stem cell marker gene according to "10" or "11", the microarray or the DNA chip for detecting a mesenchymal stem cell marker gene according to "12" or "13" in which the probe is immobilized, and the antibody according to any one of "15" to "17" ("32").

### Brief Description of Drawings

Fig. 1 shows genes exhibiting considerable individual differences between human mesenchymal stem cells or human fibroblasts, according to the semiquantitative RT-PCR method in the Examples of the present invention.
Fig. 2 shows genes exhibiting high expression in MSC, according to the semiquantitative RT-PCR in the Examples of the present invention.
Fig. 3 shows genes exhibiting low expression in MSC, according to the semiquantitative RT-PCR in the Examples of the present invention.
Fig. 4 shows the result of confirmation of the expression levels of 13 genes confirmed to exhibit high/low expression in mesenchymal stem cells, by using semiquantitative RT-PCR with total RNA extracted from mesenchymal stem cells derived from 7 individuals and fibroblasts derived from 4 individuals as a template.
Fig. 5 shows the test result of the expression state of the marker gene of the present invention in mesenchymal stem cells and fibroblasts in the Examples of the present invention.
Fig. 6 shows the expression state of differential gene of each marker gene in mesenchymal stem cells and osteoblasts in the Examples of the present invention.
Fig. 7 shows the expression state of differential gene of each marker gene in mesenchymal stem cells and osteoblasts in the Examples of the present invention.
Fig. 8 shows the expression state of differential gene of each marker gene in mesenchymal stem cells and osteoblasts in the Examples of the present invention.
Fig. 9 shows the expression state of each marker gene in mesenchymal stem cells and human fibroblasts in the Examples of the present invention.
Fig. 10 shows the expression state of each marker gene in mesenchymal stem cells and human fibroblasts in the Examples of the present invention.
Fig. 11 shows the result of flow cytometric measurement of the expression of mesenchymal stem cell DR in human cells in the Examples of the present invention.
Fig. 12 shows the measurement result of the expression state of each gene in inducing differentiation of mesenchymal stem cells into osteoblasts in the Examples of the present invention.
Fig. 13 shows the changes in gene expression of each marker gene from day 4 to day 28 in the induction of osteogenic differentiation in the Examples of the present invention.
Fig. 14 shows the changes in gene expression of each marker gene from day 4 to day 28 in the induction of osteogenic differentiation in the Examples of the present invention.
Fig. 15 shows the changes in gene expression of each marker gene from day 4 to day 28 in the induction of osteogenic differentiation in the Examples of the present invention.
Fig. 16 shows the changes in gene expression of each marker gene from day 4 to day 28 in the induction of osteogenic differentiation in the Examples of the present invention.

### Best Mode of Carrying Out the Invention

The present invention comprises distinguishing mesenchymal stem cells by detecting the expression of a marker gene for detecting mesenchymal stem cells or a marker polypeptide for detecting mesenchymal stem cells, which are expressed specifically in mesenchymal stem cells. In the present description, a gene which can serve as a gene marker for detecting mesenchymal stem cells is a gene having a base sequence shown in SEQ ID NO: 1, 2, 4, 6, 7, 9, 10, 11, 12, 14, 15, 17 or 19 in the sequence listing.

Further, in the present description, a polypeptide which serves as a polypeptide marker for detecting mesenchymal stem cells is a polypeptide having an amino acid sequence shown in SEQ ID NO: 3, 5, 8, 13, 16 or 18 in the sequence listing.

The genes shown in SEQ ID NOs: 1, 2, 4, 6, 7, 9, 10, 11, 12, 14, 15, 17, 19 and 46 in the sequence listing are genes of [serine (or cysteine) proteinase inhibitor], [adrenomedullin], [apolopoprotein D], [collagen type XV alpha 1], [CUG triplet repeat RNA binding protein2], [dermatopontin], [isocitrate dehydrogenase 2], [major histocompatibility complex class2, DR beta 3], [protein tyrosine kinase 7], [Sam68-like phosphotyrosine protein], [C-type lectin superfamily member2], [matrix metalloprotease 1], [tissue factor pathway inhibitor 2], [major histocompatibility complex DR alpha], respectively.

The DNA sequence information of the marker genes for detecting mesenchymal stem cells of the present description can be approached in NCBI gene database with Accession Nos. AI133613 (SEQ ID NO: 1), NM_001124 (SEQ ID NO: 2), NM_001647 (SEQ IDNO: 4), L01697 (SEQIDNO: 6), U69546(SEQIDNO: 7), AW016451 (SEQ ID NO: 9), AL545953 (SEQ ID NO: 10), BF732822 (SEQ ID NO: 11), AL157486 (SEQ ID NO: 12), AA112001 (SEQ ID NO: 14), XM_006626 (SEQ ID NO: 15), NM_002421 (SEQ ID NO: 17), AL550357(SEQ ID NO: 19), and BF795929(SEQ ID NO: 46), respectively.

In addition, as genes which are confirmed in the present description that they exhibit higher expression in mesenchymal stem cells than in fibroblasts, the following is exemplified:

A gene marker for detecting mesenchymal stem cells which is: a gene of INTEGRIN, ALPHA 6 (ITGA6), MRNA (NM_000210); a gene of SOLUTE CARRIER FAMILY 20 (PHOSPHATE TRANSPORTER), MEMBER 1 (NM_005415); a gene of RIBONUCLEOTIDE REDUCTASE M2 POLYPEPTIDE (RRM2), MRNA (NM_001034); a gene of FOLLISTATIN (FST), TRANSCRIPT VARIANT FST317, MRNA (NM_006350) ; a gene of SPROUTY (DROSOPHILA) HOMOLOG 2 (SPRY2), MRNA (NM_005842); a gene of RAB3B, MEMBER RAS ONCOGENE FAMILY (RAB3B), MRNA (NM_002867) ; a gene of SOLUTE CARRIER FAMILY 2 (FACILITATEDGLUCOSETRANSPORTER) (NM_006516); a gene of INTERLEUKIN 13 RECEPTOR, ALPHA 2 (IL13RA2), MRNA (NM_000640) ; a gene of SERINE/THREONINE KINASE 12 (STK12), MRNA (NM_004217) ; a gene of MINICHROMOSOME MAINTENANCE DEFICIENT (S. CEREVISIAE) 5 (CE) (NM_006739); a gene of THYROID HORMONE RECEPTOR INTERACTOR 13 (TRIP13), MRNA (NM_004237); a gene of KINESIN-LIKE 6 (MITOTIC CENTROMERE-ASSOCIATED KINESIN) (K) (NM_006845); a gene of CYCLIN-DEPENDENT KINASE INHIBITOR 3 (CDK2-ASSOCIATED DUAL (NM_005192); a gene of CHROMOSOME CONDENSATION PROTEIN G (HCAP-G), MRNA (NM_022346); a gene of CDC28 PROTEIN KINASE 1 (CKS1), MRNA (NM_001826); a gene of PROTEIN REGULATOR OF CYTOKINESIS 1 (PRC1), MRNA (NM_003981) ; a gene of CELL DIVISION CYCLE 2, G1 TO S AND G2 TOM (CDC2), MRNA (NM_001786) ; a gene of CDC20 (CELL DIVISION CYCLE 20, S. CEREVISIAE, HOMOLOG) (CD) (NM_001255); a gene of LIKELY ORTHOLOG OF MATERNAL EMBRYONIC LEUCINE ZIPPER KINAS (NM_014791); a gene of MINICHROMOSOME MAINTENANCE DEFICIENT (S. CEREVISIAE) 7 (M) (NM_005916); a gene of CYCLINA2 (CCNA2), MRNA (NM_001237) ; a gene of THYMIDINE KINASE 1, SOLUBLE (TK1), MRNA (NM_003258); or a gene of cyclin-dependent kinase inhibitor 2A (melanoma, p16, inhibits CDK4) (CDKN2A), mRNA (M_000077).

A gene of EGF-containing fibulin-like extracellular matrix protein 1 (NK_018894) ; a gene of Human insulin-like growth factor binding protein 5 (IGFBP5) mRNA (AU132011); a gene of Homo sapiens clone24775mRNA sequence (AA402981); proteoglycan 1, secretory granule (AV734015); a gene of insulin-like growth factor binding protein 5 (AA374325); a gene of solute carrier family 21 (organic anion transporter), member 3 (AF085224) ; a gene of transglutaminase 2 (C polypeptide, protein-glutamine-gamma-glutamyl transferase) (AL552373); a gene of coagulation factor II (thrombin) receptor (M62424); a gene of plasminogen activator, urokinase (NM_002658); a gene of tissue inhibitor of metalloproteinase 3 (Sorsby fundus dystrophy, pseudoinflammatory) (W96324) ; a gene of matrix Gla protein (BF668572); a gene of B-cell CLL/lymphoma 1(Z23022); a gene of CD74 antigen (invariant polypeptide of major histocompatibility complex, class II antigen-associated) (BG333618); a gene of adducin 3 (gamma) (AL135243) ; a gene of solute carrier family 16 (monocarboxylic acid transporters), member 4 (NM_004696); a gene of protein S (alpha) (NM_000313); a gene of phosphatidylinositol(4,5)bisphosphate 5-phosphatase homolog;phosphatidylinositol polyphosphate 5-phosphatase type IV(AF187891) ; a gene of progesterone membrane binding protein (BE858855); a gene of brain-derived neurotrophic factor (X60201); or a gene of apolipoprotein E (BF967316).

A gene of HOMO SAPIENS WNT INHIBITORY FACTOR-1 (WIF-1), MRNA (NM_007191); a gene of HOMO SAPIENS HYPOTHETICAL PROTEIN FLJ11175 (FLJ11175), MRNA(NM_018349); a gene of HOMO SAPIENS RHO GDP DISSOCIATION INHIBITOR (GDI) BETA (ARHGDIB), MRNA (NM_001175) ; a gene of HOMO SAPIENS POTASSIUM INTERMEDIATE/SMALL CONDUCTANCE CALCIUM-ACTIVATE (NM_021614) ; a gene of HOMO SAPIENS NUCLEAR FACTOR (ERYTHROID-DERIVED 2)-LIKE 3 (NFE2L3), MRNA (NM_004289) ; a gene of HOMO SAPIENS GS3955 PROTEIN (GS3955), MRNA (NM_021643) ; a gene of HOMO SAPIENS CDNA 3' END SIMILAR TO SIMILAR TO GLYCOPROTEIN MUC18 (AA302605) ; a gene of HOMO SAPIENS HYPOTHETICAL PROTEIN FLJ21841 (FLJ21841), MRNA (NO_024609); a gene of HOMO SAPIENS ZINC FINGER PROTEIN 185 (LIM DOMAIN) (ZNF185), MRNA (NM_007150); a gene of SC1 = PUTATIVE TRANS-ACTING FACTOR INVOLVED IN CELL CYCLE CONTROL [HUMAN, MRNA, 24] (S53374) ; a gene of HOMO SAPIENS NADH DEHYDROGENASE (UBIQUINONE) 1 ALPHA SUBCOMPLEX, 8 (19K) (NM_014222) ; a gene of HOMO SAPIENS HOMOLOG OF YEAST MCM10; HYPOTHETICAL PROTEIN PR02249 (PRO2) (NM_018518) ; a gene of HOMO SAPIENS COLLAGEN, TYPE VII, ALPHA 1 (EPIDERMOLYSIS BULLOSA., DYSTRO (NM_000094); a gene of HOMO SAPIENS AUTOCRINE MOTILITY FACTOR RECEPTOR (AMFR), MRNA (NM_001144); a gene of HOMO SAPIENS CLONE 24421 RNA SEQUENCE /CDS = UNKNOWN /GB = AF070641 /GI = 3283914 /UG = (AF070641); a gene of HOMO SAPIENS MRNA; a gene of CDNA DKFZP586E1621 (FROM CLONE DKFZP586E1621) /CDS = UNKNOWN /GB(AL080235).

A gene of HOMO SAPIENS RESERVED (KCNK12), MRNA (NM_022055); a gene of HOMO SAPIENS UBIQUITIN-CONJUGATING ENZYME E2C (UBE2C), MRNA (NM_007019) ; a gene of HOMO SAPIENS CDNA FLJ10517 FIS, CLONE NT2RP2000812 /CDS = (61, 918) /GB = AK001379 /G(AK001379); a gene of HOMO SAPIENS CARTILAGE LINKING PROTEIN 1 (CRTL1), MRNA (NM_001884) ; a gene of HOMO SAPIENS MRNA; CDNA DKFZP434F2322 (FROM CLONE DKFZP434F2322) /CDS = UNKNOWN /GB (AL133105); a gene of HOMO SAPIENS, CLONE MGC:9549 IMAGE:3857382, MRNA, COMPLETE CDS /CDS = (92,1285) /G (BC012453); a gene of HOMO SAPIENS HYPOTHETICAL PROTEIN DKFZP586J1119 (DKFZP586J1119), MRNA (NM_032270); a gene of HOMO SAPIENS GLIA MATURATION FACTOR, GAMMA (GMFG) RNA (NO_004877); a gene of HOMO SAPIENS CDNA: FLJ23095 FIS, CLONE LNG07413 /CDS = UNKNOWN /GB = AK026748 /GI = 10 (AK026748); a gene of HOMO SAPIENS HYPOTHETICAL PROTEIN DKFZP762E132 (DKFZP762E1312), MRNA(NM_018410); a gene of HOMO SAPIENS IROQUOIS-CLASS HOMEODOMAIN PROTEIN (IRX-2A), MRNA (NM_005853); or a gene of HOMO SAPIENS HYPOTHETICAL PROTEIN FLJ10540 (FLJ10540), MRNA (NM_018131).

A gene of HOMO SAPIENS MRNA; CDNA DKFZP434C1915 (FROM CLONE DKFZP434C1915); PARTIAL CDS /C (AL137698) ; a gene of HOMO SAPIENS MINICHROMOSOME MAINTENANCE DEFICIENT (S. CEREVISIAE) 7 (M) (NM_005916); a gene of HOMO SAPIENS DYNEIN, CYTOPLASMIC, INTERMEDIATE POLYPEPTIDE 1 (DNCI1), M (NM_004411); a gene of HUMAN MRNA FOR PROTEIN GENE PRODUCT (PGP) 9.5. (X04741); a gene of HOMO SAPIENS RAD51-INTERACTING PROTEIN (PIR51), MRNA (NM_006479); a gene of HOMO SAPIENS BACULOVIRAL IAP REPEAT-CONTAINING 5 (SURVIVIN) (BIRC5), MR (NM_001168); a gene of HOMO SAPIENS UDP-N-ACETYL-ALPHA-D-GALACTOSAMINE: POLYPEPTIDE N-ACETYLGAL (NM_004482); a gene of HOMO SAPIENS CYCLIN B1 (CCNB1), MRNA (NM_031966); a gene of HOMO SAPIENS FLAP STRUCTURE-SPECIFIC ENDONUCLEASE 1 (FEN1), MRNA (NM_004111); a gene of HOMO SAPIENS SERINE/THREONINE KINASE 15 (STK15), MRNA (NM_003600); a gene of HOMO SAPIENS MAD2 (MITOTIC ARREST DEFICIENT, YEAST, HOMOLOG)-LIKE 1 (MA) (NM_002358); a gene of HOMO SAPIENS NUCLEOLAR PROTEIN ANKT (ANKT), MRNA (NM_018454); a gene of HOMO SAPIENS HSPC150 PROTEIN SIMILAR TO UBIQUITIN-CONJUGATING ENZYME (H) (NM_014176); a gene of HOMO SAPIENS CYTOCHROME P450 RETINOID METABOLIZING PROTEIN (P450RAI-2), (NM_019885); a gene of HOMO SAPIENS NIMA (NEVER IN MITOSIS GENE A)-RELATED KINASE 2 (NEK2), MR (NM_002497); a gene of HOMO SAPIENS BONE MORPHOGENETIC PROTEIN 4 (BMP4), MRNA (NM_001202); a gene of HOMO SAPIENS CDNA FLJ10674 FIS, CLONE NT2RP2006436 /CDS = UNKNOWN /GB = AK001536 /GI (AK001536); or a gene of HOMO SAPIENS CENTROMERE PROTEIN A (17 KD) (CENPA), MRNA (M_001809).

A gene of HOMO SAPIENS MRNA; CDNA DKFZP564P116 (FROM CLONE DKFZP564P116) ICDS = UNKNOWN /GB = A (AL049338); a gene of HOMO SAPIENS TTK PROTEIN KINASE (TTK), MRNA (NM_003318); a gene of HOMO SAPIENS KARYOPHERIN ALPHA 2 (RAG COHORT 1, IMPORTIN ALPHA 1) (KPNA (NM_002266); a gene of HOMO SAPIENS POLYMERASE (DNA DIRECTED), THETA (POLQ), MRNA (NM_006596) ; a gene of HOMO SAPIENS ETS VARIANT GENE 5 (ETS-RELATED MOLECULE) (ETV5), MRNA (NM_004454); a gene of HOMO SAPIENS TRANSFORMING, ACIDIC COILED-COIL CONTAINING PROTEIN 3 (TAC) (NM_006342); a gene of HOMO SAPIENS KINESIN-LIKE 1 (KNSL1), MRNA (M_004523); a gene of HOMO SAPIENS CENTROMERE PROTEIN E (312 KD) (CENPE), MRNA (NM_001813); a gene of HOMO SAPIENS CDNA, 3' END /CLONE = IMAGE:1651289 /CLONE_END = 3' /GB = AI12 (AI123815) ; a gene of HOMO SAPIENS DISTAL-LESS HOMEO BOX 5 (DLX5), MRNA (NM_005221) ; a gene of HOMO SAPIENS V-MYB AVIAN MYELOBLASTOSIS VIRAL ONCOGENE HOMOLOG-LIKE 2 (NM_002466) ; or a gene of HOMO SAPIENS SERUM DEPRIVATION RESPONSE (PHOSPHATIDYLSERINE-BINDING PRO (M_004657).

A gene of HOMO SAPIENS HYPOTHETICAL PROTEIN FLJ10604 (FLJ10604), MRNA (NM_018154); a gene of HOMO SAPIENS RIBONUCLEASE HI, LARGE SUBUNIT (RNASEHI), MRNA (NM_006397); a gene of HOMO SAPIENS ANILLIN (DROSOPHILA SCRAPS HOMOLOG), ACTIN BINDING PROTEIN (NM_018685) ; a gene of HOMO SAPIENS H4 HISTONE FAMILY, MEMBER G (H4FG), MRNA (NM_003542); a gene of HOMO SAPIENS G PROTEIN-COUPLED RECEPTOR 37 (ENDOTHELIN RECEPTOR TYPE B- (NM_005302) HOMO SAPIENS UBIQUITIN CARRIER PROTEIN (E2-EPF), MRNA (NM_014501); a gene of HOMO SAPIENS, SIMILAR TO TUMOR DIFFERENTIALLY EXPRESSED 1, CLONE IMAGE:3639252, (BC007375); a gene of HOMO SAPIENS HYPOTHETICAL PROTEIN MGC2577 (MGC2577), MRNA(NM_031299); a gene of HOMO SAPIENS HYPOTHETICAL PROTEIN FLJ13912 (FLJ13912), MRNA (NM_022770); a gene of HOMO SAPIENS ANNEXIN A3 (ANXA3), MRNA (NM_005139); a gene of HOMO SAPIENS STATHMIN1/ONCOPROTEIN18 (STMN1), MRNA (NM_005563) HOMO SAPIENS, SIMILAR TO RIBOSOMAL PROTEIN L39, CLONEMGC: 20168 IMAGE: 4555759, M (BC012328) ; a gene of HOMO SAPIENS POLO (DROSOPHIA) -LIKE KINASE (PLK), MRNA (NM_005030).

Further, as genes which are confirmed in the present description that they exhibit no or extremely lower expression in mesenchymal stem cells than in fibroblasts, the following is exemplified:

A gene marker for detecting mesenchymal stem cells which is: a gene of HOMO SAPIENS PROTEASOME (PROSOME, MACROPAIN) 26S SUBUNIT, ATPASE, 5 (PS) (NM_002805); a gene of HOMO SAPIENS EUKARYOTIC TRANSLATION INITIATION FACTOR 4 GAMMA, 2 (EIF4G) (NM_001418); a gene of HOMO SAPIENS DIHYDROPYRIMIDINASE-LIKE 3 (DPYSL3), MRNA) (NM_001387); a gene of HOMO SAPIENS ADAPTOR-RELATED PROTEIN COMPLEX 1, SIGMA 2 SUBUNIT (AP1S2) (NM_003916); a gene of HOMO SAPIENS PLECTIN 1, INTERMEDIATE FILAMENT BINDING PROTEIN, 500 KD (P) (NM_000445); a gene of HOMO SAPIENS HETEROGENEOUS NUCLEAR RIBONUCLEOPROTEIN U (SCAFFOLD ATTAC (NM_031844) ; a gene of HOMO SAPIENS HYPOTHETICAL PROTEIN MGC5306 (MGC5306), MRNA (NM_024116) ; a gene of HOMO SAPIENS MHC CLASS I POLYPEPTIDE-RELATED SEQUENCE A (MICA), MRNA (NM 000247); a gene of HOMO SAPIENS PROTEIN ASSOCIATED WITH PRK1 (AWP1), MRNA (NM_019006); a gene of HOMO SAPIENS PROGESTERONE RECEPTOR MEMBRANE COMPONENT 2 (PGRMC2), MRNA (NM_006320) ; a gene of HOMO SAPIENS POLYGLUTAMINE BINDING PROTEIN 1 (PQBP1), MRNA (NM_005710); a gene of HOMO SAPIENS S-ADENOSYLMETHIONINE DECARBOXYLASE 1 (AMD1), MRNA (NM_001634); HOMO SAPIENS INTERFERON-INDUCED, HEPATITIS C-ASSOCIATED MICROTUBULAR AG (NM_006417) ; a gene of HOMO SAPIENS PROTEIN KINASE, AMP-ACTIVATED, GAMMA 1 NON-CATALYTIC SUBUN (NM_002733); HOMO SAPIENS PROCOLLAGEN-PROLINE, 2-OXOGLUTARATE 4-DIOXYGENASE (PROLINE (NM_004199); a gene of HOMO SAPIENS ENDOTHELIAL DIFFERENTIATION, LYSOPHOSPHATIDIC ACID G-PROTE (NM_012152);or a gene of HOMO SAPIENS KIAA0127 GENE PRODUCT (KIAA0127), MRNA(NM_014755).

In the present description, known methods for detecting gene expression can be used to detect the expression of the mesenchymal stem cell marker gene of the present description. For example, Northern blotting can be used to detect the expression of the mesenchymal stem cell marker gene of the present description. In addition, it is possible to use a probe having a DNA sequence which hybridizes with a DNA sequence of the mesenchymal stem cell gene marker of the present description under a stringent condition in order to detect and distinguish mesenchymal stem cells by the gene marker of the present description. For the detection of mesenchymal stem cells by using the probe, known methods can be appropriately used. For instance, the detection of mesenchymal stem cells is conducted by the following process: constructing a DNA probe with appropriate length from a DNA sequence of a gene marker shown in the sequence listing; labeling the probe appropriately by fluorescent labeling, etc.; hybridizing the probe with a test substance. As the DNA probe, it is possible to use a probe for detecting mesenchymal stem cell marker genes comprising whole or part of an antisense strand of the base sequence of the gene marker of the present description shown in the sequence listing. The probe can be used also in a form of a microarray or a DNA chip for detecting marker genes, wherein at least one of the probes is immobilized.

In the construction of the DNA probe mentioned above, the condition for the base sequence of the present description "to hybridize with a DNA sequence of a marker gene for detecting mesenchymal stem cells under a stringent condition" is exemplified by hybridization at 42°C, and washing treatment at 42°C with buffer solution containing 1 × SSC (0.15 M NaCl, 0.015 M sodium citrate), and 0.1% SDS (sodium dodecyl sulfate), and more preferably exemplified by hybridization at 65°C, and washing treatment at 65°C with buffer solution containing 0.1 × SSC, and 0.1% SDS. There are various factors other than the temperature conditions mentioned above that affect the hybridization stringency and those skilled in the art can actualize the same stringency as that of the hybridization referred to in the above by appropriately combining various factors.

Further, when detecting the polypeptide marker for detecting mesenchymal stem cells of the present description, an antibody induced by the polypeptide of the protein and specifically binds to the polypeptide can be used in the present description. Examples of the antibody include monoclonal and polyclonal antibodies. The antibody can be constructed by ordinary methods with the polypeptide marker of the present description as an antigen. In order to detect the expression of the marker polypeptide for detecting mesenchymal stem cells of the present description in a test cell by using the antigen of the present description, immunoassay methods using known antibodies can be used. As for such immunoassay methods, known immunoassay methods such as RIA, ELISA, and fluorescent antibody method are exemplified.

In the present invention, it is possible to detect and distinguish the expression of a marker gene for detecting mesenchymal stem cells and/or a marker polypeptide for detecting mesenchymal stem cells in a test cell by using the probe for detecting mesenchymal stem cells of the present description and/or the antibody for detecting mesenchymal stem cells of the present description, and to separate and obtain mesenchymal stem cells. When detecting a marker gene for detecting mesenchymal stem cells in a test cell, quantitative or semiquantitative PCR can be used to amplify the gene of the test cell. RT-PCR (reverse transcription PCR) can be used as the PCR. When conducting the PCR, primers comprising a sense primer and an antisense primer for amplifying a marker gene for detecting mesenchymal stem cells of the present description are used.

Examples of the primers for amplifying a marker gene for detecting mesenchymal stem cells of the present description include: a sense primer having a base sequence shown in SEQ ID NO: 20 in the sequence listing, and an antisense primer having a base sequence shown in SEQ ID NO: 21 in the sequence listing; a sense primer having a base sequence shown in SEQ ID NO: 22 in the sequence listing, and an antisense primer having a base sequence shown in SEQ ID NO: 23 in the sequence listing; a sense primer having a base sequence shown in SEQ ID NO: 24 in the sequence listing, and an antisense primer having a base sequence shown in SEQ ID NO: 25 in the sequence listing; a sense primer having a base sequence shown in SEQ ID NO: 26 in the sequence listing, and an antisense primer having a base sequence shown in SEQ ID NO: 27 in the sequence listing; a sense primer having a base sequence shown in SEQ ID NO: 28 in the sequence listing, and an antisense primer having a base sequence shown in SEQ ID NO: 29 in the sequence listing; a sense primer having a base sequence shown in SEQ ID NO: 30 in the sequence listing, and an antisense primer having a base sequence shown in SEQ ID NO: 31 in the sequence listing; a sense primer having a base sequence shown in SEQ ID NO: 32 in the sequence listing, and an antisense primer having a base sequence shown in SEQ ID NO: 33 in the sequence listing; a sense primer having a base sequence shown in SEQ ID NO: 34 in the sequence listing, and an antisense primer having a base sequence shown in SEQ ID NO: 35 in the sequence listing; a sense primer having a base sequence shown in SEQ ID NO: 36 in the sequence listing, and an antisense primer having a base sequence shown in SEQ ID NO: 37 in the sequence listing; a sense primer having a base sequence shown in SEQ ID NO: 38 in the sequence listing, and an antisense primer having a base sequence shown in SEQ ID NO: 39 in the sequence listing; a sense primer having a base sequence shown in SEQ ID NO: 40 in the sequence listing, and an antisense primer having a base sequence shown in SEQ ID NO: 41 in the sequence listing; a sense primer having a base sequence shown in SEQ ID NO: 42 in the sequence listing, and an antisense primer having a base sequence shown in SEQ ID NO: 43 in the sequence listing; or a sense primer having a base sequence shown in SEQ ID NO: 44 in the sequence listing, and an antisense primer having a base sequence shown in SEQ ID NO: 45 in the sequence listing.

Examples of the primers for amplifying a marker gene for detecting mesenchymal stem cells of the present description further include: a real time PCR primer for amplifying a mesenchymal stem cell marker gene which comprises a sense primer having a base sequence shown in SEQ ID NO: 47 in the sequence listing, and an antisense primer having a base sequence shown in SEQ ID NO: 48 in the sequence listing; a sense primer having a base sequence shown in SEQ ID NO: 49 in the sequence listing, and an antisense primer having a base sequence shown in SEQ ID NO: 50 in the sequence listing; a sense primer having a base sequence shown in SEQ ID NO: 51 in the sequence listing, and an antisense primer having a base sequence shown in SEQ ID NO: 52 in the sequence listing; a sense primer having a base sequence shown in SEQ ID NO: 53 in the sequence listing, and an antisense primer having a base sequence shown in SEQ ID NO: 54 in the sequence listing; a sense primer having a base sequence shown in SEQ ID NO: 55 in the sequence listing, and an antisense primer having a base sequence shown in SEQ ID NO: 56 in the sequence listing; a sense primer having a base sequence shown in SEQ ID NO: 57 in the sequence listing, and an antisense primer having a base sequence shown in SEQ ID NO: 58 in the sequence listing; a sense primer having a base sequence shown in SEQ ID NO: 59 in the sequence listing, and an antisense primer having a base sequence shown in SEQ ID NO: 60 in the sequence listing; a sense primer having a base sequence shown in SEQ ID NO: 61 in the sequence listing, and an antisense primer having a base sequence shown in SEQ ID NO: 62 in the sequence listing; a sense primer having a base sequence shown in SEQ ID NO: 63 in the sequence listing, and an antisense primer having a base sequence shown in SEQ ID NO: 64 in the sequence listing; a sense primer having a base sequence shown in SEQ ID NO: 65 in the sequence listing, and an antisense primer having a base sequence shown in SEQ ID NO: 66 in the sequence listing; a sense primer having a base sequence shown in SEQ ID NO: 67 in the sequence listing, and an antisense primer having a base sequence shown in SEQ ID NO: 68 in the sequence listing; or a sense primer having a base sequence shown in SEQ ID NO: 69 in the sequence listing, and an antisense primer having a base sequence shown in SEQ ID NO: 70 in the sequence listing.

Examples of the primers for amplifying a marker gene for detecting mesenchymal, stem cells of the present description further include: an RT-PCR primer for amplifying a mesenchymal stem cell marker gene which comprises a sense primer having a base sequence shown in SEQ ID NO: 71 in the sequence listing, and an antisense primer having a base sequence shown in SEQ ID NO: 72 in the sequence listing; a sense primer having a base sequence shown in SEQ ID NO: 73 in the sequence listing, and an antisense primer having a base sequence shown in SEQ ID NO: 74 in the sequence listing; a sense primer having a base sequence shown in SEQ ID NO: 75 in the sequence listing, and an antisense primer having a base sequence shown in SEQ ID NO: 76 in the sequence listing; a sense primer having a base sequence shown in SEQ ID NO: 77 in the sequence listing, and an antisense primer having a base sequence shown in SEQ ID NO: 78 in the sequence listing; a sense primer having a base sequence shown in SEQ ID NO: 79 in the sequence listing, and an antisense primer having a base sequence shown in SEQ ID NO: 80 in the sequence listing; a sense primer having a base sequence shown in SEQ ID NO: 81 in the sequence listing, and an antisense primer having a base sequence shown in SEQ ID NO: 82 in the sequence listing; a sense primer having a base sequence shown in SEQ ID NO: 83 in the sequence listing, and an antisense primer having a base sequence shown in SEQ ID NO: 84 in the sequence listing; a sense primer having a base sequence shown in SEQ ID NO: 85 in the sequence listing, and an antisense primer having a base sequence shown in SEQ ID NO: 86 in the sequence listing; a sense primer having a base sequence shown in SEQ ID NO: 87 in the sequence listing, and an antisense primer having a base sequence shown in SEQ ID NO: 88 in the sequence listing; a sense primer having a base sequence shown in SEQ ID NO: 89 in the sequence listing, and an antisense primer having a base sequence shown in SEQ ID NO: 90 in the sequence listing; a sense primer having a base sequence shown in SEQ ID NO: 91 in the sequence listing, and an antisense primer having a base sequence shown in SEQ ID NO: 92 in the sequence listing; a sense primer having a base sequence shown in SEQ ID NO: 93 in the sequence listing, and an antisense primer having a base sequence shown in SEQ ID NO: 94 in the sequence listing; a sense primer having a base sequence shown in SEQ ID NO: 95 in the sequence listing, and an antisense primer having a base sequence shown in SEQ ID NO: 96 in the sequence listing; a sense primer having a base sequence shown in SEQ ID NO: 97 in the sequence listing, and an antisense primer having a base sequence shown in SEQ ID NO: 98 in the sequence listing; a sense primer having a base sequence shown in SEQ ID NO: 99 in the sequence listing, and an antisense primer having a base sequence shown in SEQ ID NO: 100 in the sequence listing; a sense primer having a base sequence shown in SEQ ID NO: 101 in the sequence listing, and an antisense primer having a base sequence shown in SEQ ID NO: 102 in the sequence listing; a sense primer having a base sequence shown in SEQ ID NO: 103 in the sequence listing, and an antisense primer having a base sequence shown in SEQ ID NO: 104 in the sequence listing; a sense primer having a base sequence shown in SEQ ID NO: 105 in the sequence listing, and an antisense primer having a base sequence shown in SEQ ID NO: 106 in the sequence listing; a sense primer having a base sequence shown in SEQ ID NO: 107 in the sequence listing, and an antisense primer having a base sequence shown in SEQ ID NO: 108 in the sequence listing; a sense primer having a base sequence shown in SEQ ID NO: 109 in the sequence listing, and an antisense primer having a base sequence shown in SEQ ID NO: 110 in the sequence listing; a sense primer having a base sequence shown in SEQ ID NO: 111 in the sequence listing, and an antisense primer having a base sequence shown in SEQ ID NO: 112 in the sequence listing; a sense primer having a base sequence shown in SEQ ID NO: 113 in the sequence listing, and an antisense primer having a base sequence shown in SEQ ID NO: 114 in the sequence listing; or a sense primer having a base sequence shown in SEQ ID NO: 115 in the sequence listing, and an antisense primer having a base sequence shown in SEQ ID NO: 116 in the sequence listing.

In order to distinguish mesenchymal stem cells in the present description, a gene in a test cell is amplified by using at least one pair of primers comprising the sense primer and the antisense primer mentioned above, and the amplified gene is detected by using the probe for detecting mesenchymal stem cell marker genes of the present description.

With regard to the marker genes for detecting mesenchymal stem cells of the present description, some genes exhibit specifically high expression and some genes exhibit specifically low expression in mesenchymal stem cells. By detecting the expressions of these genes in combination, accuracy in detecting mesenchymal stem cells can be improved. Examples of genes exhibiting specifically high expression in mesenchymal stem cells include a mesenchymal stem cell marker gene comprising SEQ ID NOs: 1, 11 and 19 in the sequence listing, and those of genes exhibiting specifically low expression in mesenchymal stem cells include a mesenchymal stem cell marker gene comprising SEQ ID NOs: 2, 4, 6, 7, 9, 10, 12, 14, 15, 17 and 46 in the sequence listing. The mesenchymal stem cell marker gene confirmed in the present description is also exemplified. Therefore, by combining the expression of one or more genes from each gene group, mesenchymal stem cells can be distinguished with more improved detection accuracy.

In order to detect the expression of a mesenchymal stem cell marker polypeptide in a test cell by a fluorescent antibody method using the antibody of the present description, and to distinguish and separate mesenchymal stem cells, mesenchymal stem cells can be labeled and separated by known methods. As for the labeling method, for instance, a fluorescent antibody method is exemplified. The labeling of undifferentiated hemopoietic cells by the fluorescent antibody method can be conducted by: fluorescently labeling an antibody that specifically binds to the polypeptide marker for detecting mesenchymal stem cells of the present description, and labeling mesenchymal stem cells by binding the antibody to mesenchymal stem cells expressing an antigen (direct fluorescent antibody method); or binding the unlabeled specific antibody of the present description to a mesenchymal stem cell expressing an antigen, and then, by binding a labeled secondary antibody (anti-immunoglobulin antibody), labeling mesenchymal stem cells (indirect fluorescent antibody method). Subsequently, the labeled mesenchymal stem cells are separated and collected.

The probe for detecting mesenchymal stem cell marker genes used for detecting and distinguishing mesenchymal stem cells of the present description, the microarray or the DNA chip for detecting mesenchymal stem cell marker genes in which the probe is immobilized, and the antibody for detecting mesenchymal stem cells of the present description can be commercialized as a kit for distinguishing mesenchymal stem cells in which they are included.

The present invention is described below more specifically with reference to Examples, however, the technical scope of the present invention is not limited to these exemplification.

### Example 1 [Materials and methods]

### (Cells)

Human mesenchymal stem cells and human fibroblasts were cultured at 37°C in a Dalbecco's modified Eagle's medium (low glucose) (Sigma) supplemented with 10% fetal calf serum and 1 ng/ml bFGF.

### (DNA microarray)

Total RNA was extracted from each cell in one 10 cm-dish by using TRIZOL reagent (Invitrogen), and then mRNA was purified by using micro poly (A) purist (Ambion) to conduct DNA microarray (Incyte Genomics: Kurabo Life Array analysis service; Lot. No. KL01081). The DNA microarray analysis was commissioned to Kurabo.

### (Semiquantitative RT-PCR)

Total RNA was extracted from each cell by using TRIZOL reagent (Invitrogen), and then cDNA was synthesized by conducting a reverse transcription reaction at 42°C for 50 minutes with SuperScript first-strand synthesis system for RT-PCR (Invitrogen) using 1 mg each of total RNA as a template. Each gene was amplified with Advantage 2 PCR enzyme system (Clontech) using the amplified cDNA as a template. The reaction was conducted in 30 cycles, and each cycle included denaturation at 94°C for 30 seconds and annealing at 68°C for 1 minute. The primer sequences of each gene used for amplification are tabulated in Table 1. The amplified genes were separated by electrophoresis using 1% agarose gel, and detected by ethidium bromide staining.

**(Table 1)**

| Gene name | Primers (5' to 3') for RT-PCR | Primers (5' to 3') for real time PCR | Probes for real time PCR |
|---|---|---|---|
| Adrenomedullin | | | ACCTGGGTTCGCTCGCCTTCCTAG |
| Matrix metaroproteinase 1 | | | TCATGCTTTTCAACCAGGCCCAGGTATT |
| Tissue factor pathway inhibitor 2 | | | CTGGGAGGCTTGCGACGATGC |
| Serine (or cysteine) proteinase inhibitor | | | CTGGTGAAAGATTTGGTATCCCCAAGGG |
| Protein tyrosine kinase 7 | | | TCTCTTGGCCCACTGGTCCCACTTG |
| Collagen, type XV, alpha 1 | | | TGCCTCCACCAAACCCTATTTCAAGTGC |
| Sam 68-like phosphotyrosine protein | | | CTCTGTAGACGCCCTTTGCTGTCCTCG |
| Apolipoprotein D | | | ATGGACGCTGCATCCAGGCCAACTA |
| Major histocompatibitity complex DR beta | | | CCTGAAGTAGATGAACAGCCCGGCC |
| Major histocompatibility complex DR alpha | | | TCCGCAAGTTCCACTATCTCCCCTTCCT |
| C-type lectin | | | CTGTTGCTGCACCATCATCGCTGAG |
| CUG triplet repeat RNA-binding protein 2 | | | CTCAGCCACCAGCACCAATGCAAAC |

### Example 2 [Detection results]

### (DNA microarray)

As a result of DNA microarray, it was revealed that 63 out of 9400 genes exhibited significantly higher expression, and 141 out of 9400 genes exhibited significantly lower expression in mesenchymal stem cells than in fibroblasts. Significant differences were not observed in other genes. Among 87 genes which exhibited.a difference of three-fold or more, 29 genes exhibited higher expression and 58 genes exhibited lower expression in mesenchymal stem cells.

### (Semiquantitative RT-PCR)

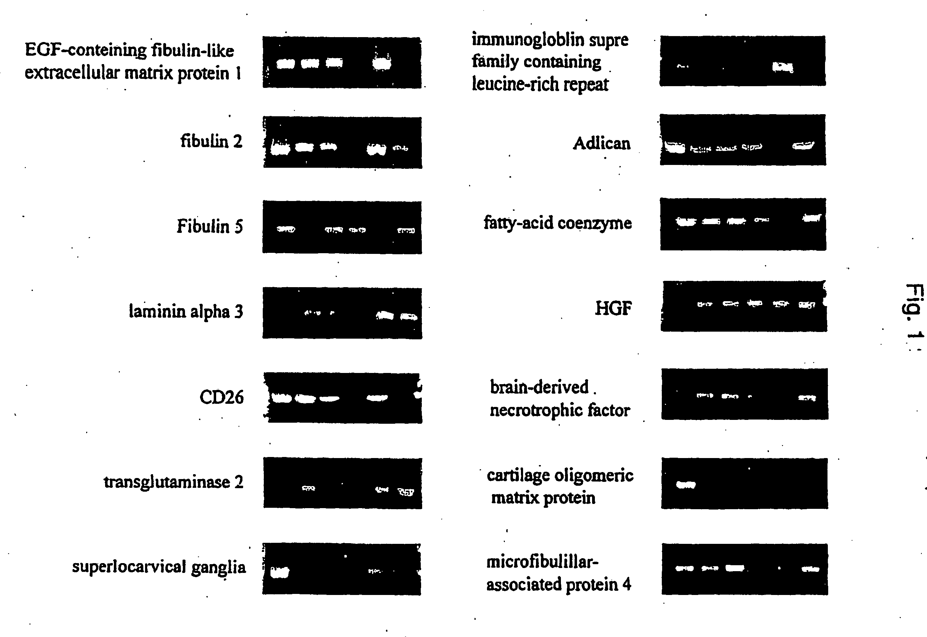

Total RNAs were extracted from mesenchymal stem cells derived from 3 individuals and fibroblasts derived from 3 individuals, and by using them as a template, the expression levels of the above-mentioned 87 genes were examined by Semiquantitative RT-PCR. As a result, most genes exhibited no difference in the expression levels. In addition, there were many cases wherein differences in the expression were considered to be caused by individual differences, because differences in the expression levels were observed between mesenchymal stem cells, or fibroblasts, from different individuals (Fig. 1).

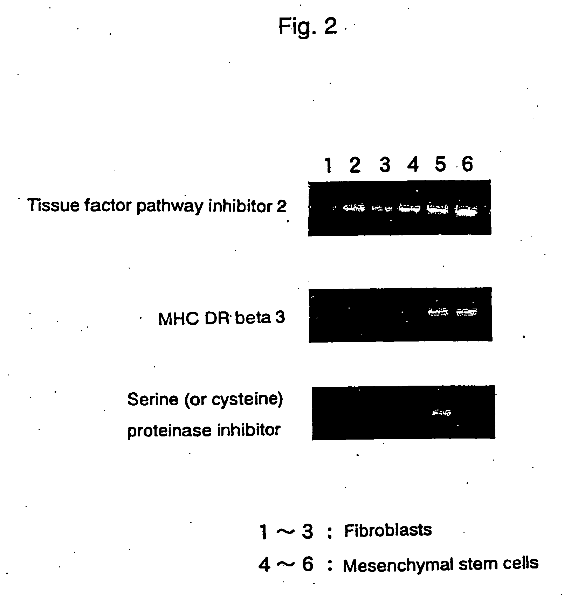

The following 3 genes were confirmed to exhibit high expression in mesenchymal stem cells by RT-PCR: serine (or cysteine) proteinase inhibitor (SEQ ID NO: 1 in the sequence listing), major histocompatibility complex class2 DR beta 3 (SEQ ID NO: 11), and tissue factor pathway inhibitor 2 (SEQ ID NO: 19) (Fig. 2).

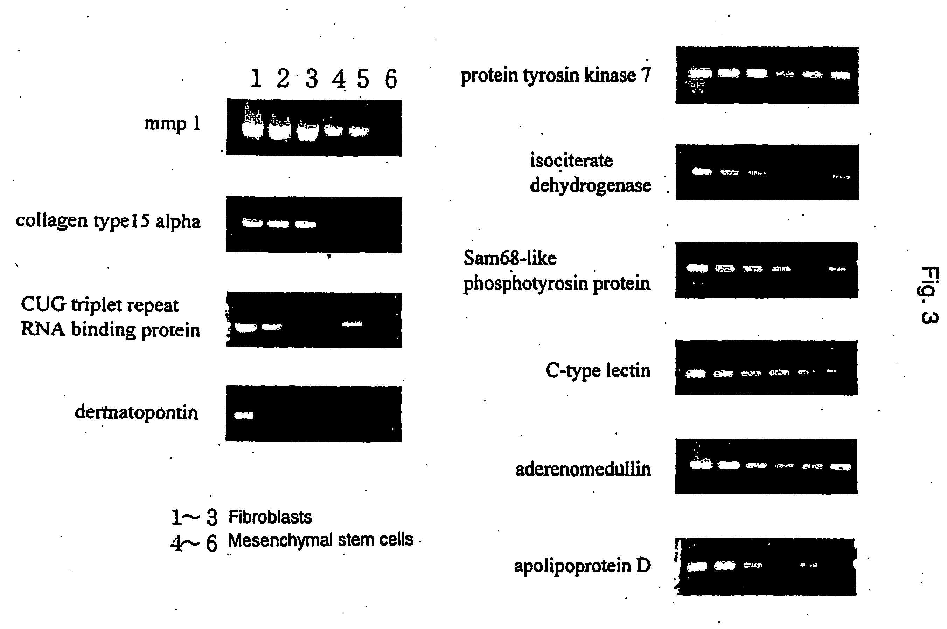

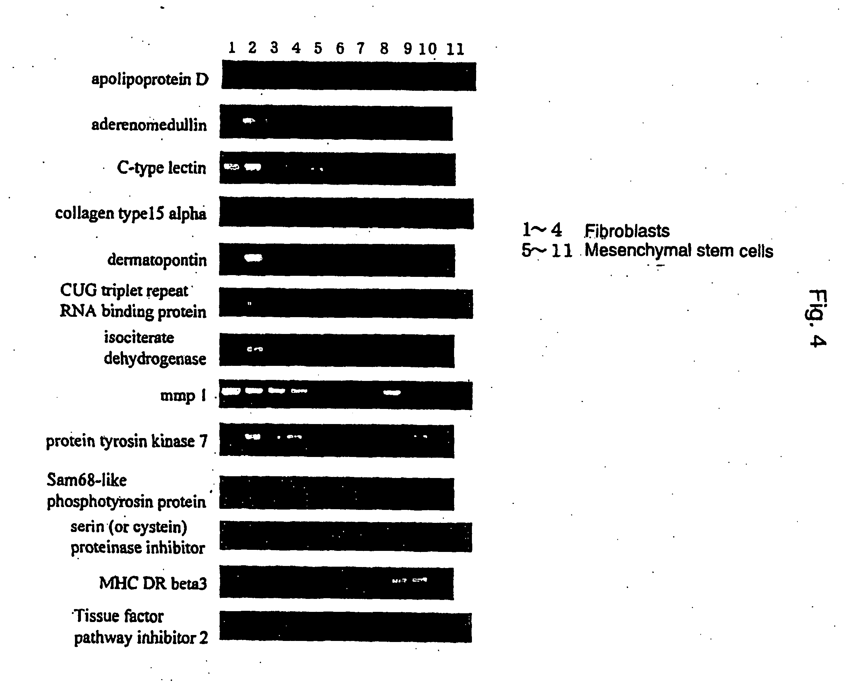

In addition, the following 10 genes were confirmed to exhibit low expression in mesenchymal stem cells by RT-PCR: adrenomedullin (SEQ ID NO: 2), apolipoprotein D (SEQ ID NO: 4), collagen type XV alpha 1 (SEQ ID NO: 6), CUG triplet repeat RNA binding protein (SEQ ID NO: 7), dermatopontin protein (SEQ ID NO: 9), isocitrate dehydrogenase 2 (SEQ ID NO: 10), tyrosine kinase 7 (SEQ ID NO: 12), Sam68-like phosphotyrosine protein (SEQ ID NO: 14), C-type lectin superfamily member 2 (SEQ ID NO: 15), and matrix metalloprotease 1 (SEQ ID NO: 17) (Fig. 3). Further, with regard to the 13 genes, the expression levels were confirmed by semiquantitative RT-PCR using total RNA extracted from mesenchymal stem cells derived from 7 individuals and fibroblasts derived from 4 individuals as a template (Fig. 4).

### Example 3 [Measurement of the expression of each gene in mesenchymal stem cells I]

The expressions of the gene marker of the present description inhumanmesenchymal stem cells and human fibroblasts were measured in the same manner as in Example 1. The primers for RT-PCR and for real time PCR, and probes for real time PCR used are shown in Table 2.

**(Table 2)**

| Gene name | Primers (5' to 3') for RT-PCR | Primers (5' to 3') for real time PCR | Probes for real time PCR |
|---|---|---|---|
| Adrenomedullin | | | ACCTGGGTTCGCTCGCCTTCCTAG |
| Matrix metaroproteinase 1 | | | TCATGCTTTTCAACCAGGCCCAGGTATT |
| Tissue factor pathway inhibitor 2 | | | CTGGGAGGCTTGCGACGATGC |
| Serine (or cysteine) proteinase inhibitor | | | CTGGTGAAAGATTTGGTATCCCCAAGGG |
| Protein tyrosine kinase 7 | | | TCTCTTGGCCCACTGGTCCCACTTG |
| Collagen, type XV, alpha 1 | | | TGCCTCCACCMACCCTATTTCAAGTGC |
| Sam 68-like phosphotyrosine protein, | | | CTCTGTAGACGCCCTTTGCTGTCCTCG |
| Apolipoprotein D | | | ATGGACGCTGCATCCAGGCCAACTA |
| Major histocompatibility complex DR beta | | | CCTGAAGTAGATGAACAGCCCGGCC |
| Major histocompatibility complex DR alpha | | | TCCGCAAGTTCCACTATCTCCCCTTCCT |
| C-type lectin | | | CTGTTGCTGCACCATCATCGCTGAG |
| CHUG triplet repeat RNA-binding protein 2 | | | CTCAGCCACCAGCACCAATGCAAAC |

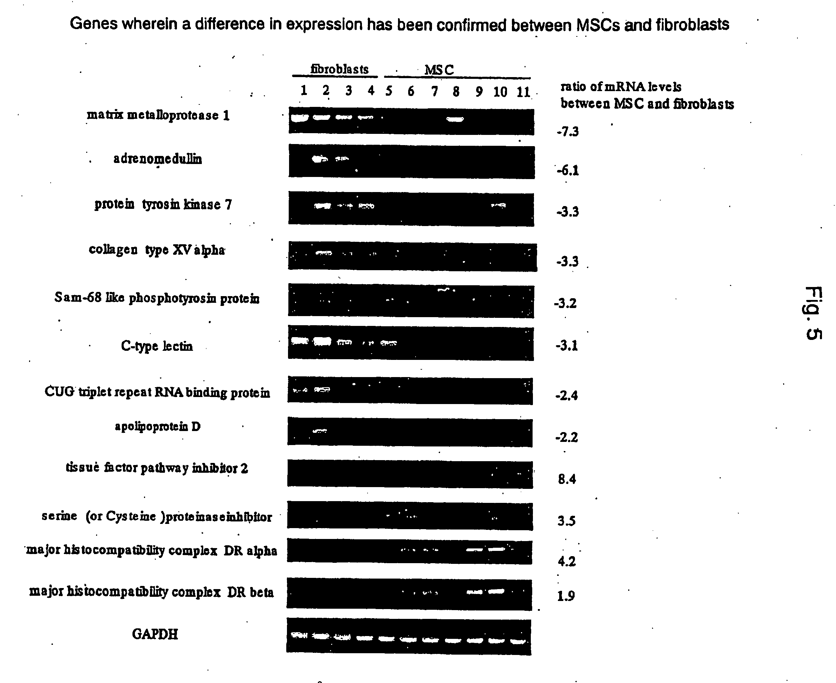

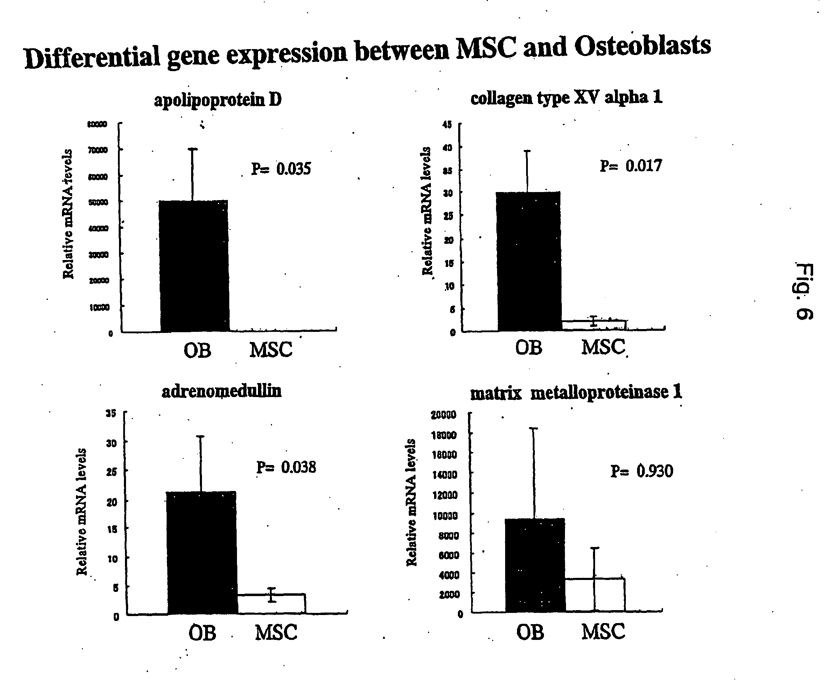

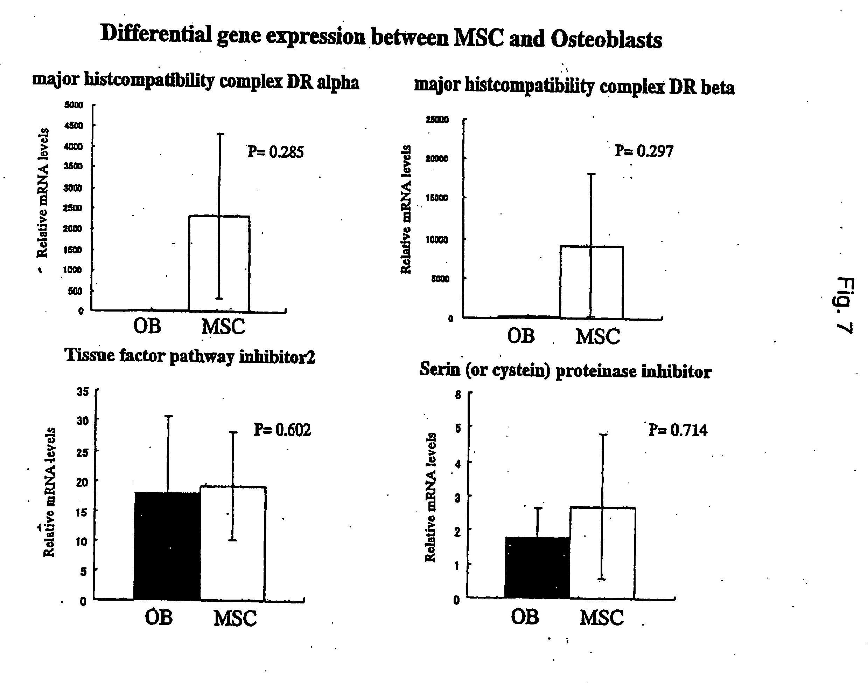

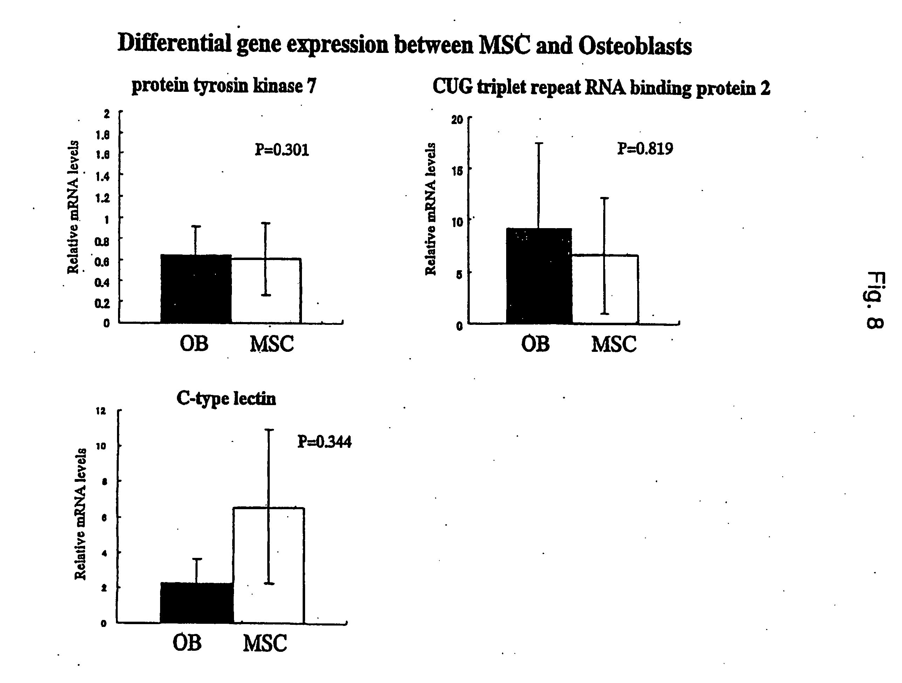

The expression state of mRNA level in mesenchymal stem cells and human fibroblasts are shown in Fig. 5. The relative expression state of mRNA level in mesenchymal stem cells and human fibroblasts are shown in Figs. 6, 7, and 8.

### Example 4 [Measurement of the expression of each gene in mesenchymal stem cells II]

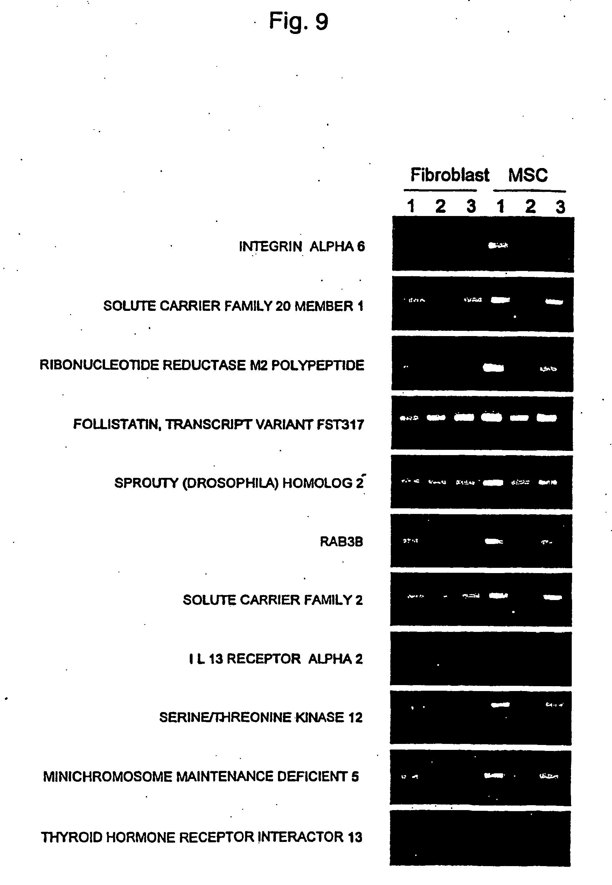

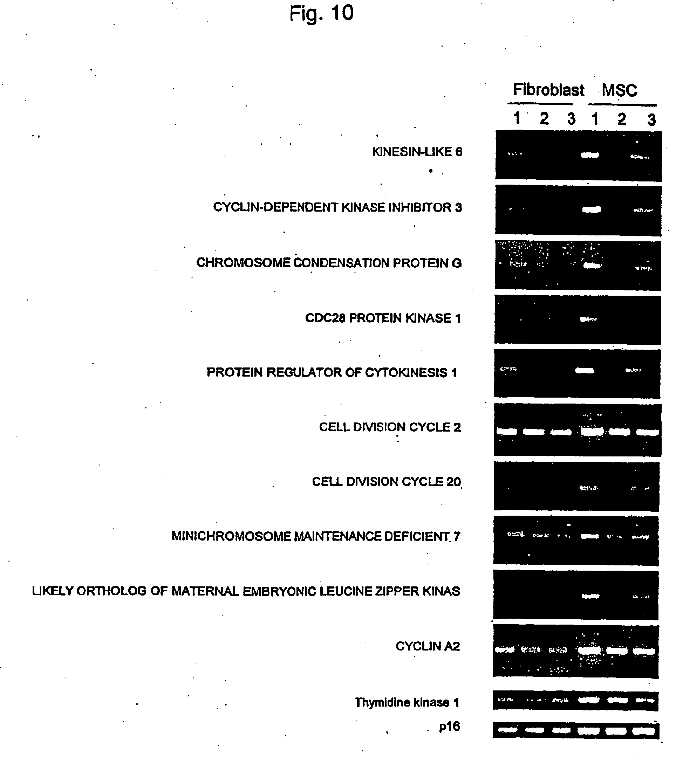

The expressions of each gene marker of the present description in human mesenchymal stem cells and human fibroblasts were measured in the same manner as in Example 1. The sense and antisense primers for RT-PCR used are shown in SEQ ID NOs: 71 to 116 in the sequence listing. The expression state of genes in mesenchymal stem cells and human fibroblasts are shown in Figs. 9 and 10. The expression ratio, mesenchymal stem cell/fibroblast, is shown in Table 3.

**(Table 3)**

| MSC/fibroblast | Gene name |
|---|---|
| 11.6 | NTEGRIN, ALPHA 6 (ITGA6), MRNA |
| 8.7 | SOLUTE CARRIER FAMILY 20 (PHOSPHATE TRANSPORTER), MEMBER 1 |
| 7.8 | RIBONUCLEOTIDE REDUCTASE M2 POLYPEPTIDE (RRM2), MRNA |
| 7.6 | FOLLISTATIN (FST), TRANSCRIPT VARIANT FST317, MRNA |
| 7.3 | SPROUTY (DROSOPHILA) HOMOLOG 2 (SPRY2), MRNA |
| 6.9 | RAB3B, MEMBER RAS ONCOGENE FAMILY (RAB3B), MRNA |
| 6.5 | SOLUTE CARRIER FAMILY 2 (FACILITATED GLUCOSE TRANSPORTER) |
| 6.1 | INTERLEUKIN 13 RECEPTOR, ALPHA 2 (IL13RA2), MRNA |
| 5.9 | SERINE/THREONINE KINASE 12 (STK12), MRNA |
| 5.7 | MINICHROMOSOME MAINTENANCE DEFICIENT (S. CEREVISIAE) |
| 5.6 | THYROID HORMONE RECEPTOR INTERACTOR 13 (TRIP13), MRNA |
| 5.5 | KINESIN-LIKE 6 (MITOTIC CENTROMERE-ASSOCIATED KINESIN) |
| 5.5 | CYCLIN-DEPENDENT KINASE INHIBITOR 3 |
| 5.4 | CHROMOSOME CONDENSATION PROTEIN G (HCAP-G), MRNA |
| 5.2 | CDC28 PROTEIN KINASE 1 (CKS1), MRNA |
| 5.2 | PROTEIN REGULATOR OF CYTOKINESIS 1 (PRC1), MRNA |
| 5.2 | CELL DIVISION CYCLE 2, G1 TO S AND G2 TO M (CDC2), MRNA |
| 5.2 | CDC20 (CELL DIVISION CYCLE 20, S. CEREVISIAE, HOMOLOG) (CD |
| 5.1 | LIKELY ORTHOLOG OF MATERNAL EMBRYONIC LEUCINE ZIPPER KINAS |
| 5.1 | MINICHROMOSOME MAINTENANCE DEFICIENT (S. CEREVISIAE) 7 |
| 4.9 | YCLIN A2 (CCNA2), MRNA |
| 3.6 | THYMIDINE KINASE 1, SOLUBLE (TK1), MRNA |
| 0.6 | cyclin-dependent kinase inhibitor 2A (melanoma, p16, inhibits CDK4) (CDKN2A), mRNA |

### Example 5 [Measurement of the expression of each gene in mesenchymal stem cells III]

The expressions of the gene marker of the present description inhuman mesenchymal stem cells and human fibroblasts were measured in the same manner as in Example 1. The expression ratio, mesenchymal stem cell/fibroblast, is shown in Table 4.

**(Table 4)**

| (Mesenchymal stem cells/fibroblasts) (Gene name) | |
|---|---|
| 8.9 | EGF-containing fibulin-like extracellular matrix protein 1 |
| 8.4 | Human insulin-like growth factor binding protein 5 (IGFBP5) mRNA |
| 7.7 | Homo sapiens clone 24775 mRNA sequence |
| 7 | "proteoglycan 1, secretory granule" |
| 6.8 | insulin-like growth factor binding protein 5 |
| 6.7 | "solute carrier family 21 (organic anion transporter), member 3" |
| 6.5 | "transglutaminase 2 (C polypeptide, proteinglutamine-gamma-glutamyl transferase)" |
| 5.8 | coagulation factor II (thrombin) receptor |
| 5.6. | "plasminogen activator, urokinase" |
| 5.5 | "tissue inhibitor of metalloproteinase 3 (Sorsby fundus dystrophy, pseudoinflammatory)" |
| 5.1 | matrix Gla protein |
| 4.6 | Bell CLL/lymphoma 1 |
| 4.6. | "CD74 antigen (invariant polypeptide of major histocompatibility complex, class II antigen-associated)" |
| 4.2 | adducin 3 (gamma) |
| 4.2 | "solute carrier family 16 (monocarboxylic acid transporters), member 4" |
| 3.4 | protein S (alpha) |
| 3.3 | "phosphatidylinositol (4,5) bisphosphate 5-phosphatase homolog; phosphatidylinositol polyphosphate 5-phosphatase type IV" |
| 3.2 | progesterone membrane binding protein |
| 3.2 | brain-derived neurotrophic factor |
| 3 | apolipoprotein E |
| 38.5 | "GI\|6005949\|REF\|NM_007191.1\| HOMO SAPIENS WNT INHIBITORY FACTOR-1 (WIF-1), MRNA" |
| 12.7 | "GI\|8922916\|REF\|NM_018349.1\| HOMO SAPIENS HYPOTHETICAL PROTEIN FLJ11175 (FLJ11175), MRNA" |
| 11.2 | "GI\|10835001\|REF\|NM_001175.1\| HOMO SAPIENS RHO GDP DISSOCIATION INHIBITOR (GDI) BETA (ARHGDIB), MRNA" |
| 10.3 | "GI\|11055973\|REF\|NM_021614.1\| HOMO SAPIENS POTASSIUM INTERMEDIATE/SMALL CONDUCTANCE CALCIUM.ACTIVATE" |
| 8.7 | "GI\|5731346\|REF\|NM_004289.3\| HOMO SAPIENS NUCLEAR FACTOR (ERYTHROID-DERIVED 2)-LIKE 3 (NFE2L3), MRNA" |
| 7.6 | "GI\|11056053\|REF\|NM_021643.1\| HOMO SAPIENS GS3955 PROTEIN (GS3955), MRNA" |
| 7.5 | "EST10324 ADIPOSE TISSUE, WHITE I HOMO SAPIENS CDNA 3' FND SIMILAR TO SIMILAR TO GLYCOPROTEIN MUC18," |
| 6.6 | "GI\|13375818\|REF\|NM_024609.1\| HOMO SAPIENS HYPOTHETICAL PROTEIN FLJ21841 (FLJ21841), MRNA" |
| 6.5 | "GI\|6005971\|REF\|NM_007150.1\| HOMO SAPIENS ZINC FINGER PROTEIN 185 (LIM DOMAIN) (ZNF185), MRNA" |
| 6.5 | "GNL\|UG\|HS#S2334464 SC1=PUTATIVE TRANS-ACTING FACTOR INVOLVED IN CELL CYCLE CONTROL [HUMAN, MRNA, 24" |
| 6.4 | "GI\|7657368\|REF\|NM_014222.1\| HOMO SAPIENS NADH DEHYDROGENASE (UBIQUINONE) 1 ALPHA SUBCOMPLEX, 8 (19K" |
| 6.3 | "GI\|8924142\|REF\|NM_018518.1\| HOMO SAPIENS HOMOLOG OF YEAST MCM10; HYPOTHETICAL PROTEIN PRO2249 (PRO2" |
| 6.2 | "GI\|4502960\|REF\|NM_000094.1\|HOMO SAPIENS COLLAGEN, TYPE VII, ALPHA 1 (EPIDERMOLYSIS BULLOSA, DYSTRO" |
| 6.2 | "GI\|4502074\|REF\|NM_001144.1\| HOMO SAPIENS AUTOCRINE MOBILITY FACTOR RECEPTOR (AMFR), MRNA" |
| 6.2 | GNL\|UG\|HS#S1090551 HOMO SAPIENS CLONE 24421 MRNA SEQUENCE /ICDS=UNKNOWN /GB=AF070641 /GI=3283914 /UG= |
| 6.2 | GNL\|UG\|HS#S1570341 HOMO SAPIENS MRNA; CDNA DKFZP586E1621 (FROM CLONE DKFZP586E1621) ICDS=UNKNOWN /GB |
| 6.1 | "GI\|11545760\|REF\|NM_022055.1\| HOMO SAPIENS RESERVED (KCNK12), MRNA" |
| 6.0 . | "GI\|5902145\|REF\|NM_007019.1\| HOMO SAPIENS UBIQUITIN-CONJUGATING ENZYME E2C (UBE2C), MRNA" |
| 5.9 | "GNL\|UG\|HS#S1970614 HOMO SAPIENS CDNA FLJ10517 FIS, CLONE |
| | NT2RP20008121/CDS=(61,918) /GB=AK001379 /G" |
| 5.8 | "GI\|4503052\|REF\|NM_001884.1\| HOMO SAPIENS CARTILAGE LINKING PROTEIN 1 (CRTL1), MRNA" |
| 5.8 | GNL\|UG\|HS#S1972520 HOMO SAPIENS MRNA; CDNA DKFZP434F2322 (FROM CLONE DKFZP434F2322) /CDS=UNKNOWN /GB |
| 5.7 | "GNL\|UG\|HS#S3837475 HOMO SAPIENS, CLONE MGC:9549 IMAGE:3857382, MRNA, COMPLETE CDS /CDS=(92,1285) /G |
| 5.4 | "GI\|4150008\|REF\|NM_032270.1\| HOMO SAPIENS HYPOTHETICAL PROTEIN DKFZP586J1119 (DKFZP586J1119), MRNA" |
| 5.4 | "GI\|4758439\|REF\|NM_004877.1\| HOMO SAPIENS GLIA MATURATION FACTOR, GAMMA (GMFG), MRNA" |
| 5.4 | "GNL\|UG\|HS#S2654530 HOMO SAPIENS CDNA: FLJ23095 FIS, CLONE LNG07413 /CDS=UNKNOWN /GB=AK026748 /GI=10" |
| 5.3 | "GI\|8922180\|REF\|NM_018410.1\| HOMO SAPIENS HYPOTHETICAL PROTEIN DKFZP762E1312 (DKFZP762E1312), MRNA" |
| 5.3 | "GI\|5031806\|REF\|NM_005853.1\| HOMO SAPIENS IROQUOIS-CLASS HOMEODOMAIN PROTEIN (IRX-2A), MRNA" |
| 5.2 | "GI\|8922501\|REF\|NM_018131.1\| HOMO SAPIENS HYPOTHETICAL PROTEIN FLJ10540 (FLJ10540), MRNA" |
| 5.1 | "GNL\|UG\|HS#S1972896 HOMO SAPIENS MRNA; CDNA DKFZP434C1915 (FROM CLONE DKFZP434C1915); PARTIAL CDS /C" |
| 5.1 | "GI\|14149622\|REF\|NM_005916.11 \|HOMO SAPIENS MINICHROMOSOME MAINTENANCE DEFICIENT (S. CEREVISIAE) 7 (M" |
| 5.0 | "GI\|4758177\|REF\|NM_004411.1\| HOMO SAPIENS DYNEIN, CYTOPLASMIC, INTERMEDIATE POLYPEPTIDE 1 (DNCI1), M" |
| 5.0 | HUMAN MRNA FOR PROTEIN GENE PRODUCT (PGP) 9.5. |
| 5.0 | "GI\|5453903\|REF\|NM_006479.1\| HOMO SAPIENS RAD51-INTERACTING PROTEIN (PIR51), MRNA" |
| 4.9 | "GI\|4502144\|REF\|NM_001168.1\| HOMO SAPIENS BACULOVIRAL IAP REPEAT-CONTAINING 5 (SURVIVIN) (BIRC5), MR" |
| 4.9 | "GI\|9945386\|REF\|NM_004482.2\| HOMO SAPIENS UDP-N-ACETYL-ALPHA -D-GALACTOSAMINE:POLYPEPTIDE N-ACETYLGAL" |
| 4.9 | "GI\|14327895\|REF\|NM_031966.1\| HOMO SAPIENS CYCLIN B1 (CCNB1), MRNA" |
| 4.8 | "GI\|6325465\|REF\|NM_004111.3\| HOMO SAPIENS FLAP STRUCTURE-SPECIFIC ENDONUCLEASE 1 (FEN1), MRNA" |
| 4.8 | "GI\|4507274\|REF\|NM_003600.1\| HOMO SAPIENS SERINE/THREONINE KINASE 15 (STK15), MRNA" |
| 4.8 | "GI\|6466452\|REF\|NM_02358.2\| HOMO SAPIENS MAD2 (MITOTIC ARREST DEFICIENT, YEAST, HOMOLOG)-LIKE 1 (MA" |
| 4.7 | "GI\|15421859\|REF\|NM_018454.2\| HOMO SAPIENS NUCLEOLAR PROTEIN ANKT (ANKT), MRNA" |
| 4.7 | "GI\|7661807\|REF\|NM_014176.1\| HOMO SAPIENS HSPC150 PROTEIN SIMILAR TO UBIQUITIN-CONJUGATING ENZYME (H" |
| 4.7 | "GI\|9845284\|REF\|NM_019885.1\| HOMO SAPIENS CYTOCHROME P450 RETINOID METABOLIZING PROTEIN (P450RAI-2)," |
| 4.7 | "GI\|4505372\|REF\|NM_002497.11 HOMO SAPIENS NIMA (NEVER IN MITOSIS GENE A)-RELATED KINASE 2 (NEK2), MR" |
| 4.7 | "GI\|4502422\|REF\|NM_001202.1\| HOMO SAPIENS BONE MORPHOGENETIC PROTEIN 4 (BMP4), MRNA". |
| 4.7 | "GNL\|UG\|HS#S1970775 HOMO SAPIENS CDNA FLJ10674 FIS, CLONE . NT2RP2006436 /CDS=UNKNOWN /GB=AK001536 /GI" |
| 4.7 | "GI\|4585861\|REF\|NM_00.1809.2\|. HOMO SAPIENS CENTROMERE PROTEIN A (17KD) (CENPA), MRNA" |
| 4.6 | GNL\|UG\|HS#S1368141 HOMO SAPIENS MRNA; CDNA DKFZP564P116 (FROM CLONE DKFZP564P116) /CDS=UNKNOWN /GB=A |
| 4.6 | "GI\|4507718\|REF\|NM_003318.1\| HOMO SAPIENS TTK PROTEIN KINASE (TTK), MRNA" |
| 4.6 | "GI\|4504896\|REFINM_002266.1\| HOMO SAPIENS KARYOPHERIN ALPHA 2 (RAG COHORT 1, IMPORTIN ALPHA 1) (KPNA" |
| 4.6 | "G1\|5729983\|REF\|NM_006596.1\|. HOMO SAPIENS POLYMERASE (DNA |
| | DIRECTED), THETA (POLQ), MRNA" |
| 4.5 | "GI\|4758315\|REF\|NM_004454.1\| HOMO SAPIENS ETS VARIANT GENE 5 (ETS-RELATED MOLECULE) (ETV5), MRNA" |
| 4.5 | "HOMO SAPIENS TRANSFORMING, ACIDIC COILED-COIL CONTAINING PROTEIN 3 (TAC" |
| 4.5 | "HOMO SAPIENS KINESIN-LIKE 1 (KNSL1), MRNA" |
| 4.5 | "HOMO SAPIENS CENTROMERE PROTEIN E (312KD) (CENPE), MRNA" |
| 4.5 | "HOMO SAPIENS CDNA, 3' END /CLONE=IMAGE:1651289 /CLONE_END=3' /GB=Al12" |
| 4.4 | "HOMO SAPIENS DISTAL-LESS HOMEO BOX 5 (DLX5), MRNA" |
| 4.4 | "HOMO SAPIENS V-MYB AVIAN MYELOBLASTOSIS VIRAL ONCOGENE HOMOLOG-LIKE 2 " |
| 4.4 | "HOMO SAPIENS SERUM DEPRIVATION RESPONSE (PHOSPHATIDYLSERINE -BINDING PRO" |
| 4.4 | "HOMO SAPIENS HYPOTHETICAL PROTEIN FLJ10604 (FLJ10604), MRNA" |
| 4.4 | "HOMO SAPIENS RIBONUCLEASE HI, LARGE SUBUNIT(RNASEHI), MRNA |
| 4.4 | "HOMO SAPIENS ANILLIN (DROSOPHILA SCRAPS HOMOLOG), ACTIN BINDING PROTEIN" |
| 4.4 | "HOMO SAPIENS H4 HISTONE FAMILY, MEMBER G (H4FG), MRNA" |
| 4.3 | "HOMO SAPIENS G PROTEIN-COUPLED RECEPTOR 37 (ENDOTHELIN RECEPTOR TYPE B-" |
| 4.3 | "HOMO SAPIENS UBIQUITIN CARRIER PROTEIN (E2-EPF), MRNA" |
| 4.3 | "HOMO SAPIENS, SIMILAR TO TUMOR DIFFERENTIALLY EXPRESSED 1, CLONE IMAGE:3639252," |
| 4.2 | "HOMO SAPIENS HYPOTHETICAL PROTEIN MGC2577 (MGC2577), MRNA" |
| 4.2 | "HOMO SAPIENS HYPOTHETICAL PROTEIN FLJ13912 (FLJ13912), MRNA" |
| 4.2 | "HOMO SAPIENS ANNEXIN A3 (ANXA3), MRNA" |
| 4.2 | "HOMO SAPIENS STATHMIN 1/ONCOPROTEIN 18 (STMN1), MRNA" |
| 4.2 | "GNL\|UG\|HS#S3837587 HOMO SAPIENS, SIMILAR TO RIBOSOMAL PROTEIN L39, CLONE MGC:20168 IMAGE:4555759, M" |
| 4.2 | "HOMO SAPIENS POLO (DROSOPHIA)-LIKE KINASE (PLK), MRNA" |

### Example 6 [Measurement of the expression of each gene in mesenchymal stem cells VI]

The expression of the gene marker of the present description in humanmesenchymal stem cells and human fibroblasts was measured in the same manner as in Example 1. The expression ratio, mesenchymal stem cell/fibroblast, is shown in Table 5. In the table, "-" indicates that the gene exhibits no or extremely low expression in mesenchymal stem cells.

**(Table 5)**

| (Mesenchymal stem cells/fibroblasts) (Gene name) | |
|---|---|
| -53.576 | "HOMO SAPIENS PROTEASOME (PROSOME, MACROPAIN) 26S SUBUNIT, ATPASE, 5 (PS" |
| -45.692 | "HOMO SAPIENS EUKARYOTIC TRANSLATION INITIATION FACTOR 4 GAMMA, 2 (EIF4G" |
| -27.6354 | "HOMO SAPIENS DIHYDROPYRIMIDINASE-LIKE 3 (DPYSL3), MRNA" |
| -20.5446 | "HOMO SAPIENS ADAPTOR-RELATED PROTEIN COMPLEX 1, SIGMA 2 SUBUNIT (AP1S2)" |
| -19.5809 | "HOMO SAPIENS PLECTIN 1, INTERMEDIATE FILAMENT BINDING PROTEIN, 500KD P" |
| -16.9025 | "HOMO SAPIENS HETEROGENEOUS NUCLEAR RIBONUCLEOPROTEIN U (SCAFFOLD ATTAC" |
| -14.97 | "HOMO SAPIENS HYPOTHETICAL PROTEIN MGC5306 (MGC5306), MRNA" |
| -14.6106 | "HOMO SAPIENS MHC CLASS I POLYPEPTIDE-RELATED SEQUENCE A (MICA), MRNA" |
| -13.8886 | "HOMO SAPIENS PROTEIN ASSOCIATED WITH PRK1 (AWP1), MRNA" |
| -13.6578 | "HOMO SAPIENS PROGESTERONE RECEPTOR MEMBRANE COMPONENT 2 (PGRMC2), MRNA" |
| -11.4783 | "HOMO SAPIENS POLYGLUTAMINE BINDING PROTEIN 1 (PQBP1), MRNA" |
| -10.2392 | "HOMO SAPIENS S-ADENOSYLMETHIONINE DECARBOXYLASE 1 (AMD1), MRNA" |
| -9.9506 | "HOMO SAPIENS INTERFERON-INDUCED, HEPATITIS C-ASSOCIATED MICROTUBULARAG" |
| -9.2594 | "HOMO SAPIENS PROTEIN KINASE, AMP-ACTIVATED, GAMMA 1 NON-CATALYTIC SUBUN" |
| -7.518 | "HOMO SAPIENS PROCOLLAGEN-PROLINE, 2-OXOGLUTARATE 4-DIOXYGENASE (PROLINE" |
| -6.0365 | "HOMO SAPIENS ENDOTHELIAL DIFFERENTIATION, LYSOPHOSPHATIDIC ACID G-PROTE" |
| -5.4442 | "HOMO SAPIENS KIAA0127 GENE PRODUCT (KIAA0127), MRNA""" |

### Example 7 [Measurement of the expression of mesenchymal stem cell DR in human cells]

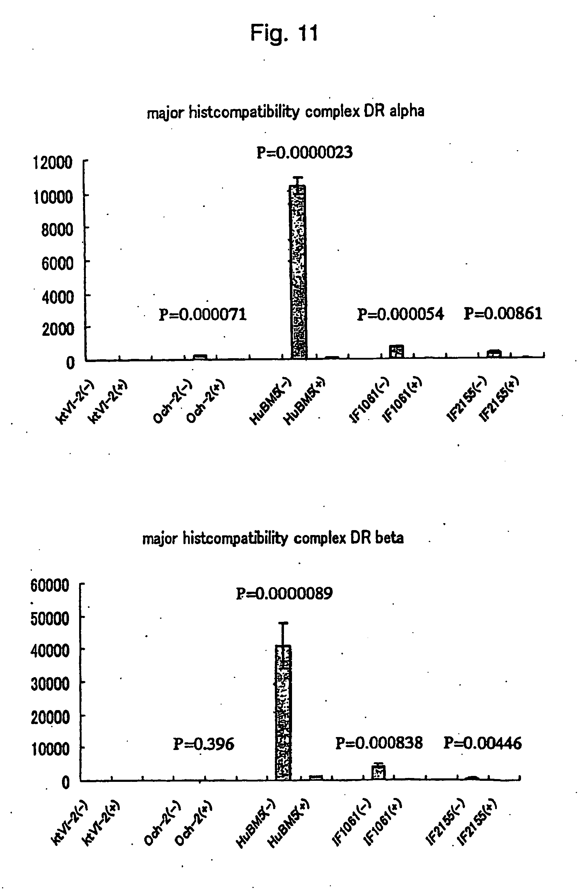

The expression of mesenchymal stem cell DR in human cells was measured with flow cytometry. The results are shown in Fig. 11.

### Example 8 [Changes in the expression of each gene in differentiation induction of mesenchymal stem cells]

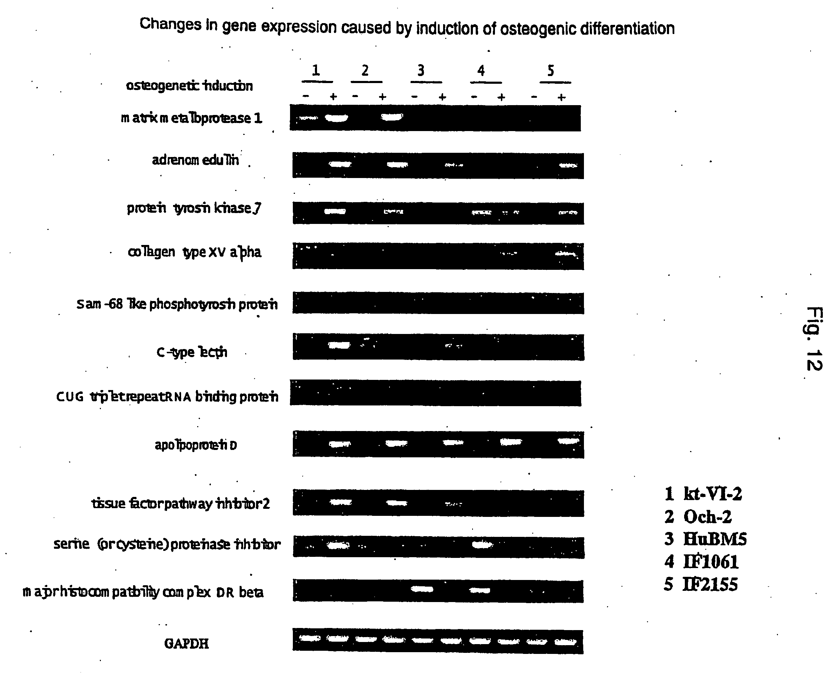

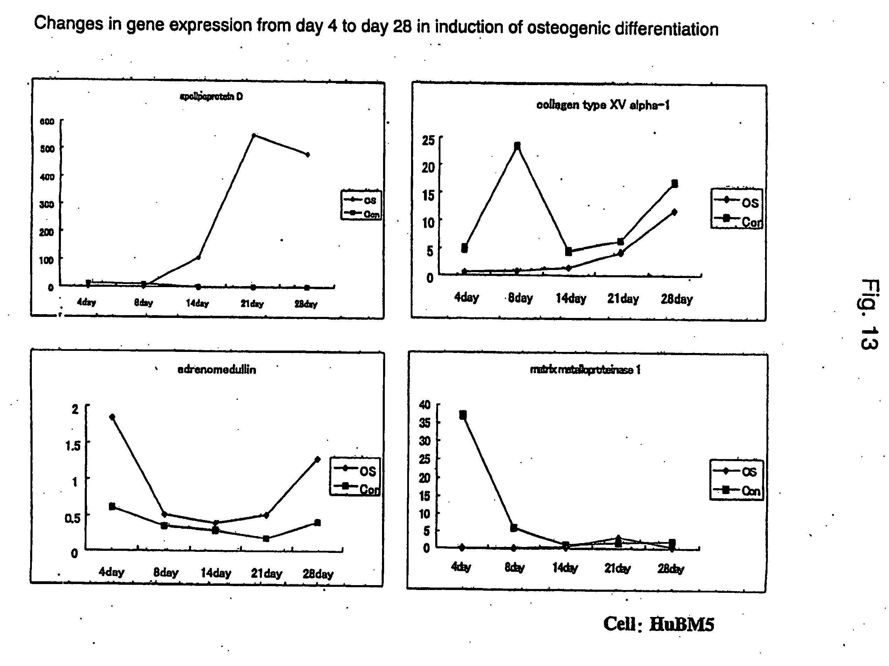

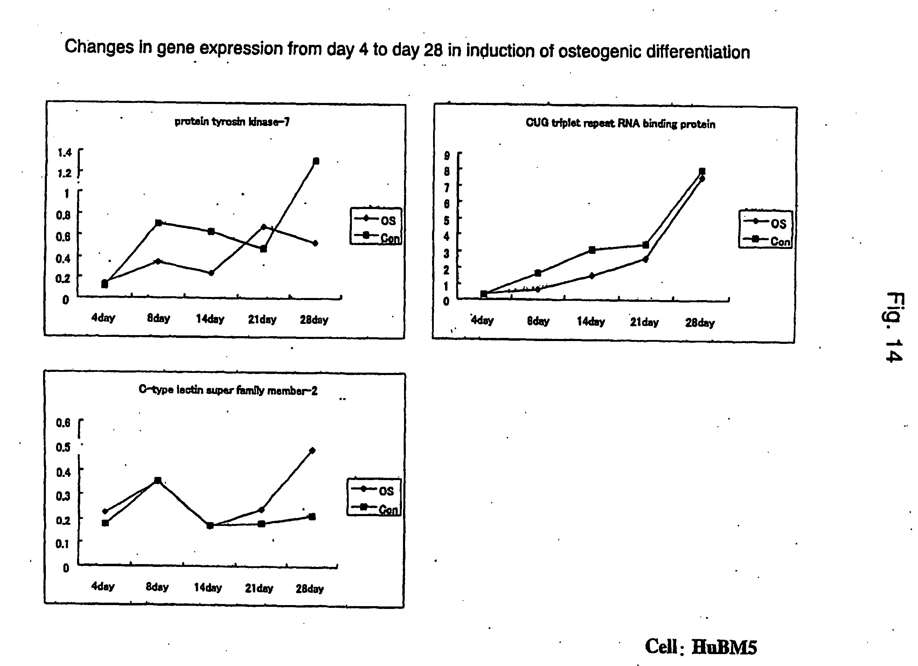

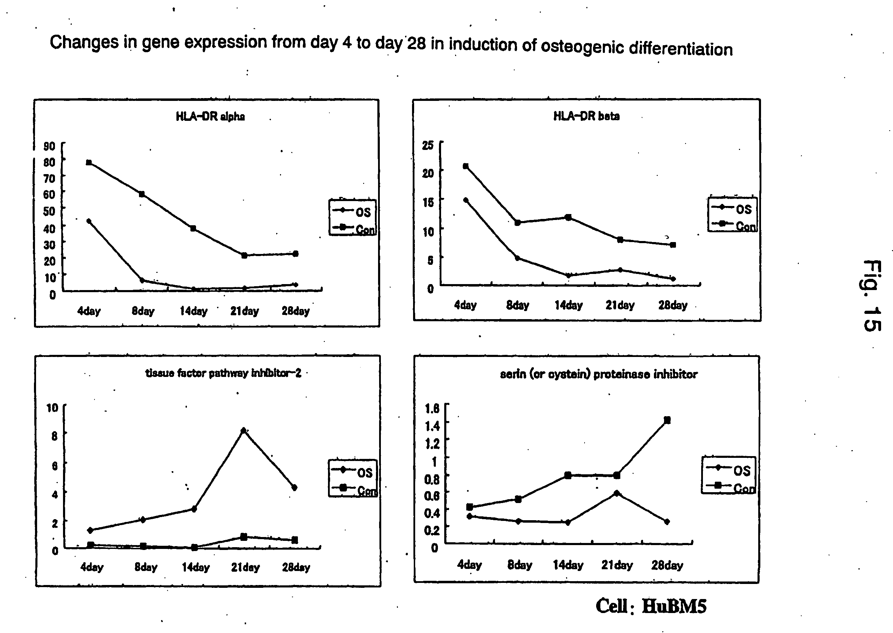

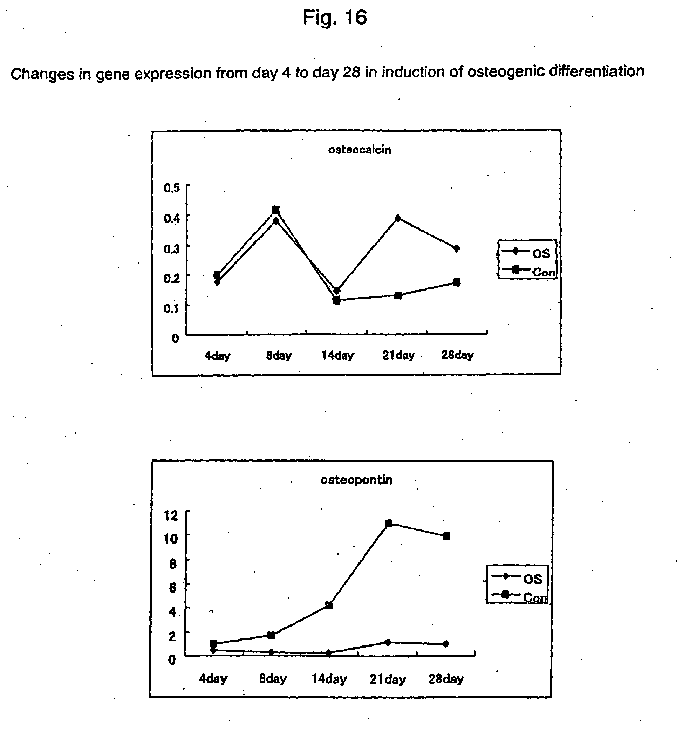

The changes in the expression of each gene in differentiation induction of mesenchymal stem cells were measured by using RT-PCR in the same manner as in Example 1. The expressions of each gene in differentiation induction of osteoblasts are shown in Figs. 12 to 16.

### Industrial Applicability

Recently, with the development of regenerative medicine, mesenchymal stem cells draw attention as xenograft, homograft and autograft materials for regenerative medicine of many tissues. In order to use mesenchymal stem cells for regenerative medicine of tissues, it is necessary to develop techniques for securing a sufficient amount of mesenchymal stem cells, and to confirm that the cultured cells are mesenchymal stem cells, and a method for detecting and distinguishing the mesenchymal stem cells becomes important. It is also helpful for separating mesenchymal stem cells from a group of many kinds of cells by using antibodies, etc. Marker genes which characterize mesenchymal stem cells are identified in the present description, and as a result, it becomes possible to detect and distinguish mesenchymal stem cells easily and assuredly, and thereby substantial contribution to the practical use of mesenchymal stem cells in regenerative medicine is expected.

### SUMMARY PARAGRAPHS

The present invention is defined in the claims and the accompanying description.

For convenience other aspects of the present description are presented herein by way of numbered paragraphs (paras) 1- 32, which are not part of the claimed subject-matter.
1. A gene marker for detecting a mesenchymal stem cell which is a gene having a base sequence shown in SEQ ID NO: 1, 2, 4, 6, 7, 9, 10, 11, 12, 14, 15, 17, 19 or 46 in the sequence listing.
2. A gene marker for detecting a mesenchymal stem cell which is a gene of INTEGRIN, ALPHA 6 (ITGA6), MRNA (NO_000210); a gene of SOLUTE CARRIER FAMILY 20 (PHOSPHATE TRANSPORTER) ; MEMBER 1 (NM_005415); a gene of RIBONUCLEOTIDE REDUCTASE M2 POLYPEPTIDE (RRM2), MRNA(NM_001034) ; agene of FOLLISTATIN (FST), TRANSCRIPT VARIANT FST317, MRNA (M_006350); a gene of SPROUTY (DROSOPHILA) HOMOLOG 2 (SPRY2), MRNA (NM_005842); a gene of RAB3B, MEMBER RAS ONCOGENE FAMILY (RAB3B) , MRNA (NM_002867); a gene of SOLUTE CARRIER FAMILY 2 (FACILITATED GLUCOSE TRANSPORTER) (NM_006516) ; a gene of INTERLEUKIN 13 RECEPTOR, ALPHA 2 (TL13RA2), MRNA (NM_000640); a gene of SERINE/THREONINE KINASE 12 (STK12) , MRNA (NM_004217) ; a gene of MINICHROMOSOME MAINTENANCE DEFICIENT (S. CEREVISIAE) 5 (CE) (NM_006739); a gene of THYROID HORMONE RECEPTOR INTERACTOR 13 (TRIP13), MRNA (NM_004237); a gene of KINESIN-LIKE 6 (MITOTIC CENTROMERE-ASSOCIATED KINESIN) (K) (NM_006845); a gene of CYCLIN-DEPENDENT KINASE INHIBITOR 3 (CDK2-ASSOCIATED DUAL (NM_005192); a gene of CHROMOSOME CONDENSATION PROTEIN G (HCAP-G), MRNA (NM_022346); a gene of CDC28 PROTEIN KINASE 1 (CKS1) , MRNA (NM_001826) ; a gene of PROTEIN REGULATOR OF CYTOKINESIS 1 (PRC1), MRNA (NM_003981); a gene of CELL DIVISION CYCLE 2, G1 TO S AND G2 TO M (CDC2) , MRNA (M_001786); a gene of CDC20 (CELL DIVISION CYCLE 20, S. CEREVISIAE, HOMOLOG) (CD) (NM_001255) ; a gene of LIKELY ORTHOLOG OF MATERNAL EMBRYONIC LEUCINE ZIPPER KINAS (NM_014791); a gene of MINICHROMOSOME MAINTENANCE DEFICIENT (S. CEREVISIAE) 7 (M) (NM_005916) ; a gene of CYCLINA2 (CCNA2), MRNA (NM_001237); a gene of THYMIDINE KINASE 1. SOLUBLE (TK1), MRNA (NM_003258); or a gene of cyclin-dependent kinase inhibitor 2A (melanoma, p16, inhibits CDK4) (CDKN2A), mRNA (NM_000077).
3. A gene marker for detecting a mesenchymal stem cell which is a gene of EGF-containing fibulin-like extracellular matrix protein 1 (NM_018894) ; a gene of Human insulin-like growth factor binding protein 5 (IGFBP5) mRNA (AU132011); a gene of Homo sapiens clone 24775mRNA sequence (AA402981); proteoglycan 1, secretory granule (AV734015) ; a gene of insulin-like growth factor binding protein 5 (AA374325) ; a gene of solute carrier family 21 (organic anion transporter), member 3 (AF085224); a gene of transglutaminase 2 (C polypeptide, protein-glutamine-gamma-glutamyl transferase) (AL552373); a gene of coagulation factor II (thrombin) receptor (M62424) ; a gene of plasminogen activator, urokinase (NM_002658); a gene of tissue inhibitor of metalloproteinase 3 (Sorsby fundus dystrophy, pseudoinflammatory) (W96324) ; a gene of matrix Gla protein (BF668572) ; a gene of B-cell CLL/lymphoma 1 (Z23022) ; a gene of CD74 antigen (invariant polypeptide of major histocompatibility complex, class II antigen-associated) (BG333618); a gene of adducin 3 (gamma) (AL135243); a gene of solute carrier family 16 (monocarboxylic acid transporter), member 4 (M_004696); a gene of protein S (alpha) (NM_000313) ; a gene of phosphatidylinositol (4,5) bisphosphate 5-phosphatase homolog; phosphatidylinositol polyphosphate 5-phosphatase type IV (AF187891) ; a gene of progesterone membrane binding protein (BE858855) ; a gene of brain-derived neurotrophic factor (X60201); or a gene of apolipoprotein E (BF967316).
4. A gene marker for detecting a mesenchymal stem cell which is a gene of HOMO SAPIENS WNT INHIBITORY FACTOR-1 (WIF-1), MRNA(NM_007191); a gene of HOMO SAPIENS HYPOTHETICAL PROTEIN FLJ11175 (FLJ11175) , MRNA (NM_018349); a gene of HOMO SAPIENS RHO GDP DISSOCIATION INHIBITOR (GDI) BETA (ARHGDIB), MRNA (NM_001175); a gene of HOMO SAPIENS POTASSIUM INTERMEDIATE/SMALL CONDUCTANCE CALCIUM-ACTIVATE (NM_021614); a gene of HOMO SAPIENS NUCLEAR FACTOR (ERYTHROID-DERIVED 2)-LIKE 3 (NFE2L3), MRNA (NM_004289); a gene of HOMO SAPIENS GS3955 PROTEIN (GS3955), MRNA (NM_021643); a gene of HOMO SAPIENS CDNA 3' END SIMILAR TO SIMILAR TO GLYCOPROTEIN MUC18 (AA302605); a gene of HOMO SAPIENS HYPOTHETICAL PROTEIN FLJ21841 (FLJ21841), MRNA (NM_024609); a gene of HOMO SAPIENS ZINC FINGER PROTEIN 185 (LIM DOMAIN) (ZNF185), MRNA (NM_007150); a gene of SC1 = PUTATIVE TRANS-ACTING FACTOR INVOLVED IN CELL CYCLE CONTROL [HUMAN, MRNA, 24] (S53374); a gene of HOMO SAPIENS NADH DEHYDROGENASE (UBIQUINONE) 1 ALPHA SUBCOMPLEX, 8 (19K) (NM_014222); a gene of HOMO SAPIENS HOMOLOG OF YEAST MCM10; HYPOTHETICAL PROTEIN PRO2249 (PR02) (NM_018518); a gene of HOMO SAPIENS COLLAGEN, TYPE VII, ALPHA 1 (EPIDERMOLYSIS BULLOSA, DYSTRO (NM_000094); a gene of HOMO SAPIENS AUTOCRINE MOTILITY FACTOR RECEPTOR (AMFR) , MRNA (NM_001144); ageneof HOMO SAPIENS CLONE 24421 MRNA SEQUENCE /CDS = UNKNOWN /GB = AF070641 /GI = 3283914 /UG = (AF070641) ; a gene of HOMO SAPIENS MRNA; a gene of CDNA DKFZP586E1621 (FROM CLONE DKFZP586E1621) /CDS = UNKNOWN /GB (AL080235).
5. A gene marker for detecting a mesenchymal stem cell which is a gene of HOMO SAPIENS RESERVED (KCNK12) , MRNA (NM_022055); a gene of HOMO SAPIENS UBIQUITIN-CONJUGATING ENZYME E2C (UBE2C), MRNA (NM_007019) ; a gene of HOMO SAPIENS CDNA FLJ10517 FIS, CLONE NT2RP2000812 /CDS = (61,918) /GB = AK001379 /G (AX001379); a gene of HOMO SAPIENS CARTILAGE LINKING PROTEIN 1 (CRTL1), MRNA (NM_001884); a gene of HOMO SAPIENS MRNA; CDNA DKFZP434F2322 (FROM CLONE DKFZP434F2322) /CDS = UNKNOWN /GB (AL133105); a gene of HOMO SAPIENS, CLONE MGC:9549 IMAGE:3857382, MRNA, COMPLETE CDS /CDS = (92,1285) /G (BC012453); a gene of HOMO SAPIENS HYPOTHETICAL PROTEIN DKFZP586J1119 (DKFZP586J1119), MRNA (NM_032270); a gene of HOMO SAPIENS GLIA MATURATION FACTOR, GAMMA (GMFG), MRNA (NM_004877); a gene of HOMO SAPIENS CDNA: FLJ23095 FIS, CLONE LNG07413 /CDS = UNKNOWN /GB = AK026748 /GI = 10 (AK026748); a gene of HOMO SAPIENS HYPOTHETICAL PROTEIN DKFZP762E1312 (DKFZP762E1312), MRNA (NM_018410) ; a gene of HOMO SAPIENS IROQUOIS-CLASS HOMEODOMAIN PROTEIN (IRX-2A), MRNA (NM_005853); or a gene of HOMO SAPIENS HYPOTHETICAL PROTEIN FLJ10540 (FLJ10540), MRNA (NM_018131).
6. A gene marker for detecting a mesenchymal stem cell which is a gene of HOMO SAPIENS MRNA; CDNA DKFZP434C1915 (FROM CLONE DKFZP434C1915) ; PARTIAL CDS /C (AL137698) ; a gene of HOMO SAPIENS MINICHROMOSOME MAINTENANCE DEFICIENT (S. CEREVISIAE) 7 (M) (NM_005916); a gene of HOMO SAPIENS DYNEIN, CYTOPLASMIC, INTERMEDIATE POLYPEPTIDE 1 (DNCI1), M (NM_004411); a gene of HUMAN MRNA FOR PROTEIN GENE PRODUCT (PGP) 9.5. (X04741); a gene of HOMO SAPIENS RAD51-INTERACTING PROTEIN (PIR51), MRNA (M_006479); a gene of HOMO SAPIENS BACULOVIRAL IAP REPEAT-CONTAINING 5 (SURVIVIN) (BIRC5), MR (NM_001168); a gene of HOMO SAPIENS UDP-N-ACETYL-ALPHA-D-GALACTOSAMINE:POLYPEPTIDE N-ACETYLGAL (NM_004482); a gene of HOMO SAPIENS CYCLIN B1 (CCNB1), MRNA (NM_031966); a gene of HOMO SAPIENS FLAP STRUCTURE-SPECIFIC ENDONUCLEASE 1 (FEN1), MRNA (NM_004111) ; a gene of HOMO SAPIENS SERINE/THREONINE KINASE 15 (STK15), MRNA (NM_003600); a gene of HOMO SAPIENS MAD2 (MITOTIC ARREST DEFICIENT, YEAST, HOMOLOG)-LIKE 1 (MA) (NM_002358); a gene of HOMO SAPIENS NUCLEOLAR PROTEIN ANKT (ANKT), MRNA (NM_018454) ; a gene of HOMO SAPIENS HSPC150 PROTEIN SIMILAR TO UBIQUITIN-CONJUGATING ENZYME (H) (NM_014176) ; a gene of HOMO SAPIENS CYTOCHROME P450 RETINOID METABOLIZING PROTEIN (P450RAI-2), (NM_019885); a gene of HOMO SAPIENS NIMA (NEVER IN MITOSIS GENE A) -RELATED KINASE 2 (NEK2), MR (NM_002497); a gene of HOMO SAPIENS BONE MORPHOGENETIC PROTEIN 4 (BMP4), MRNA (NM_001202); a gene of HOMO SAPIENS CDNA FLJ10674 FIS, CLONE NT2RP2006436 /CDS = UNKNOWN /GB = AK001536 /GI (AK001536) ; or a gene of HOMO SAPIENS CENTROMERE PROTEIN A (17 KD) (CENPA), MRNA (NM_001809).
7. A gene marker for detecting a mesenchymal stem cell which is a gene of HOMO SAPIENS MRNA; CDNA DKFZP564P116 (FROM CLONE DKFZP564P116) /CDS = UNKNOWN /GB = A (AL049338) ; a gene of HOMO SAPIENS TTK PROTEIN KINASE (TTK), MRNA (NM_003318); a gene of HOMO SAPIENS KARYOPHERIN ALPHA 2 (RAG COHORT 1, IMPORTIN ALPHA 1) (KPNA (NM_002266); a gene of HOMO SAPIENS POLYMERASE (DNA DIRECTED), THETA (POLQ) , MRNA (NM_006596) ; a gene of HOMO SAPIENS ETS VARIANT GENE 5 (ETS-RELATED MOLECULE) (ETV5), MRNA (NM_004454); a gene of HOMO SAPIENS TRANSFORMING, ACIDIC COILED-COIL CONTAINING PROTEIN 3 (TAC) (NM_006342); a gene of HOMO SAPIENS KINESIN-LIKE 1 (KNSL1) , MRNA (NM_004523) ; a gene of HOMO SAPIENS CENTROMERE PROTEIN E (312 KD) (CENPE), MRNA (NM_001813) ; a gene of HOMO SAPIENS CDNA, 3' END /CONE = IMAGE:1651289 /CLONE_END = 3' /GB = AI12 (AI123815); a gene of HOMO SAPIENS DISTAL-LESS HOMEO BOX 5 (DLX5), MRNA (NM_005221); a gene of HOMO SAPIENS V-MYB AVIAN MYELOBLASTOSIS VIRAL ONCOGENE HOMOLOG-LIKE 2 (NM_002466); or a gene of HOMO SAPIENS SERUM DEPRIVATION RESPONSE (PHOSPHATIDYLSERINE-BINDING PRO (NM_004657).
8. A gene marker for detecting a mesenchymal stem cell which is a gene of HOMO SAPIENS HYPOTHETICAL PROTEIN FLJ10604 (FLJ10604), MRNA (NM_018154) ; a gene of HOMO SAPIENS RIBONUCLEASE HI, LARGE SUBUNIT (RNASEHI), MRNA (NM_006397); a gene of HOMO SAPIENS ANILLIN (DROSOPHILA SCRAPS HOMOLOG), ACTIN BINDING PROTEIN (NM_018685) ; a gene of HOMO SAPIENS H4 HISTONE FAMILY, MEMBER G (H4FG), MRNA (NM_003542); a gene of HOMO SAPIENS G PROTEIN-COUPLED RECEPTOR 37 (ENDOTHELIN RECEPTOR TYPE B-(NM_005302) HOMO SAPIENS UBIQUITIN CARRIER PROTEIN (E2-EPF), MRNA (NM_014501); a gene of HOMO SAPIENS, SIMILAR TO TUMOR DIFFERENTIALLY EXPRESSED 1, CLONE IMAGE:3639252, (BC007375); a gene of HOMO SAPIENS HYPOTHETICAL PROTEIN MGC2577 (MGC2577), MRNA (NM_031299); a gene of HOMO SAPIENS HYPOTHETICAL PROTEIN FLJ13912 (FLJ13912), MRNA (NM_022770); a gene of HOMO SAPIENS ANNEXIN A3 (ANXA3), MRNA (NM_005139); a gene of HOMO SAPIENS STATHMIN 1/ONCOPROTEIN 18 (STMN1) , MRNA (NM_005563) HOMO SAPIENS, SIMILAR TO RIBOSOMAL PROTEIN L39,CLONE MGC:20168 IMAGE:4555759, M (BC012328) ; or a gene of HOMO SAPIENS POLO (DROSOPHIA) -LIKE KINASE (PLK), MRNA (NM_005030).
9. A gene marker for detecting a mesenchymal stem cell which is a gene of HOMO SAPIENS PROTEASOME (PROSOME, MACROPAIN) 26S SUBUNIT, ATPASE, 5 (PS) (NM_002805); a gene of HOMO SAPIENS EUKARYOTIC TRANSLATION INITIATION FACTOR 4 GAMMA, 2 (EIF4G) (NM_001418); a gene of HOMO SAPIENS DIHYDROPYRIMIDINASE-LIKE 3 (DPYSL3), MRNA) (NM_001387); a gene of HOMO SAPIENS ADAPTOR-RELATED PROTEIN COMPLEX 1, SIGMA 2 SUBUNIT (AP1S2) (NM_003916); a gene of HOMO SAPIENS PLECTIN 1, INTERMEDIATE FILAMENT BINDING PROTEIN, 500 KD (P) (NM_000445); a gene of HOMO SAPIENS HETEROGENEOUS NUCLEAR RIBONUCLEOPROTEIN U (SCAFFOLD ATTAC (NM_031844) ; a gene of HOMO SAPIENS HYPOTHETICAL PROTEIN MGC5306 (MGC5306), MRNA (NM_024116); a gene of HOMO SAPIENS MHC CLASS I POLYPEPTIDE-RELATED SEQUENCE A(MLCA), MRNA (NM_000247); a gene of HOMO SAPIENS PROTEIN ASSOCIATED WITH PRK1 (AWP1), MRNA (NM_019006); a gene of HOMO SAPIENS PROGESTERONE RECEPTOR MEMBRANE COMPONENT 2 (PGRMC2), MRNA (NM_006320) ; a gene of HOMO SAPIENS POLYGLUTAMINE BINDING PROTEIN 1 (PQBP1), MRNA (NM_005710); a gene of HOMO SAPIENS S-ADENOSYLMETHIONINE DECARBOXYLASE 1 (AMD1), MRNA (NM_001634); HOMO SAPIENS INTERFERON-INDUCED, HEPATITIS C-ASSOCIATED MICROTUBULAR AG (NM_006417); a gene of HOMO SAPIENS PROTEIN KINASE, AMP-ACTIVATED, GAMMA 1 NON-CATALYTIC SUBUN (NM_002733); HOMO SAPIENS PROCOLLAGEN-PROLINE, 2-OXOGLUTARATE 4-DIOXYGENASE (PROLINE (NM_004199); a gene of HOMO SAPIENS ENDOTHELIAL DIFFERENTIATION, LYSOPHOSPHATIDIC ACID G-PROTE (NM_012152); or a gene of HOMO SAPIENS KIAA0127 GENE PRODUCT (KIAA0127), MRNA (NM_014755).
10. A probe for detecting a mesenchymal stem cell marker gene having a DNA sequence which hybridizes with the marker gene for detecting a mesenchymal stem cell according to any one of para.s 1 to 9 under a stringent condition.
11. The probe for detecting a mesenchymal stem cell marker gene according to para. 10, which comprises whole or part of an antisense strand of the base sequence according to any one of para.s 1 to 9.
12. A microarray or a DNA chip for detecting a mesenchymal stem cell marker gene, wherein at least one of the DNA according to para. 10 or 11 is immobilized.
13. The microarray or the DNA chip for detecting a mesenchymal stem cell marker gene according to para. 12, wherein a probe for detecting two or more genes in a group of genes having a base sequence shown in SEQ ID NO : 1, 2, 4, 6, 7, 9, 10, 11, 12, 14, 15, 17, 19 or 46 in the sequence listing and a group of the genes according to any one of para.s 2 to 9 is immobilized.
14. A polypeptide marker for detecting a mesenchymal stem cell which is a polypeptide having an amino acid sequence shown in SEQ ID NO: 3, 5, 8, 13, 16 or 18 in the sequence listing.
15. An antibody which is induced by using the polypeptide according to para. 14 and which specifically binds to the polypeptide.
16. The antibody according to para. 15, which is a monoclonal antibody.
17. The antibody according to para. 15, which is a polyclonal antibody.
18. A method for distinguishing a mesenchymal stem cell wherein expression of the mesenchymal stem cell marker gene according to any one of para.s 1 to 9 in a test cell is detected.
19. The method for distinguishing a mesenchymal stem cell according to para. 18, wherein the expression of the mesenchymal stem cell marker gene is detected by using Northern blotting.
20. The method for distinguishing a mesenchymal stem cell according to para 18, wherein the expression of the mesenchymal stem cell marker gene is detected by using the probe for detecting a mesenchymal stem cell marker gene according to para. 10 or 11.
21. The method for distinguishing a mesenchymal stem cell according to para. 18, wherein the expression of the mesenchymal stem cell marker gene is detected by using the microarray or the DNA chip for detecting a mesenchymal steam cell marker gene according to para. 12 or 13.
22. A method for distinguishing a mesenchymal stem cell, wherein the detection of the expression of the mesenchymal stem cell marker gene according to any one of para.s 18 to 21 comprises use of quantitative or semiquantitative PCR.
23. The method for distinguishing a mesenchymal stem cell according to para. 22, wherein the use of quantitative or semiquantitative PCR is RT-PCR method.
24. An RT-PCR primer for amplifying a mesenchymal stem cell marker gene which comprises a sense primer having a base sequence shown in SEQ ID NO: 20 in the sequence listing, and an antisense primer having a base sequence shown in SEQ ID NO: 21 in the sequence listing; a sense primer having a base sequence shown in SEQ ID NO: 22 in the sequence listing, and an antisense primer having a base sequence shown in SEQ ID NO: 23 in the sequence listing; a sense primer having a base sequence shown in SEQ ID NO: 24 in the sequence listing, and an antisense primer having a base sequence shown in SEQ ID NO: 25 in the sequence listing; a sense primer having a base sequence shown in SEQ ID NO: 26 in the sequence listing, and an antisense primer having a base sequence shown in SEQ ID NO: 27 in the sequence listing; a sense primer having a base sequence shown in SEQ ID NO: 28 in the sequence listing, and an antisense primer having a base sequence shown in SEQ ID NO: 29 in the sequence listing; a sense primer having a base sequence shown in SEQ ID NO: 30 in the sequence listing, and an antisense primer having a base sequence shown in SEQ ID NO: 31 in the sequence listing; a sense primer having a base sequence shown in SEQ ID NO: 32 in the sequence listing, and an antisense primer having a base sequence shown in SEQ ID NO: 33 in the sequence listing; a sense primer having a base sequence shown in SEQ ID NO: 34 in the sequence listing, and an antisense primer having a base sequence shown in SEQ ID NO: 35 in the sequence listing; a sense primer having a base sequence shown in SEQ ID NO: 36 in the sequence listing, and an antisense primer having a base sequence shown in SEQ ID NO: 37 in the sequence listing; a sense primer having a base sequence shown in SEQ ID NO: 38 in the sequence listing, and an antisense primer having a base sequence shown in SEQ ID NO: 39 in the sequence listing; a sense primer having a base sequence shown in SEQ ID NO: 40 in the sequence listing, and an antisense primer having a base sequence shown in SEQ ID NO: 41 in the sequence listing; a sense primer having a base sequence shown in SEQ ID NO: 42 in the sequence listing, and an antisense primer having a base sequence shown in SEQ ID NO: 43 in the sequence listing; or a sense primer having a base sequence shown in SEQ ID NO: 44 in the sequence listing, and an antisense primer having a base sequence shown in SEQ ID NO: 45 in the sequence listing.
25. A real time PCR primer for amplifying a mesenchymal stem cell marker gene which comprises a sense primer having a base sequence shown in SEQ ID NO: 47 in the sequence listing, and an antisense primer having a base sequence shown in SEQ ID NO: 48 in the sequence listing; a sense primer having a base sequence shown in SEQ ID NO: 49 in the sequence listing, and an antisense primer having a base sequence shown in SEQ ID NO: 50 in the sequence listing; a sense primer having a base sequence shown in SEQ ID NO: 51 in the sequence listing, and an antisense primer having a base sequence shown in SEQ ID NO: 52 in the sequence listing; a sense primer having a base sequence shown in SEQ ID NO: 53 in the sequence listing, and an antisense primer having a base sequence shown in SEQ ID NO: 54 in the sequence listing; a sense primer having a base sequence shown in SEQ ID NO: 55 in the sequence listing, and an antisense primer having a base sequence shown in SEQ ID NO: 56 in the sequence listing; a sense primer having a base sequence shown in SEQ ID NO: 57 in the sequence listing, and an antisense primer having a base sequence shown in SEQ ID NO: 58 in the sequence listing; a sense primer having a base sequence shown in SEQ ID NO: 59 in the sequence listing, and an antisense primer having a base sequence shown in SEQ ID NO: 60 in the sequence listing; a sense primer having a base sequence shown in SEQ ID NO: 61 in the sequence listing, and an antisense primer having a base sequence shown in SEQ ID NO: 62 in the sequence listing; a sense primer having a base sequence shown in SEQ ID NO: 63 in the sequence listing, and an antisense primer having a base sequence shown in SEQ ID NO: 64 in the sequence listing; a sense primer having a base sequence shown in SEQ ID NO: 65 in the sequence listing, and an antisense primer having a base sequence shown in SEQ ID NO: 66 in the sequence listing; a sense primer having a base sequence shown in SEQ ID NO: 67 in the sequence listing, and an antisense primer having a base sequence shown in SEQ ID NO: 68 in the sequence listing; or a sense primer having a base sequence shown in SEQ ID NO: 69 in the sequence listing, and an antisense primer having a base sequence shown in SEQ ID NO: 70 in the sequence listing.
26. An RT-PCR primer for amplifying a mesenchymal stem cell marker gene which comprises a sense primer having a base sequence shown in SEQ ID NO: 71 in the sequence listing, and an antisense primer having a base sequence shown in SEQ ID NO: 72 in the sequence listing; a sense primer having a base sequence shown in SEQ ID NO: 73 in the sequence listing, and an antisense primer having a base sequence shown in SEQ ID NO: 74 in the sequence listing; a sense primer having a base sequence shown in SEQ ID NO: 75 in the sequence listing, and an antisense primer having a base sequence shown in SEQ ID NO: 76 in the sequence listing; a sense primer having a base sequence shown in SEQ ID NO: 77 in the sequence listing, and an antisense primer having a base sequence shown in SEQ ID NO: 78 in the sequence listing; a sense primer having a base sequence shown in SEQ ID NO: 79 in the sequence listing, and an antisense primer having a base sequence shown in SEQ ID NO: 80 in the sequence listing; a sense primer having a base sequence shown in SEQ ID NO: 81 in the sequence listing, and an antisense primer having a base sequence shown in SEQ ID NO: 82 in the sequence listing; a sense primer having a base sequence shown in SEQ ID NO: 83 in the sequence listing, and an antisense primer having a base sequence shown in SEQ ID NO: 84 in the sequence listing; a sense primer having a base sequence shown in SEQ ID NO: 85 in the sequence listing, and an antisense primer having a base sequence shown in SEQ ID NO: 86 in the sequence listing; a sense primer having a base sequence shown in SEQ ID NO: 87 in the sequence listing, and an antisense primer having a base sequence shown in SEQ ID NO: 88 in the sequence listing; a sense primer having a base sequence shown in SEQ ID NO: 89 in the sequence listing, and an antisense primer having a base sequence shown in SEQ ID NO: 90 in the sequence listing; a sense primer having a base sequence shown in SEQ ID NO: 91 in the sequence listing, and an antisense primer having a base sequence shown in SEQ ID NO: 92 in the sequence listing; a sense primer having a base sequence shown in SEQ ID NO: 93 in the sequence listing, and an antisense primer having a base sequence shown in SEQ ID NO: 94 in the sequence listing; a sense primer having a base sequence shown in SEQ ID NO: 95 in the sequence listing, and an antisense primer having a base sequence shown in SEQ ID NO: 96 in the sequence listing; a sense primer having a base sequence shown in SEQ ID NO: 97 in the sequence listing, and an antisense primer having a base sequence shown in SEQ ID NO: 98 in the sequence listing; a sense primer having a base sequence shown in SEQ ID NO: 99 in the sequence listing, and an antisense primer having a base sequence shown in SEQ ID NO: 100 in the sequence listing; a sense primer having a base sequence shown in SEQ ID NO: 101 in the sequence listing, and an antisense primer having a base sequence shown in SEQ ID NO: 102 in the sequence listing; a sense primer having a base sequence shown in SEQ ID NO: 103 in the sequence listing, and an antisense primer having a base sequence shown in SEQ ID NO: 104 in the sequence listing; a sense primer having a base sequence shown in SEQ ID NO: 105 in the sequence listing, and an antisense primer having a base sequence shown in SEQ ID NO: 106 in the sequence listing; a sense primer having a base sequence shown in SEQ ID NO: 107 in the sequence listing, and an antisense primer having a base sequence shown in SEQ ID NO: 108 in the sequence listing; a sense primer having abase sequence shown in SEQ ID NO: 109 in the sequence listing, and an antisense primer having a base sequence shown in SEQ ID NO: 110 in the sequence listing; a sense primer having a base sequence shown in SEQ ID NO: 111 in the sequence listing, and an antisense primer having a base sequence shown in SEQ ID NO: 112 in the sequence listing; a sense primer having a base sequence shown in SEQ ID NO: 113 in the sequence listing, and an antisense primer having a base sequence shown in SEQ ID NO: 114 in the sequence listing; or a sense primer having a base sequence shown in SEQ ID NO: 115 in the sequence listing, and an antisense primer having a base sequence shown in SEQ ID NO: 116 in the sequence listing.
27. The method for distinguishing a mesenchymal stem cell according to para 18, wherein a gene in a test cell is amplified by using at least one pair of primers comprising the sense primer and the antisense primer according to any one of para.s 24 to 26, and the amplified gene is detected by using the probe for detecting a mesenchymal stem cell marker gene according to para. 10 or 11.
28. A method for distinguishing a mesenchymal stem cell, wherein expression of a mesenchymal stem cell marker polypeptide in a test cell is detected by using the antibody according to any one of para.s 15 to 17.
29. A method for distinguishing a mesenchymal stem cell, wherein expression of one or more genes in a group of mesenchymal stem cell marker genes comprising SEQ ID NOs: 1, 11 and 19 in the sequence listing, and expression of one or more genes in a group of mesenchymal stem cell marker genes comprising SEQ ID NOs: 2, 4, 6, 7, 9, 10, 12, 14, 15 and 17 in the sequence listing, in a test cell, are evaluated in combination.
30. A method for distinguishing and separating a mesenchymal stem cell, wherein expression of a marker gene for detecting a mesenchymal stem cell and/or a marker polypeptide for detecting a mesenchymal stem cell in a test cell is detected by using the probe for detecting a mesenchymal stem cellmarker gene according to para. 10 or 11 and/or the antibody according to any one of para.s 15 to 17, and the distinguished mesenchymal stem cell is separated.
31. The method for distinguishing and separating a mesenchymal stem cell according to para. 30, wherein a mesenchymal stem cell in a test cell is labeled by a fluorescent antibody method using the antibody according to any one of para.s 15 to 17, and the labeled mesenchymal stem cell is separated.
32. A kit for distinguishing a mesenchymal stem cell which comprises at least one of the probe for detecting a mesenchymal stem cell marker gene according to para. 10 or 11, the microarray or the DNA chip for detecting a mesenchymal stem cell marker gene according to para. 12 or 13 in which.the probe is immobilized, and the antibody according to any one of para.s 15 to 17.

## Claims

1. A method for distinguishing a mesenchymal stem cell from fibroblasts *in vitro,* wherein expression of all of the following mesenchymal stem cell marker genes in a test cell are detected; apolipoprotein D (SEQ ID NO 4 in the sequence listing); collagen type XV alpha 1 (SEQ ID NO 6 in the sequence listing); CUG triplet repeat RNA-binding protein 2 (SEQ ID NO 7 in the sequence listing); matrix metalloprotease 1 (SEQ ID NO 17 in the sequence listing); Homo sapiens bone morphogenetic protein 4 (BMP4: Gen Bank Acc. No. NM_001202); Homo sapiens clone 24775 mRNA sequence (Gen Bank Acc. No.AA402981); matrix Gla protein (MGP: Gen Bank Acc. No.BF668572); proteoglycan 1, secretory granule (Gen Bank Acc. No.AV734015); Homo sapiens GS3955 protein (Gen Bank Acc. No. NM_021643); Homo sapiens dynein, cytoplasmic, intermediate polypeptide 1 (DNCI1: Gen Bank Acc. No. M_004411); and, brain-derived neurotrophic factor (Gen Bank Acc. No. X60201).

2. The method for distinguishing a mesenchymal stem cell from fibroblasts according to claim 1, wherein the expression of the mesenchymal stem cell marker genes are detected by using a probe for detecting mesenchymal stem cell marker genes having a DNA sequence which hybridizes under stringent conditions with one of the mesenchymal stem cell marker genes, each having a base sequence shown in SEQ ID NO. 4, 6, 7 or 17 in the sequence listing, or Gen Bank Acc. No. NM_001202, AA402981, BF668572, AV734015, NM_021643, NM_004411 or X60201 or whole or part of an antisense strand of the base sequence shown in SEQ ID NO. 4, 6, 7 or 17 in the sequence listing, or Gen Bank Acc. No. NM_001202, AA402981, BF668572, AV734015, NM_021643, NM_004411 or X60201.

3. The method for distinguishing a mesenchymal stem cell from fibroblasts according to claim 1, wherein the expression of the mesenchymal stem cell marker genes are detected by using a microarray or a DNA chip for detecting mesenchymal stem cell marker genes, each having a base sequence shown in SEQ ID NO. 4, 6, 7 or 17 in the sequence listing, or Gen Bank Acc. No. NM_001202, AA402981, BF668572, AV734015, M_021643, NM_004411 or X60201.

4. The method for distinguishing a mesenchymal stem cell from fibroblasts according to any one of claims 1 to 3, wherein the detection of the expression of the mesenchymal stem cell marker genes comprises the use of quantitative or semiquantitative PCR.

5. The method for distinguishing a mesenchymal stem cell from fibroblasts according to claim 4 wherein the quantitative or semiquantitative PCR is an RT-PCR or real time PCR method.

6. The method for distinguishing a mesenchymal stem cell from fibroblasts according to claim 5, wherein said RT-PCR method comprises a pair of sense and antisense primers for amplifying said stem cell marker genes, each having a base sequence shown in SEQ ID NO. 4, 6, 7 or 17, wherein said primers are:
a sense primer having a base sequence shown in SEQ ID NO: 24 in the sequence listing and an antisense primer having a base sequence shown in SEQ ID NO: 25 in the sequence listing;
a sense primer having a base sequence shown in SEQ ID NO.26 in the sequence listing, and an antisense primer having a base sequence shown in SEQ ID NO: 27 in the sequence listing;
a sense primer having a base sequence shown in SEQ ID NO: 28 in the sequence listing, and an antisense primer having a base sequence shown in SEQ ID NO: 29 in the sequence listing; and
a sense primer having a base sequence shown in SEQ ID NO: 42 in the sequence listing, and an antisense primer having a base sequence shown in SEQ ID NO: 43 in the sequence listing.

7. The method for distinguishing a mesenchymal stem cell from fibroblasts according to claim 5, wherein said real time PCR method comprises a pair of sense and antisense primers for amplifying said stem cell marker genes, each having a base sequence shown in SEQ ID NO. 4, 6, 7 or 17 wherein said primers are:
a sense primer having a base sequence shown in SEQ ID NO: 49 in the sequence listing, and an antisense primer having a base sequence shown in SEQ 10 NO:50 in the sequence listing;
a sense primer having a base sequence shown in SEQ ID NO: 57 in the sequence listing, and an antisense primer having a base sequence shown in SEQ ID NO:58 in the sequence listing;
a sense primer having a base sequence shown in SEQ ID NO: 61 in the sequence listing, and an antisense primer having a base sequence shown in SEQ ID NO: 62 in the sequence listing; and,
a sense primer having a base sequence shown in SEQ ID NO: 69 in the sequence listing and an antisense primer having a base sequence shown in SEQ ID NO:70 in the sequence listing.

8. The method for distinguishing a mesenchymal stem cell from fibroblasts according to claim 1, wherein the genes in a test cell are amplified by using at least one pair of primers for each gene, comprising the sense primer and the antisense primer according to claim 6 or claim 7, and the amplified genes are detected by using a probe for detecting a mesenchymal stem cell marker genes.

## Patentansprüche

1. Verfahren zum Unterscheiden einer mesenchymalen Stammzelle von Fibroblasten *in vitro,* wobei die Expression von allen der folgenden mesenchymalen Stammzell-Markergenen in einer Testzelle erfasst wird: Apolipoprotein D (SEQ ID Nr. 4 in dem Sequenzprotokoll); Kollagen Typ XV alpha 1 (SEQ ID Nr. 6 in dem Sequenzprotokoll); CUG-Triplet-Wiederholung-RNA-bindendes Protein 2 (SEQ ID Nr. 7 in dem Sequenzprotokoll); Matrix-Metalloprotease 1 (SEQ ID Nr. 17 in dem Sequenzprotokoll); Homo sapiens-Morphogenetisches Knochenprotein 4 (BMP4: Gen Bank Zugangsnummer NM_001202); Homo sapiens-Klon 24775 mRNA-Sequenz (Gen Bank Zugangsnummer AA402981); Matrix-Gla-Protein (MGP: Gen Bank Zugangsnummer BF668572); Proteoglycan 1, Sekretorisches Granulum (Gen Bank Zugangsnummer AV734015); Homo sapiens-GS3955-Protein (Gen Bank Zugangsnummer NM_021643); Homo sapiens-Dynein, zytoplasmatisches intermediäres Polypeptid 1 (DNCI1: Gen Bank Zugangsnummer Nu_004411) und hirnabstammender neurotropher Faktor (Gen Bank Zugangsnummer X60201).

2. Verfahren zum Unterscheiden einer mesenchymalen Stammzelle von Fibroblasten nach Anspruch 1, wobei die Expression der mesenchymalen Stammzell-Markergene erfasst wird unter Verwendung einer Sonde zum Erfassen von mesenchymalen Stammzell-Markergenen mit einer DNA-Sequenz, die unter stringenten Bedingungen mit einem der mesenchymalen Stammzell-Markergenen hybridisiert, von denen jedes eine Basensequenz aufweist, die in SEQ ID Nr. 4, 6, 7 oder 17 im Sequenzprotokoll dargestellt ist, oder die die Gen Bank Zugangsnummer NM_001202, AA402981, BF668572, AV734015, NM_021643, NM_004411 oder X60201 aufweist, oder die vollständig oder teilweise einen Antisense-Strang der Basensequenz aufweist, die in SEQ ID Nr. 4, 6, 7 oder 17 in dem Sequenzprotokoll dargestellt ist, oder die Gen Bank Zugangsnummer NM_001202, AA402981, BF668572, AV734015, NM_021643, NM_004411 oder X60201.

3. Verfahren zum Unterscheiden einer mesenchymalen Stammzelle von Fibroblasten nach Anspruch 1, wobei die Expression der mesenchymalen Stammzell-Markergene erfasst wird unter Anwendung eines Mikroarrays oder eines DNA-Chips zum Erfassen von mesenchylen Stammzell-Markergenen, von denen jedes eine Basensequenz aufweist, die in den SEQ ID Nr. 4, 6, 7 oder 17 in dem Sequenzprotokoll dargestellt ist oder die Gen Bank Zugangsnummer NM_001202, AA402981, BF668572, AV734015, NM_021643, NM_004411 oder X60201.

4. Verfahren zum Unterscheiden einer mesenchymalen Stammzelle von Fibroblasten nach einem der Ansprüche 1 bis 3, wobei die Erfassung der Expression der mesenchymalen Stammzell-Markergene die Anwendung von quantitativer oder semiquantitativer PCR umfasst.

5. Verfahren zum Unterscheiden einer mesenchymalen Stammzelle von Fibroblasten nach Anspruch 4, wobei die quantitative oder semiquantitative PCR ein RT-PCR- oder ein Echtzeit-PCR-Verfahren ist.

6. Verfahren zum Unterscheiden einer mesenchymalen Stammzelle von Fibroblasten nach Anspruch 5, wobei das RT-PCR-Verfahren ein Paar von Sense- und Antisense-Primern zum Amplifizieren der Stammzell-Markergene umfasst, wobei jedes eine Basensequenz aufweist, die in SEQ ID Nr. 4, 6, 7 oder 17 dargestellt ist, wobei die Primer die folgenden sind:
ein Sense-Primer mit einer Basensequenz, wie sie in SEQ ID Nr. 24 in dem Sequenzprotokoll dargestellt ist, und ein Antisense-Primer mit einer Basensequenz, wie sie in SEQ ID Nr. 25 in dem Sequenzprotokoll dargestellt ist,
ein Sense-Primer mit einer Basensequenz, wie sie in SEQ ID Nr. 26 in dem Sequenzprotokoll dargestellt ist, und ein Antisense-Primer mit einer Basensequenz, wie sie in SEQ ID Nr. 27 in dem Sequenzprotokoll dargestellt ist,
ein Sense-Primer mit einer Basensequenz, wie sie in SEQ ID Nr. 28 in dem Sequenzprotokoll dargestellt ist, und ein Antisense-Primer mit einer Basensequenz, wie sie in SEQ ID Nr. 29 in dem Sequenzprotokoll dargestellt ist und
ein Sense-Primer mit einer Basensequenz, wie sie in SEQ ID Nr. 42 in dem Sequenzprotokoll dargestellt ist, und ein Antisense-Primer mit einer Basensequenz, wie sie in SEQ ID Nr. 43 in dem Sequenzprotokoll dargestellt ist.

7. Verfahren zum Unterscheiden einer mesenchymalen Stammzelle von Fibroblasten nach Anspruch 5, wobei das Echtzeit-PCR-Verfahren ein Paar von Sense- und Antisense-Primern zum Amplifizieren der Stammzell-Markergene umfasst, wobei jedes eine Basensequenz aufweist, die in SEQ ID Nr. 4, 6, 7 oder 17 dargestellt ist, wobei die Primer die folgenden sind:
ein Sense-Primer mit einer Basensequenz, wie sie in SEQ ID Nr. 49 in dem Sequenzprotokoll dargestellt ist, und ein Antisense-Primer mit einer Basensequenz, wie sie in SEQ ID Nr. 50 in dem Sequenzprotokoll dargestellt ist,
ein Sense-Primer mit einer Basensequenz, wie sie in SEQ ID Nr. 57 in dem Sequenzprotokoll dargestellt ist, und ein Antisense-Primer mit einer Basensequenz, wie sie in SEQ ID Nr. 58 in dem Sequenzprotokoll dargestellt ist,
ein Sense-Primer mit einer Basensequenz, wie sie in SEQ ID Nr. 61 in dem Sequenzprotokoll dargestellt ist, und ein Antisense-Primer mit einer Basensequenz, wie sie in SEQ ID Nr. 62 in dem Sequenzprotokoll dargestellt ist und
ein Sense-Primer mit einer Basensequenz, wie sie in SEQ ID Nr. 69 in dem Sequenzprotokoll dargestellt ist, und ein Antisense-Primer mit einer Basensequenz, wie sie in SEQ ID Nr. 70 in dem Sequenzprotokoll dargestellt ist.

8. Verfahren zum Unterscheiden einer mesenchymalen Stammzelle von Fibroblasten nach Anspruch 1, wobei die Gene in einer Testzelle amplifiziert werden unter Verwendung von wenigstens einem Paar von Primern für jedes Gen, umfassend den Sense-Primer und den Antisense-Primer nach Anspruch 6 oder Anspruch 7, und wobei die amplifizierten Gene erfasst werden unter Verwendung einer Sonde zum Erfassen von mesenchymalen Stammzell-Markergenen.

## Revendications

1. Procédé pour la distinction d'une cellule souche mésenchymateuse des fibroblastes *in vitro,* dans lequel l'expression de tous les gènes marqueurs des cellules souches mésenchynateuses suivants dans une cellule d'essai est détectée : l'apolipoprotéine D (SEQ ID NO : 4 dans la liste des séquences) ; le collagène de type XV alpha 1 (SEQ ID NO : 6 dans la liste des séquences) ; la protéine de liaison à l'ARN de la répétition de triplets CUG 2 (SEQ ID NO : 7 dans la liste des séquences) ; la métalloprotéase matricielle 1 (SEQ ID NO : 17 dans la liste des séquences) ; la protéine morphogénétique osseuse 4 d'Homo sapiens (BMP4 : numéro d'accès GenBank NM_001202) ; la séquence d'ARNm du clone 24775 d'Homo sapiens (numéro d'accès GenBank AA402981) ; la protéine Gla matricielle (MGP : numéro d'accès GenBank BF668572) ; le granule sécrétoire de la protéoglycane 1, (numéro d'accès GenBank AV734015) ; la protéine GS3955 d'Homo sapiens (numéro d'accès GenBank NM_021643) ; le polypeptide intermédiaire cytoplasmique 1 de la dynéine d'Homo sapiens (DNCI1 : numéro d'accès GenBank NM_004411); et le facteur neurotrophique dérivé du cerveau (numéro d'accès GenBank X60201).

2. Procédé pour la distinction d'une cellule souche mésenchymateuse des fibroblastes selon la revendication 1, dans lequel l'expression des gènes marqueurs des cellules souches mésenchymateuses est détectée à l'aide d'une sonde pour la détection des gènes marqueurs des cellules souches mésenchymateuses ayant une séquence d'ADN qui s'hybride dans des conditions stringentes avec l'un des gènes marqueurs des cellules souches mésenchymateuses, ayant chacun une séquence de bases représentée par la SEQ ID NO : 4, 6, 7 ou 17 dans la liste des séquences, ou le numéro d'accès GenBank NM_001202, AA402981, BF668572, AV734015, NM_021643, NM_004411 ou X60201, ou la totalité ou une partie d'un brin anti-sens de la séquence de bases représentée par la SEQ ID NO : 4, 6, 7 ou 17 dans la liste des séquences, ou le numéro d'accès GenBank NM_001202, AA402981, BF668572, AV734015, NM_021643, NM_004411 ou X60201.

3. Procédé pour la distinction d'une cellule souche mésenchymateuse des fibroblastes selon la revendication 1, dans lequel l'expression des gènes marqueurs des cellules souches mésenchymateuses est détectée à l'aide d'une micromatrice ou d'une puce à ADN pour la détection des gènes marqueurs des cellules souches mésenchymateuses, ayant chacun une séquence de bases représentée par la SEQ ID NO : 4, 6, 7 ou 17 dans la liste des séquences, ou le numéro d'accès GenBank NM_001202, AA402981, BF668572, AV734015, NM_021643, NM_004411 ou X60201.

4. Procédé pour la distinction d'une cellule souche mésenchymateuse des fibroblastes selon l'une quelconque des revendications 1 à 3, dans lequel la détection de l'expression des gènes marqueurs des cellules souches mésenchymateuses comprend l'utilisation de l'amplification en chaîne par polymérase (PCR) quantitative ou semi-quantitative.

5. Procédé pour la distinction d'une cellule souche mésenchymateuse des fibroblastes selon la revendication 4, dans lequel la PCR quantitative ou semi-quantitative est un procédé d'amplification en chaîne par polymérase en présence de transcriptase inverse (RT-PCR) ou d'amplification en chaîne par polymérase en temps réel.

6. Procédé pour la distinction d'une cellule souche mésenchymateuse des fibroblastes selon la revendication 5, dans lequel ledit procédé RT-PCR comprend une paire d'amorces sens et anti-sens pour amplifier lesdits gènes marqueurs des cellules souches, ayant chacun une séquence de bases représentée par la SEQ ID NO : 4, 6, 7 ou 17, dans lequel lesdites amorces sont :
- une amorce sens ayant une séquence de bases représentée par la SEQ ID NO : 24 dans la liste des séquences et une amorce anti-sens ayant une séquence de bases représentée par la SEQ ID NO : 25 dans la liste des séquences ;
- une amorce sens ayant une séquence de bases représentée par la SEQ ID NO : 26 dans la liste des séquences et une amorce anti-sens ayant une séquence de bases représentée par la SEQ ID NO : 27 dans la liste des séquences ;
- une amorce sens ayant une séquence de bases représentée par la SEQ ID NO : 28 dans la liste des séquences et une amorce anti-sens ayant une séquence de bases représentée par la SEQ ID NO : 29 dans la liste des séquences ; et
- une amorce sens ayant une séquence de bases représentée par la SEQ ID NO : 42 dans la liste des séquences et une amorce anti-sens ayant une séquence de bases représentée par la SEQ ID NO : 43 dans la liste des séquences.

7. Procédé pour la distinction d'une cellule souche mésenchymateuse des fibroblastes selon la revendication 5, dans lequel ledit procédé PCR en temps réel comprend une paire d'amorces sens et anti-sens pour amplifier lesdits gènes marqueurs des cellules souches, ayant chacun une séquence de bases représentée par la SEQ ID NO : 4, 6, 7 ou 17, dans lequel lesdites amorces sont :
- une amorce sens ayant une séquence de bases représentée par la SEQ ID NO : 49 dans la liste des séquences et une amorce anti-sens ayant une séquence de bases représentée par la SEQ ID NO : 50 dans la liste des séquences ;
- une amorce sens ayant une séquence de bases représentée par la SEQ ID NO : 57 dans la liste des séquences et une amorce anti-sens ayant une séquence de bases représentée par la SEQ ID NO : 58 dans la liste des séquences ;
- une amorce sens ayant une séquence de bases représentée par la SEQ ID NO : 61 dans la liste des séquences et une amorce anti-sens ayant une séquence de bases représentée par la SEQ ID NO : 62 dans la liste des séquences ; et
- une amorce sens ayant une séquence de bases représentée par la SEQ ID NO : 69 dans la liste des séquences et une amorce anti-sens ayant une séquence de bases représentée par la SEQ ID NO : 70 dans la liste des séquences.

8. Procédé pour la distinction d'une cellule souche mésenchymateuse des fibroblastes selon la revendication 1, dans lequel les gènes dans une cellule d'essai sont amplifiés à l'aide d'au moins une paire d'amorces pour chaque gène, comprenant l'amorce sens et l'amorce anti-sens selon la revendication 6 ou la revendication 7, et les gènes amplifiés sont détectés à l'aide d'une sonde pour la détection des gènes marqueurs des cellules souches mésenchymateuses.
